# FASCICULE DE BREVET EUROPEEN

(11) **EP 1 551 360 B1**
(45) Date de publication et mention de la délivrance du brevet: **24.01.2007**
(21) Numéro de dépôt: 03798958.9
(22) Date de dépôt: 03.10.2003
(51) Int. Cl.: A61K 8/49, C07D 213/74, A61Q 5/10

(54) **COUPLEURS 6-ALCOXY- 2,3-DIAMINOPYRIDINE DONT LE RADICAL AMINO EN POSITION 2 EST UN RADICAL AMINO DISUBSTITUE ET UTILISATION DE CES COUPLEURS POUR LA TEINTURE DES FIBRES KERATINIQUES**
2-AMINODISUBSTITUIERTE 6-ALKOXY-2,3-DIAMINOPYRIDIN-FARBSTOFFKUPPLER UND IHRE VERWENDUNG ZUM FÄRBEN KERATINISCHER FASERN
6-ALKOXY-2,3-DIAMINOPYRIDINE COUPLERS IN WHICH THE POSITION 2 AMINO RADICAL IS A DISUBSTITUTED AMINO RADICAL AND USE OF SAID COUPLERS FOR THE DYEING OF KERATINOUS FIBRES

(30) Priorité: 04.10.2002 FR 0212351
(43) Date de publication de la demande: 13.07.2005
(73) Titulaire: L'ORÉAL, 75008 Paris (FR)
(72) Inventeur: VIDAL, Laurent, F-75013 Paris (FR); FADLI, Aziz, F-77500 Chelles (FR)
(74) Mandataire: Fevrier, Murielle Françoise E.
(86) Numéro de dépôt international: PCT/FR2003/002920
(87) Numéro de publication internationale: WO 2004/030643

(56) Documents cités:
- EP-A- 0 728 464
- DE-A- 3 233 540
- DE-A- 4 115 148
- DE-A- 19 936 442
- FR-A- 1 397 551
- FR-A- 2 779 952
- FR-A- 2 835 741
- US-A1- 2002 013 973
- DATABASE CHEMLIST [en ligne] 154036, XP002243965

## Description

L'invention a pour objet une composition tinctoriale utile pour la teinture des fibres kératiniques contenant au moins une base d'oxydation et au moins un coupleur du type 6-alcoxy-2,3-diaminopyridine dont le radical amino en position 2 est un radical amino disubstitué, l'utilisation de cette composition pour la teinture des fibres kératiniques ainsi que le procédé de teinture mettant en oeuvre cette composition. L'invention a aussi pour objet de nouveaux composés 6-alcoxy-2,3-diaminopyridines utiles comme coupleurs.

Il est connu de teindre les fibres kératiniques et en particulier les cheveux humains avec des compositions tinctoriales contenant des précurseurs de colorant d'oxydation, appelés généralement bases d'oxydation, tels que des ortho ou paraphénylènediamines, des ortho ou para-aminophénols et des composés hétérocycliques. Ces bases d'oxydation sont des composés incolores ou faiblement colorés qui, associés à des produits oxydants, peuvent donner naissance par un processus de condensation oxydative à des composés colorés.

On sait également que l'on peut faire varier les nuances obtenues avec ces bases d'oxydation en les associant à des coupleurs ou modificateurs de coloration, ces derniers étant choisis notamment parmi les métadiamines aromatiques, les métaaminophénols, les métadiphénols et certains composés hétérocycliques tels que des composés indoliques.

La variété des molécules mises en jeu au niveau des bases d'oxydation et des coupleurs, permet l'obtention d'une riche palette de couleurs.

La coloration dite "permanente" obtenue grâce à ces colorants d'oxydation, doit par ailleurs satisfaire un certain nombre d'exigences. Ainsi, elle doit être sans inconvénient sur le plan toxicologique, elle doit permettre d'obtenir des nuances dans l'intensité souhaitée et présenter une bonne tenue face aux agents extérieurs tels que la lumière, les intempéries, le lavage, les ondulations permanentes, la transpiration et les frottements.

Les colorants doivent également permettre de couvrir les cheveux blancs, et être enfin les moins sélectifs possibles, c'est-à-dire permettre d'obtenir des écarts de coloration les plus faibles possibles tout au long d'une même fibre kératinique, qui est en général différemment sensibilisée (i.e. abîmée) entre sa pointe et sa racine.

Le document FR 1397551 décrit des compositions tinctoriales contenant des précurseurs de colorant d'oxydation du type dérivé pyridinique tri-substitués, chacun des substituants pouvant être un radical hydroxy, alcoxy, amino, ou NR₁R₂ avec R₁R₂ représentant un H, alkyle, aryle. La coloration est obtenue soit par oxydation à l'air soit par un milieu oxydant contenant de l'eau oxygénée à pH basique. En raison de la forte oxydabilité de ces précurseurs pyridiniques, les teintures obtenues sur cheveux ont tendance à évoluer dans le temps en changeant de couleur, ce qui s'avère particulièrement inesthétique.

Le document DE 3 233 540 propose des compositions pour la teinture des cheveux contenant à titre de coupleur des dérivés de 6-alcoxy-3-aminopyridine substitués en position 2 par un radical NH₂ ou NHR₃ avec R₃ = H, alkyle en C₁-C₄ ou hydroxyalkyle en C₂-C₄ en association avec des bases d'oxydation classiques. Ces compositions donnent en présence de certaines bases comme la para-phenylène diamine ou la para-toluènediamine des nuances bleues foncées fragiles à la lumière et manquant d'intensité et d'uniformité entre la racine et la pointe des cheveux.

Les documents DE 4 115 148, FR 2 779 952, EP 728 464, DE 199 36442 proposent d'associer ce type de coupleurs pyridiniques particuliers avec des bases d'oxydation spécifiques telles que des base pyrazolo-pyrimidines, para-aminophenols, pyrimidiniques ou 4,5 ou 3,4-diaminopyrazoles.

Toutes ces compositions ne permettent cependant pas d'obtenir des colorations intenses dans des nuances variées qui sont uniformes de la racine à la pointe des cheveux, peu sélectives et particulièrement résistantes, et qui présentent une bonne chromaticité.

Ce but est atteint avec la présente invention qui a pour objet une composition tinctoriale comprenant, dans un milieu de teinture approprié,
- au moins une base d'oxydation, et
- au moins un coupleur 6-alcoxy-2,3-diaminopyridine de formule (I) ou l'un de ses sels d'addition correspondants : 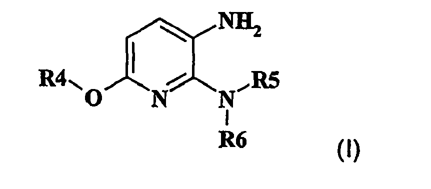 dans laquelle:
   - R₄ représente un radical alkyle en C₁-C₄ linéaire ou ramifié éventuellement substitué par un ou plusieurs radicaux hydroxyle, alcoxy en C₁-C₂, NR₇R₈ où R₇ et R₈ sont choisis parmi un atome d'hydrogène, un alkyle en C₁-C₄, un (poly)hydroxyalkyle en C₂-C₆, un (poly)aminoalkyle en C₂-C₆ ou un amino-hydroxyalkyle en C₂-C₆ ;
   - R₅ représente un radical alkyle linéaire ou ramifié en C₁-C₆ éventuellement substitué par un ou plusieurs radicaux choisis parmi les radicaux alcoxy en C₁-C₂, hydroxy, carboxy, sulfonique (-SO₃H), NR₉R₁₀ ; un radical 2-acylaminoéthyle ; un radical 2-(2-hydroxyéthyloxy)-éthyle ;
   - R₆ représente un radical alkyle en C₁-C₆ linéaire ou ramifié éventuellement substitué par un ou plusieurs radicaux choisis parmi les radicaux alcoxy en C₁-C₂, hydroxy, carboxy, sulfonique, NR'₉R'₁₀, ou acylamino (RCONH- avec R étant un alkyle en C₁-C₄) ;un radical 2-(2-hydroxyéthyloxy)-éthyle ;
   - R₉, R₁₀, R₉' et R₁₀' désignent, indépendamment l'un de l'autre, un atome d'hydrogène ; un radical alkyle en C₁-C₄ éventuellement substitué par un ou plusieurs radicaux hydroxy, amino, mono- ou di- alkylamino, alcoxy ou acylamino.

Dans le cadre de la présente invention, on entend par alkyle, des radicaux linéaires ou ramifiés par exemple méthyle, éthyle, n-propyle, iso-propyle, butyle, etc, des radicaux cycloalkyle tels que des radicaux cyclobutyle, cyclopentyle, cyclohexyle, diazépane etc. Un radical alcoxy est un radical alk-O, le radical alkyle ayant la définition donnée ci dessus.

A titre d'exemple, R₅ et R₆ représentent indépendamment un radical méthyle, éthyle, propyle, hydroxyéthyle par exemple le 2-hydroxyéthyle, aminoéthyle par exemple le 2-aminoéthyle, carboxyéthyle par exemple 2-carboxyéthyle, acylaminoéthyle, hydroxypropyle par exemple 2-hydroxypropyle' ou 3-hydroxypropyle, aminopropyle par exemple 3-aminopropyle, N,N-diméthylaminoéthyle par exemple 2-N,N-diméthylaminoéthyle, N-méthylaminoéthyle par exemple 2-N-méthylaminoéthyle, (2-hydroxyéthylamino)-éthyle, (2-hydroxyéthyloxy)-éthyle.

De préférence, R₅ représente un radical alkyle en C₁-C₄ éventuellement substitué par un ou plusieurs radicaux choisis parmi les radicaux hydroxy, alcoxy en C₁-C₂, amino, (di)alkylamino en C₁-C₂, carboxy, (di)hydroxyalkylamino en C₁-C₂ ou sulfonique ; un radical 2-acylaminoéthyle ; un radical 2-(2-hydroxyéthyloxy)-éthyle. En particulier R₅ représente un radical alkyle ou un radical alkyle substitué par un ou plusieurs radicaux choisis parmi un hydroxy, alcoxy en C₁-C₂, amino, (di)alkylamino en C₁-C₂, carboxy, (di)hydroxyalkylamino en C₁-C₂ ; un radical 2-acylaminoéthyle ; un radical 2-(2-hydroxyéthyloxy)-éthyle. Selon un mode de réalisation particulièrement préféré, R₅ représente un radical méthyle, éthyle, propyle, 2-hydroxyéthyle, 2-aminoéthyle, 2-carboxyéthyle, 2-acylaminoéthyle, 2-hydroxypropyle, 3-hydroxypropyle , 3-aminopropyle, 2-N,N-diméthylaminoéthyle, 2-N-méthylaminoéthyle, 2-(2-hydroxyéthylamino)-éthyle ou 2-(2-hydroxyéthyloxy)-éthyle.

Selon un mode de réalisation particulier, R₆ représente un radical alkyle substitué.

A titre d'exemple, R₆ représente un radical hydroxyéthyle, aminoéthyle, carboxyéthyle, acylaminoéthyle, hydroxypropyle, aminopropyle, N,N-diméthytaminoéthyle, N-méthylaminoéthyle, (2-hydroxyéthylamino)-éthyle, (2-hydroxyéthyloxy)-éthyle.

De préférence, R₆ représente un radical alkyle en C₁-C₄ substitué par un ou plusieurs radicaux choisis parmi les radicaux hydroxy, alcoxy en C₁-C₂, amino, (di)alkylamino en C₁-C₂, carboxy, acylamino, (di)hydroxyalkylamino en C₁-C₂ ou sulfonique ; un radical 2-(2-hydroxyéthyloxy)-éthyle, en particulier R₆ est un radical alkyle en C₁-C₄ substitué par un ou plusieurs radicaux choisis parmi un hydroxy, alcoxy en C₁-C₂, amino ou (di)alkylamino en C₁-C₂, carboxy, acylamino, (di)hydroxyalkylamino ; un radical 2-(2-hydroxyéthyloxy)-éthyle. Selon un mode de réalisation particulièrement préféré, R₆ représente un radical 2-hydroxyéthyle, 2-aminoéthyle, 2-carboxyéthyle, 2-acylaminoéthyle, 2-hydroxypropyle, 3-hydroxypropyle, 3-aminopropyle, 2-N,N-diméthylaminoéthyle, 2-N-méthylaminoéthyle, 2-(2-hydroxyéthylamino)-éthyle, 2-(2-hydroxyéthyloxy)-éthyle.

Dans la formule (I), R₄ représente de préférence un radical alkyle en C₁-C₄ éventuellement substitué par un ou plusieurs radicaux hydroxyle, alcoxy en C₁-C₂. De préférence, R₄ représente de préférence un radical alkyle en C₁-C₄.

Selon un mode de réalisation particulier, R₅ est un radical alkyle et R₆ est un radical alkyle substitué par un ou plusieurs hydroxy. Selon un autre mode de réalisation, R₅ et R₆ représentent un radical alkyle substitué par un ou plusieurs hydroxy.

Les composés de formule (I) sont par exemple les composés

| | |
|---|---|
| 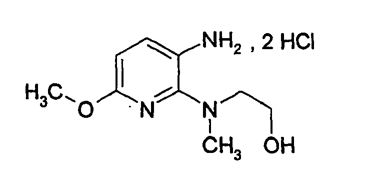 | 2-[(3-amino-6-méthoxy-pyridin-2-yl)-méthyl-amino]-éthanol |
| 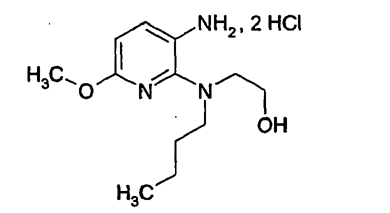 | 2-[(3-amino-6-méthoxy-pyridin-2-yl)-butyl-amino]-éthanol |
| 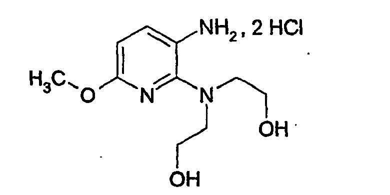 | 2-[(3-amino-6-méthoxy-pyridin-2-yl)-(2-hydroxy-éthyl)-amino]-éthanol |
| 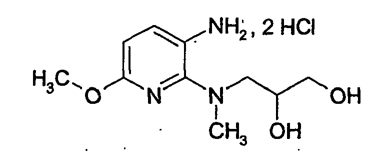 | 3-[(3-amino-6-méthoxy-pyridin-2-yl)-méthyl-amino]-propane-1,2-diol |
| 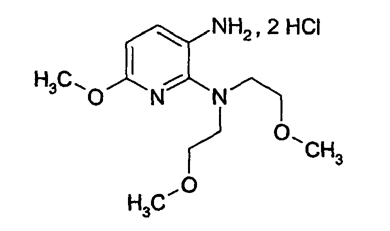 | 6-méthoxy-N2,N2-bis-(2-méthoxy-éthyl)-pyridine-2,3-diamine |
| 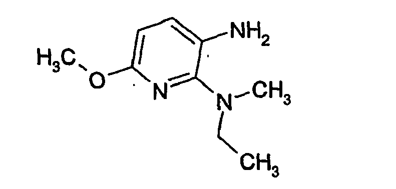 | 6-méthoxy-N2-éthyl-N2-méthyl-pyridine-2,3-diamine |
| 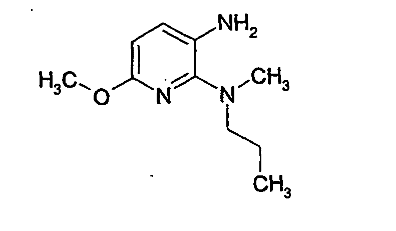 | 6-méthoxy-N2-méthyl-N2-propyl-pyridine-2,3-diamine |
| 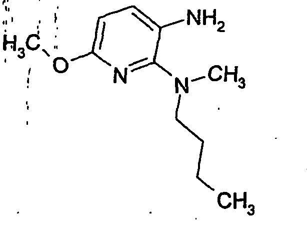 | 6-méthoxy-N2-butyl-N2-méthyl-pyridine-2,3-diamine |
| 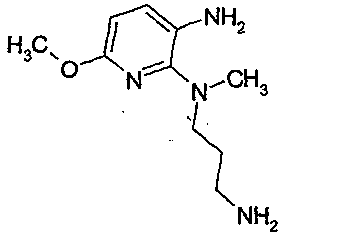 | 6-méthoxy-N2-(3-amino-propyl)-N2-méthyl-pyridine-2,3-diamine |
| 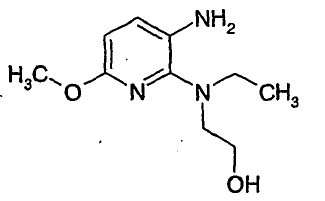 | 2-[(3-amino-6-méthoxy-pyridin-2-yl)-éthyl-amino]-éthanol |
| 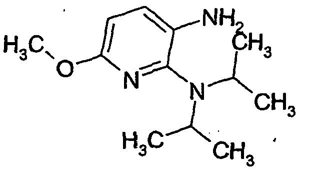 | 6-méthoxy-N2,N2-diisopropyl-pyridine-2,3-diamine |
| 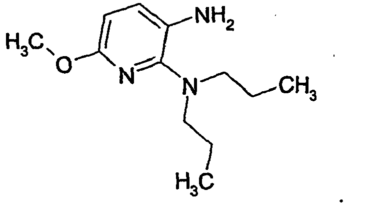 | 6-méthoxy-N2,N2-dipropyl-pyridine-2,3-diamine |
| 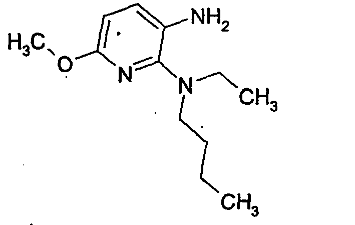 | 6-méthoxy-N2-butyl-N2-éthyl-pyridine-2,3-diamine |
| 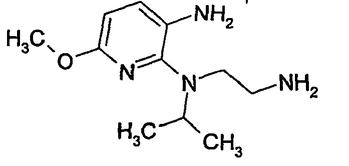 | 6-méthoxy-N2-(2-amino-éthyl)-N2-isopropyl-pyridine-2,3-diamine |
| 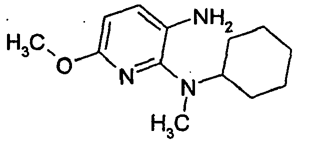 | 6-méthoxy-N2-cyclohexyl-N2-méthyl-pyridine-2,3-diamine |
| 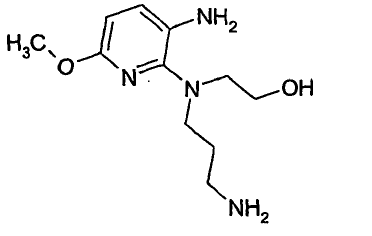 | 2-[(3-amino-6-méthoxy-pyridin-2-yl)-(3-amino-propyl)-amino]-éthanol |
| 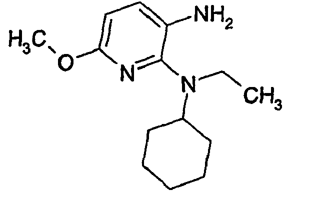 | 6-méthoxy-N2-cyclohexyl-N2-éthyl-pyridine-2,3-diamine |
| 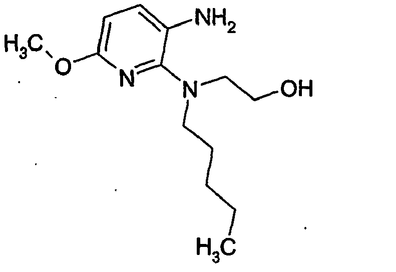 | 2-[(3-amino-6-méthoxy-pyridin-2-yl)-pentyl-amino]-éthanol |
| 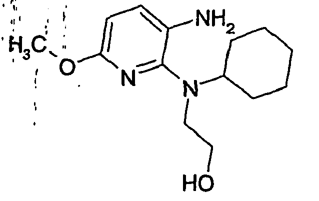 | 2-[(3-amino-6-méthoxy-pyridin-2-yl)-cyclohexyl-amino]-éthanol |
| 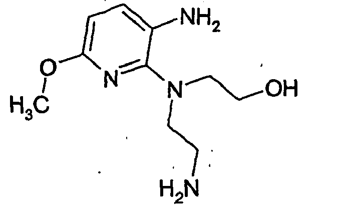 | 2-[(3-amino-6-méthoxy-pyridin-2-yl)-(2-amino-éthyl)-amino]-éthanol |
| 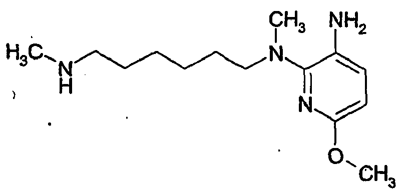 | 6-méthoxy-N2-méthyl-N2-(6-méthylamino-hexyl)-pyridine-2,3-diamine |

ainsi que leurs sels d'addition.

La composition de teinture par oxydation de la présente invention comprend une ou plusieurs bases d'oxydation classiquement utilisées en teinture d'oxydation. A titre d'exemple, ces bases d'oxydation additionnelles sont choisies parmi les para-phénylènediamines, les bis-phénylalkylènediamines, les para-aminophénols, les bis-para-aminophénols, les ortho-aminophénols, les bases hétérocycliques et leurs sels d'addition.

Parmi les para-phénylènediamines, on peut citer à titre d'exemple, la paraphénylènediamine, la para-toluènediamine, la 2-chloro para-phénylènediamine, la 2,3-diméthyl para-phénylènediamine, la 2,6-diméthyl para-phénylènediamine, la 2,6-diéthyl para-phénylènediamine, la 2,5-diméthyl para-phénylènediamine, la N,N-diméthyl paraphénylènediamine, la N,N-diéthyl para-phénylènediamine, la N,N-dipropyl paraphénylènediamine, la 4-amino N,N-diéthyl 3-méthyl aniline, la N,N-bis-(β-hydroxyéthyl) para-phénylènediamine, la 4-N,N-bis-(β-hydroxyéthyl)amino 2-méthyl aniline, la 4-N,N-bis-(β-hydroxyéthyl)amino 2-chloro aniline, la 2-β-hydroxyéthyl paraphénylènediamine, la 2-fluoro para-phénylènediamine, la 2-isopropyl . paraphénylènediamine, la N-(β-hydroxypropyl) para-phénylènediamine, la 2-hydroxyméthyl para-phénylènediamine, la N,N-diméthyl 3-méthyl para-phénylènediamine, la N,N-(éthyl, β-hydroxyéthyl) para-phénylènediamine, la N-(β,γ-dihydroxypropyl) paraphénylènediamine, la N-(4'-aminophényl) para-phénylènediamine, la N-phényl paraphénylènediamine, la 2-β-hydroxyéthyloxy para-phénylènediamine, la 2-β-acétylaminoéthyloxy para-phénylènediamine, la N-(β-méthoxyéthyl) para-phénylènediamine, la 4-aminophénylpyrrolidine, la 2-thiényl para-phénylènediamine, le 2-β hydroxyéthylamino 5-amino toluène, la 3-hydroxy 1-(4'-aminophényl)pyrrolidine et leurs sels d'addition avec un acide.

Parmi les para-phénylènediamines citées ci-dessus, la paraphénylènediamine, la para-toluènediamine, la 2-isopropyl para-phénylènediamine, la 2-β-hydroxyéthyl para-phénylènediamine, la 2-β-hydroxyéthyloxy para-phénylènediamine, la 2,6-diméthyl para-phénylènediamine, la 2,6-diéthyl para-phénylènediamine, la 2,3-diméthyl para-phénylènediamine, la N,N-bis-(β-hydroxyéthyl) paraphénylènediamine, la 2-chloro para-phénylènediamine, la 2-β-acétylaminoéthytoxy para-phénylènediamine, et leurs sels d'addition avec un acide sont particulièrement préférées.

Parmi les bis-phénylalkylènediamines, on peut citer à titré d'exemple, le N,N'-bis-(β-hydroxyéthyl) N,N'-bis-(4'-aminophényl) 1,3-diamino propanol, la N,N'-bis-(β-hydroxyéthyl) N,N'-bis-(4'-aminophényl) éthylènediamine, la N,N'-bis-(4-aminophényl) tétraméthylènediamine, la N,N'-bis-(β-hydroxyéthyl) N,N'-bis-(4-aminophényl) tétraméthylènediamine, la N,N'-bis-(4-méthyl-aminophényl) tétraméthylènediamine, la N,N'-bis-(éthyl) N,N'-bis-(4'-amino, 3'-méthylphényl) éthylènediamine, le 1,8-bis-(2,5-diamino phénoxy)-3,6-dioxaoctane, et leurs sels d'addition avec un acide.

Parmi les para-aminophénols, on peut citer à titre d'exemple, le paraaminophénol, le 4-amino 3-méthyl phénol, le 4-amino 3-fluoro phénol, le 4-amino 3-hydroxyméthyl phénol, le 4-amino 2-méthyl phénol, le 4-amino 2-hydroxyméthyl phénol, le 4-amino 2-méthoxyméthyl phénol, le 4-amino 2-aminométhyl phénol, le 4-amino 2-(β-hydroxyéthyl aminométhyl) phénol, le 4-amino 2-fluoro phénol, et leurs sels d'addition avec un acide.

Parmi les ortho-aminophénols, on peut citer à titre d'exemple, le 2-amino phénol, le 2-amino 5-méthyl phénol, le 2-amino 6-méthyl phénol, le 5-acétamido 2-amino phénol, et leurs sels d'addition avec un acide.

Parmi les bases hétérocycliques, on peut citer à titre d'exemple, les dérivés pyridiniques, les dérivés pyrimidiniques et les dérivés pyrazoliques.

Parmi les dérivés pyridiniques, on peut citer les composés décrits par exemple dans les brevets GB 1 026 978 et GB 1 153 196, comme la 2,5-diamino pyridine, la 2-(4-méthoxyphényl)amino 3-amino pyridine, la 2,3-diamino 6-méthoxy pyridine, la 2-(β-méthoxyéthyl)amino 3-amino 6-méthoxy pyridine, la 3,4-diamino pyridine, et leurs sels d'addition avec un acide.

D'autres bases d'oxydation pyridiniques utiles dans la présente invention sont les bases d'oxydation 3-amino pyrazolo-[1,5-a]-pyridines ou leurs sels d'addition décrits par exemple dans la demande de brevet FR 2801308. A titre d'exemple, on peut citer la pyrazolo[1,5-a]pyridin-3-ylamine ; la 2-acétylamino pyrazolo-[1,5-a] pyridin-3-ylamine ; la 2-morpholin-4-yl-pyrazolo[1,5-a]pyridin-3-ylamine ; l'acide 3-amino-pyrazolo[1,5-a]pyridin-2-carboxylique ; la 2-méthoxy-pyrazolo[1,5-a]pyridine-3-ylamino ; le (3-amino-pyrazolo[1,5-a]pyridine-7-yl)-méthanol ; le 2-(3-amino-pyrazolo[1,5-a]pyridine-5-yl)-éthanol ; le 2-(3-amino-pyrazolo[1,5-a]pyridine-7-yl)-éthanol ; le (3-amino-pyrazolo[1,5-a]pyridine-2-yl)-méthanol ; la 3,6-diamino-pyrazoio[1,5-a]pyridine ; la 3,4-diamino-pyrazolo[1,5-a]pyridine ; la pyrazolo[1,5-a]pyridine-3,7-diamine ; la 7-morpholin-4-yl-pyrazolo[1,5-a]pyridin-3-ylamine ; la pyrazolo[1,5-a]pyridine-3,5-diamine ; la 5-morpholin-4-yl-pyrazolo[1,5-a]pyridin-3-ylamine ; le 2-[(3-amino-pyrazolo[1,5-a]pyridin-5-yl)-(2-hydroxyéthyl)-amino]-éthanol ; le 2-[(3-amino-pyrazolo[1,5-a]pyridin-7-yl)-(2-hydroxyéthyl)-amino]-éthanol ; la 3-amino-pyrazolo[1,5-a]pyridine-5-ol ; 3-amino-pyrazolo[1,5-a]pyridine-4-ol ; la 3-amino-pyrazolo[1,5-a]pyridine-6-ol ; la 3-amino-pyrazolo[1,5-a]pyridine-7-ol ; ainsi que leurs d'addition avec un acide ou avec une base.

Parmi les dérivés pyrimidiniques, on peut citer les composés décrits par exemple dans les brevets DE 2359399 ; JP 88-169571 ; JP 05-63124 ; EP 0770375 ou demande de brevet WO 96/15765 comme la 2,4,5,6-tétra-aminopyrimidine, la 4-hydroxy 2,5,6-triaminopyrimidine, la 2-hydroxy 4,5,6-triaminopyrimidine, la 2,4-dihydroxy 5,6-diaminopyrimidine, la 2,5,6-triaminopyrimidine, et les dérivés pyrazolo-pyrimidiniques tels ceux mentionnés dans la demande de brevet FR-A-2750048 et parmi lesquels on peut citer la pyrazolo-[1,5-a]-pyrimidine-3,7-diamine ; la 2,5-diméthyl pyrazolo-[1,5-a]-pyrimidine-3,7-diamine ; la pyrazolo-[1,5-a]-pyrimidine-3,5-diamine ; la 2,7-diméthyl pyrazolo-[1,5-a]-pyrimidine-3,5-diamine; le 3-amino pyrazolo-[1,5-a]-pyrimidin-7-ol ; le 3-amino pyrazolo-[1,5-a]-pyrimidin-5-ol ; le 2-(3-amino pyrazolo-[1,5-a]-pyrimidin-7-ylamino)-éthanol, le 2-(7-amino pyrazolo-[1,5-a]-pyrimidin-3-ylamino)-éthanol, le 2-[(3-amino-pyrazolo[1,5-a]pyrimidin-7-yl)-(2-hydroxy-éthyl)-amino]-éthanol, le 2-[(7-amino-pyrazolo[1,5-a]pyrimidin-3-yl)-(2-hydroxy-éthyl)-amino]-éthanol, la 5,6-diméthyl pyrazolo-[1,5-a]-pyrimidine-3,7-diamine, la 2,6-diméthyl pyrazolo-[1,5-a]-pyrimidine-3,7-diamine, la 2, 5, N 7, N 7-tetraméthyl pyrazolo-[1,5-a]-pyrimidine-3,7-diamine, la 3-amino-5-méthyl-7-imidazolylpropylamino pyrazolo-[1,5-a]-pyrimidine et leurs sels d'addition avec un acide et leurs formes tautomères, lorsqu'il existe un équilibre tautomériqué.

Parmi les dérivés pyrazoliques, on peut citer les composés décrits dans les brevets DE 3843892, DE 4133957 et demandes de brevet WO 94/08969, WO 94/08970, FR-A-2 733 749 et DE 195 43 988 comme le 4,5-diamino 1-méthyl pyrazole, le 4,5-diamino 1-(β-hydroxyéthyl) pyrazole, le 3,4-diamino pyrazole, le 4,5-diamino 1-(4'-chlorobenzyl) pyrazole, le 4,5-diamino 1,3-diméthyl pyrazole, le 4,5-diamino 3-méthyl 1-phényl pyrazole, le 4,5-diamino 1-méthyl 3-phényl pyrazole, le 4-amino 1,3-diméthyl 5-hydrazino pyrazole, le 1-benzyl 4,5-diamino 3-méthyl pyrazole, le 4,5-diamino 3-tert-butyl 1-méthyl pyrazole, le 4,5-diamino 1-tert-butyl 3-méthyl pyrazole, le 4,5-diamino 1-(β-hydroxyéthyl) 3-méthyl pyrazole, le 4,5-diamino 1-éthyl 3-méthyl pyrazole; le 4,5-diamino 1-éthyl 3-(4'-méthoxyphényl) pyrazole, le 4,5-diamino 1-éthyl 3-hydroxyméthyl pyrazole, le 4,5-diamino 3-hydroxyméthyl 1-méthyl pyrazole, le 4,5-diamino 3-hydroxyméthyl 1-isopropyl pyrazole, le 4,5-diamino 3-méthyl 1-isopropyl pyrazole, le 4-amino 5-(2'-aminoéthyl)amino 1,3-diméthyl pyrazole, le 3,4,5-triamino pyrazole, le 1-méthyl 3,4,5-triamino pyrazole, le 3,5-diamino 1-méthyl 4-méthylamino pyrazole, le 3,5-diamino 4-(β-hydroxyéthyl)amino 1-méthyl pyrazole, et leurs sels d'addition avec un acide.

La ou les bases d'oxydation présentes dans la composition de l'invention sont en général présentent chacune en quantité comprise entre 0,001 à 10 % en poids environ du poids total de la composition tinctoriale, de préférence entre 0,005 et 6 %.

La composition selon l'invention peut contenir un ou plusieurs coupleurs additionnels conventionnellement autres que le coupleur de formule (I). Parmi ces coupleurs additionnels, on peut notamment citer les métaphénylènediamines, les méta-aminophénols, les métadiphénols, les coupleurs naphtaléniques, les coupleurs hétérocycliques autres que les coupleurs décrits précédemment et leur sels d'addition.

A titre d'exemple, on peut citer le 2-méthyl 5-aminophénol, le 5-N-(β-hydroxyéthyl)amino 2-méthyl phénol, le 6-chloro-2-méthyl-5-aminophénol, le 3-amino phénol, le 1,3-dihydroxy benzène, le 1,3-dihydroxy 2-méthyl benzène, le 4-chloro 1,3-dihydroxy benzène, le 2,4-diamino 1-(β-hydroxyéthyloxy) benzène, le 2-amino 4-(β-hydroxyéthylamino) 1-méthoxybenzène, le 1,3-diamino benzène, le 1,3-bis-(2,4-diaminophénoxy) propane, la 3-uréido aniline, le 3-uréido 1-diméthylamino benzène, le sésamol, le 1-β-hydroxyéthylamino-3,4-méthylènedioxybenzène, l'α-naphtol, le 2 méthyl-1-naphtol, le 6-hydroxy indole, le 4-hydroxy indole, le 4-hydroxy N-méthyl indole, la 2-amino-3-hydroxy pyridine, la 6- hydroxy benzomorpholine la 3,5-diamino-2,6-diméthoxypyridine, le 1-N-(β-hydroxyéthyl)amino-3,4-méthylène dioxybenzène, le 2,6-bis-(β-hydroxyéthylamino)toluène et leurs sels d'addition avec un acide.

Dans la composition de la présente invention, le ou les coupleurs sont chacun généralement présents en quantité comprise entre 0,001 et 10 % en poids environ du poids total de la composition tinctoriale, de préférence entre 0,005 et 6 %.

D'une manière générale, les sels d'addition des bases d'oxydation et des coupleurs utilisables dans le cadre de l'invention sont notamment choisis parmi les sels d'addition avec un acide tels que les chlorhydrates, les bromhydrates, les sulfates, les citrates, les succinates, les tartrates, les lactates, les tosylates, les benzènesulfonates, les phosphates et les acétates et les sels d'addition avec une base telles que la soude, la potasse, l'ammoniaque, les amines ou les alcanolamines.

La composition tinctoriale conforme à l'invention peut en outre contenir un ou plusieurs colorants directs pouvant notamment être choisis parmi les colorants nitrés de la série benzénique, les colorants directs azoïques, les colorants directs méthiniques. Ces colorants directs peuvent être de nature non ionique, anionique ou cationique.

La composition tinctoriale de la présente invention est particulièrement utile pour la teinture des fibres kératiniques, en particulier les fibres kératiniques humaines. Dans ce cas, le milieu est un milieu cosmétique approprié à la teinture de ces fibres.

Ce milieu approprié pour la teinture appelé aussi support de teinture est généralement constitué par de l'eau ou par un mélange d'eau et d'au moins un solvant organique pour solubiliser les composés qui ne seraient pas suffisamment solubles dans l'eau. A titre de solvant organique, on peut par exemple citer les alcanols inférieurs en C₁-C₄, tels que l'éthanol et l'isopropanol ; les polyols et éthers de polyols comme le 2-butoxyéthanol, le propylèneglycol, le monométhyléther de propylèneglycol, le monoéthyléther et le monométhyléther du diéthylèneglycol, ainsi que les alcools aromatiques comme l'alcool benzylique ou le phénoxyéthanol, et leurs mélanges.

Les solvants sont, de préférence, présents dans des proportions de préférence comprises entre 1 et 40 % en poids environ par rapport au poids total de la composition tinctoriale, et encore plus préférentiellement entre 5 et 30 % en poids environ.

La composition tinctoriale conforme à l'invention peut également renfermer divers adjuvants utilisés classiquement dans les compositions pour la teinture des cheveux, tels que des agents tensio-actifs anioniques, cationiques, non-ioniques, amphotères, zwitterioniques ou leurs mélanges, des polymères anioniques, cationiques, non-ioniques, amphotères, zwitterioniques ou leurs mélanges, des agents épaississants minéraux ou organiques, et en particulier les épaississants associatifs polymères anioniques, cationiques, non ioniques et amphotères, des agents antioxydants, des agents de pénétration, des agents séquestrants, des parfums, des tampons, des agents dispersants, des agents de conditionnement tels que par exemple des silicones volatiles ou non volatiles, modifiées ou non modifiées, des agents filmogènes, des céramides, des agents conservateurs, des agents opacifiants.

Les adjuvants ci dessus sont en général présents en quantité comprise pour chacun d'eux entre 0,01 et 20 % en poids par rapport au poids de la composition.

Bien entendu, l'homme de l'art veillera à choisir ce ou ces éventuels composés complémentaires de manière telle que les propriétés avantageuses attachées intrinsèquement à la composition de teinture d'oxydation conforme à l'invention ne soient pas, ou substantiellement pas, altérées par la ou les adjonctions envisagées.

Le pH de la composition tinctoriale conforme à l'invention est généralement compris entre 3 et 12 environ, et de préférence entre 5 et 11 environ. Il peut être ajusté à la valeur désirée au moyen d'agents acidifiants ou alcalinisants habituellement utilisés en teinture des fibres kératiniques ou bien encore à l'aide de systèmes tampons classiques.

Parmi les agents acidifiants, on peut citer, à titre d'exemple, les acides minéraux ou organiques comme l'acide chlorhydrique, l'acide orthophosphorique, l'acide sulfurique, les acides carboxyliques comme l'acide acétique, l'acide tartrique, l'acide citrique, l'acide lactique, les acides sulfoniques.

Parmi les agents alcalinisants on peut citer, à titre d'exemple, l'ammoniaque, les carbonates alcalins, les alcanolamines telles que les mono-, di- et triéthanotamines ainsi que leurs dérivés, les hydroxydes de sodium ou de potassium et les composés de formule (II) suivante : 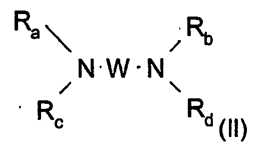 dans laquelle W est un reste propylène éventuellement substitué par un groupement hydroxyle ou un radical alkyle en C₁-C₄ ; Rₐ, R_{b}, R_{c} et R_{d}, identiques ou différents, représentent un atome d'hydrogène, un radical alkyle en C₁-C₄ ou hydroxyalkyle en C₁-C₄.

La composition tinctoriale selon l'invention peut se présenter sous des formes diverses, telles que sous forme de liquides, de crèmes, de gels, ou sous toute autre forme appropriée pour réaliser une teinture des fibres kératiniques, et notamment des cheveux humains.

Le procédé de la présente invention est un procédé dans lequel on applique sur les fibres la composition selon la présente invention telle que définie précédemment, en présence d'un agent oxydant pendant un temps suffisant pour développer la coloration désirée. La couleur peut être révélée à pH acide, neutre ou alcalin et l'agent oxydant peut être ajouté à la composition de l'invention juste au moment de l'emploi ou il peut être mis en oeuvre à partir d'une composition oxydante le contenant, appliquée simultanément ou séquentiellement à la composition de l'invention.

Selon un mode de réalisation particulier, la composition selon la présente invention est mélangée, de préférence au moment de l'emploi, à une composition contenant, dans un milieu approprié pour la teinture, au moins un agent oxydant, cet agent oxydant étant présent en une quantité suffisante pour développer une coloration. Le mélange obtenu est ensuite appliqué sur les fibres kératiniques. Après un temps de pose de 3 à 50 minutes environ, de préférence 5 à 30 minutes environ, les fibres kératiniques sont rincées, lavées au shampooing, rincées à nouveau puis séchées.

Les agents oxydants classiquement utilisés pour la teinture d'oxydation des fibres kératiniques sont par exemple le peroxyde d'hydrogène, le peroxyde d'urée, les bromates de métaux alcalins, les persels tels que les perborates et persulfates, les peracides et les enzymes oxydases parmi lesquelles on peut citer les peroxydases, les oxydo-réductases à 2 électrons telles que les uricases et les oxygénases à 4 électrons comme les laccases. Le peroxyde d'hydrogène est particulièrement préféré.

La composition oxydante peut également renfermer divers adjuvants utilisés classiquement dans les compositions pour la teinture des cheveux et tels que définis précédemment.

Le pH de la composition oxydante renfermant l'agent oxydant est tel qu'après mélange avec la composition tinctoriale, le pH de la composition résultante appliquée sur les fibres kératiniques varie de préférence entre 3 et 12 environ, et encore plus préférentiellement entre 5 et 11. Il peut être ajusté à la valeur désirée au moyen d'agents acidifiants ou alcalinisants habituellement utilisés en teinture des fibres kératiniques et tels que définis précédemment.

La composition prête à l'emploi qui est finalement appliquée sur les fibres kératiniques peut se présenter sous des formes diverses, telles que sous forme de liquides, de crèmes, de gels ou sous toute autre forme appropriée pour réaliser une teinture des fibres kératiniques, et notamment des cheveux humains.

La composition de l'invention peut se présenter sous forme de kit. Un tel kit comprend d'une part une composition telle que définie précédemment et d'autre part une composition oxydante.

L'invention a aussi pour objet un dispositif à plusieurs compartiments dans lequel un premier compartiment renferme la composition tinctoriale de la présente invention définie ci-dessus et un deuxième compartiment renferme un agent oxydant. Ce dispositif peut être équipé d'un moyen permettant de délivrer sur les cheveux le mélange souhaité, tel que les dispositifs décrits dans le brevet FR-2 586 913 au nom de la demanderesse.

A partir de ce dispositif, il est possible de teindre les fibres kératiniques à partir d'un procédé qui comprend le mélange d'une composition tinctoriale comprenant de l'invention avec un agent oxydant, et l'application du mélange obtenu sur les fibres kératiniques pendant un temps suffisant pour développer la coloration désirée.

La présente invention a enfin pour objet les composés 6-alcoxy-2,3-diaminopyridine de formule (I) ainsi que leurs sels d'addition correspondants tels que définis précédemment à l'exception de la 2-(N-méthyl N-(β-hydroxy-éthyl)amino) 3-amino 6-méthoxy pyridine.

Ces composés peuvent être synthétisés selon le schéma de synthèse suivant :

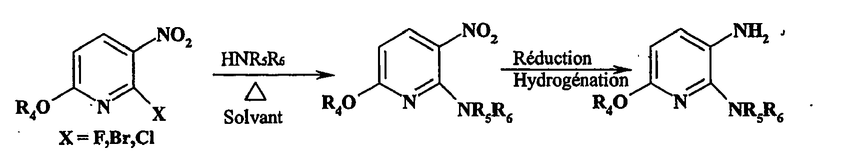

La première étape consiste à faire réagir un dérivé de 6-alcoxy-3-nitro-2-halogéno-pyridine avec une amine de type HNR₅R₆ dans laquelle R₅ et R₆ ont les mêmes significations indiquées ci-dessus dans un solvant polaire de point d'ébullition compris entre 70°C et 180°C. La température de réaction varie selon les dérivés de pyridine et l'amine nucléophile, de 75°C à -140°C. De préférence, on choisira comme solvant, les alcools tels que l'éthanol, isopropanol, le butanol, le pentanol ainsi que l'acide acétique, formique ou le dioxane et la DMF.

La deuxième étape est une réaction de réduction réalisée soit par hydrogénation en catalyse hétérogène, soit par transfert d'hydrogène ou encore par des hydrures métalliques ou encore par le couple acide formique-acide acétique en présence de palladium.

Par exemple, on utilise la méthode largement illustrée dans la littérature d'hydrogénation catalysée par le palladium (0), Pd (II), ou encore par du nickel de Raney ou PtO₂.

La réduction par transfert d'hydrogène faisant réagir du cyclohexène en présence de palladium s'avère également très efficace

Les exemples qui suivent servent à illustrer l'invention sans toutefois présenter un caractère limitatif.

### EXEMPLES DE SYNTHESE :

### Exemple n°1 : 2-[(3-amino-6-méthoxy-pyridin-2-yl)-méthyl-aminol-éthanol

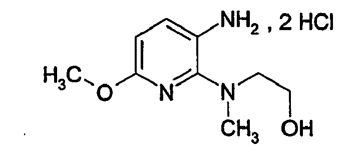

### A) Synthèse du 2-[(6-méthoxy-3-nitro-pyridin-2-yl)-méthyl-amino]-éthanol

Dans un ballon tout équipé, on charge 1,9 g (0,01 mole) de produit 2-chloro-3-nitro-6-méthoxypyridine, 30 ml de dioxane, 5 ml d'eau et 1,6 ml (0,02 mole) de 2-méthylaminoéthanol. Le mélange est porté au reflux pendant deux heures sous agitation puis le mélange est versé sur un mélange de glace/eau sous agitation. Le précipité formé est essoré et séché sous vide jusqu'à poids constant. On obtient 1,6 g de poudre jaune, soit un rendement égal à 70 %.

### B) Synthèse du 2-[(3-amino-6-méthoxy-pyridin-2-yl)-méthyl-amino]-éthanol

Dans un ballon tout équipé, on charge 1,6 g (0,007 mole) de produit 2-[(6-méthoxy-3-nitro-pyridin-2-yl)-méthyl-amino]-éthanol synthétisé selon le mode opératoire (A) ci-dessus, 15 ml d'éthanol, 5 ml de cyclohexène et 0,5 g de palladium sur charbon. Le mélange est porté au reflux pendant deux heures sous agitation puis le catalyseur est éliminé par filtration puis le filtrat est acidifié par de l'acide chlorhydrique. Après dilution à l'éther diisopropylique, le précipité formé est essoré et séché sous vide jusqu'à poids constant. On obtient 2,0 g de poudre, soit un rendement de 100 %.
L'analyse en spectrométrie de masse, en spectroscopie de résonance magnétique est conforme à la structure attendue.

| Analyse pondérale | | | | | |
|---|---|---|---|---|---|
| Trouvée : | C39,93 | H6,33 | N 15,52 | O 13,00 | Cl 24,93 |
| Calculée: | C40,01 | H6,34 | N 15,55 | O 11,84 | Cl 26,25 |

### Exemple 2: 2-[(3-amino-6-méthoxy-pyridin-2-yl)-butyl-amino]-éthanol

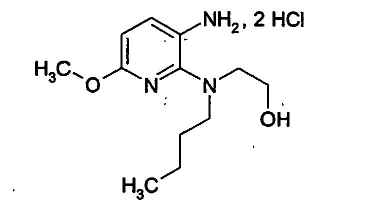

### A) Synthèse du 2-[butyl-(6-méthoxy-3-nitro-pyridin-2-yl)-amino]-éthanol

Dans un ballon tout équipé, on charge 1,9 g (0,01 mole) de produit 2-chloro-3-nitro-6-méthoxypyridine, 30 ml de dioxane, 5 ml d'eau et 2,62 ml (0,02 mole) de 2-butylaminoéthanol. Le mélange est porté au reflux pendant deux heures sous agitation puis le mélange est versé sur un mélange de glace/eau sous agitation. Le précipité formé est essoré et séché sous vide jusqu'à poids constant. On obtient 1,9 g de poudre jaune, soit un rendement de 70 %.

### B) Synthèse du 2-[(3-amino-6-méthoxy-pyridin-2-yl)-butyl-amino] éthanol

Dans un ballon tout équipé, on charge 1,9 g (0,007 mole) de produit 2-[butyl-(6-méthoxy-3-nitro-pyridin-2-yl)-amino]-éthanol synthétisé selon le mode opératoire (A) ci-dessus, 15 ml d'éthanol, 5 ml de cyclohexène et 0,5 g de palladium sur charbon. Le mélange est porté au reflux pendant deux heures sous agitation puis le catalyseur est éliminé par filtration puis le filtrat est acidifié par de l'acide chlorhydrique. Après dilution à l'éther diisopropylique le précipité formé est essoré et séché sous vide jusqu'à poids constant. On obtient 1,63 g de poudre, soit un rendement de 84,5 %.

L'analyse en spectrométrie de masse, en spectroscopie de résonance magnétique est conforme à la structure attendue.

### Exemple 3 : 2-[(3-amino-6-méthoxy-pyridin-2-yl)-(2-hydroxy-éthyl)-amino]-éthanol

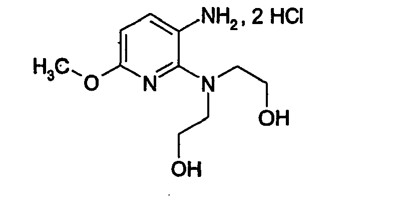

### A) Synthèse du 2-[(2-hydroxy-éthyl)-(6-méthoxy-3-nitro-pyridin-2-yl)-amino]-éthanol

Dans un ballon tout équipé, on charge 1,9 g (0,01 mole) de produit 2-chloro-3-nitro-6-méthoxypyridine, 30 ml de dioxane, 5 ml d'eau et 2,1 ml (0,02 mole) de diéthanolamine. Le mélange est porté au reflux pendant deux heures sous agitation puis le mélange est versé sur un mélange de glace/eau sous agitation. Le précipité formé est essoré et séché. On obtient 1,9 g de poudre jaune, soit un rendement de 74 %.

### B) Synthèse du 2-[(3-amino-6-méthoxy-pyridin-2-yl)-(2-hydroxy-éthyl)-amino]-éthanol

Dans un ballon tout équipé, on charge 2 g (0,0078 mole) de produit 2-[(2-hydroxy-éthyl)-(6-méthoxy-3-nitro-pyridin-2-yl)-amino]-éthanol synthétisé selon le mode opératoire (A) ci-dessus, 15 ml d'éthanol, 5 ml de cyclohexène et 0,5 g de palladium sur charbon. Le mélange est porté au reflux pendant deux heures sous agitation puis le catalyseur est éliminé par filtration puis le filtrat est acidifié par de l'acide chlorhydrique. Après dilution à l'ether diisopropylique, le précipité formé est essoré et séché sous vide jusqu'à poids constant. On obtient 2,21 g de poudre, soit un rendement de 100 %.
L'analyse en spectrométrie de masse, en spectroscopie de résonance magnétique est conforme à la structure attendue.

| Analyse: | | | | | |
|---|---|---|---|---|---|
| Trouvée : | C39,7 | H 6,46 | N13,73 | O16,26 | Cl 22,94 |
| Calculée : | C40,01 | H 6,38 | N14 | 015,99 | Cl 23,62 |

### Exemple 4 : 3-[(3-amino-6-méthoxy-pyridin-2-yl)-méthyl-amino]-propane-1,2-diol

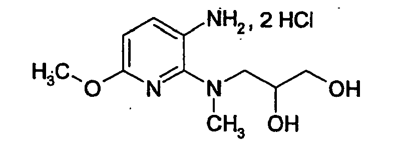

### A) Synthèse du 3-[(6-méthoxy-3-nitro-pyridin-2-yl)-méthyl-amino]-propane-1, 2-diol

Dans un ballon tout équipé, on charge 4 g (0,0219 mole) de produit 2-chloro-3-nitro-6-méthoxypyridine, 50 ml d'éthanol et 4,15 ml (0,0438 mole) de 3-méthylamino 1,2-dihydroxy propane. Le mélange est porté au reflux pendant deux heures sous agitation puis le mélange est versé sur un mélange de glace/eau sous agitation. Le précipité formé est essoré et séché sous vide jusqu'à poids constant. On obtient 4,74 g de poudre jaune, soit un rendement de 87,1 %.

### B) Synthèse du 3-[(3-amino-6-méthoxy-pyridin-2-yl)-méthyl-amino]-propane-1,2-diol

Dans un ballon tout équipé, on charge 4,65 g (0,018 mole) de produit 3-[(6-méthoxy-3-nitro-pyridin-2-yl)-méthyl-amino]-propane-1,2-diol synthétisé selon le mode opératoire (A) ci-dessus, 50 ml d'éthanol, 10 ml de cyclohexène et 2,1 g de palladium sur charbon: Le mélange est porté au reflux pendant deux heures sous agitation puis le catalyseur est éliminé par filtration puis le filtrat est acidifié par de l'acide chlorhydrique. Après dilution à l'éther diisopropylique le précipité formé est essoré et séché sous vide jusqu'à poids constant. On obtient 4,33 g de poudre, soit un rendement de 91 %.
L'analyse en spectrométrie de masse, en spectroscopie de résonance magnétique est conforme à la structure attendue.

| Analyse pondérale : | | | | | |
|---|---|---|---|---|---|
| Trouvée: | C 39,70 | H 6,71 | N10,87 | 019,65 | |
| Calculée: | C 40,01 | H 6,38 | N14 | 015,99 | Cl23,62 |

### Exemple 5 : 6-méthoxy-N2,N2-bis-(2-méthoxy-éthyl)-pyridine-2,3-diamine

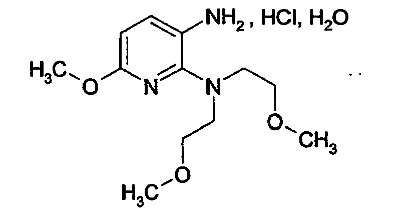

### A) Synthèse du bis-(2-méthoxy-éthyl)-(6-méthoxy-3-nitro-pyridin-2-yl)-amine

Dans un ballon tout équipé, on charge 4 g (0,0212 mole) de produit 2-chloro-3-nitro-6-méthoxypyridine, 50 ml d'éthanol et 6,32 ml (0,0424 mole) de bis-2-méthoxyéthylamine. Le mélange est porté au reflux pendant deux heures sous agitation puis le mélange est versé sur un mélange de glace/eau sous agitation. Le précipité formé est essoré et séché sous vide jusqu'à poids constant. On obtient 5,47 g de poudre jaune, soit un rendement de 90,6 %.

### B) Synthèse du 6-méthoxy-N2,N2-bis-(2-méthoxy-éthyl)-pyridine-2, 3-diamine

Dans un ballon tout équipé, on charge 5,3 g (0,0186 mole) de produit bis-(2-méthoxy-éthyl)-(6-méthoxy-3-nitro-pyridin-2-yl)-amine synthétisé selon le mode opératoire (A) ci-dessus, 40 ml d'éthanol, 15 ml de cyclohexène et 2,3 g de palladium sur charbon. Le mélange est porté au reflux pendant deux heures sous agitation puis le catalyseur est éliminé par filtration puis le filtrat est acidifié par de l'acide chlorhydrique. Après dilution à l'éther diisopropylique le précipité formé est essoré et séché sous vide jusqu'à poids constant. On obtient 4,96 g de poudre, soit un rendement de 91,3 %.
L'analyse en spectrométrie de masse, en spectroscopie de résonance magnétique est conforme à la structure attendue.

| Analyse pondérale : | | | | | |
|---|---|---|---|---|---|
| Trouvée : | C47,6 | H7,36 | N13,7 | O17,85 | CI13,45 |
| Calculée : | C46,53 | H7,81 | N13,56 | 020,66 | CI11,44 |

### Exemples 6 à 21

Les composés 6-alcoxy-2,3-diaminopyridine suivants ont été obtenus selon le procédé décrit pour l'exemple 1 B à partir des composés nitrés indiqués dans le tableau ci dessous.

| | **Composés nitrés** | **Composés 6-alcoxy-2,3-diaminopyridine selon l'invention** | **Masse trouvée** | **Masse théorique** |
|---|---|---|---|---|
| **Ex.6** | 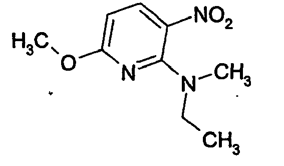 | 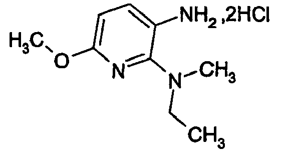 | 217 | 217,70 |
| **Ex.7** | 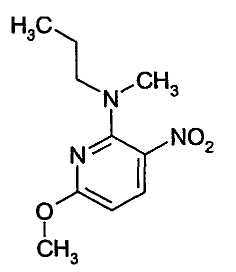 | 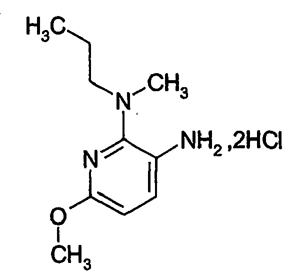 | 231 | 231,72 |
| **Ex.8** | 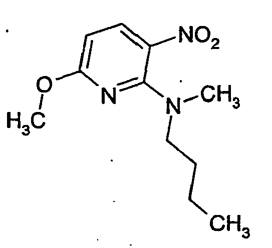 | 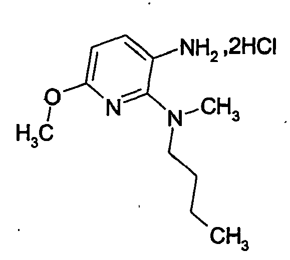 | 245 | 245,75 |
| **Ex.9** | 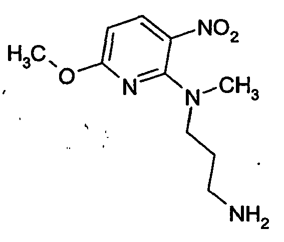 | 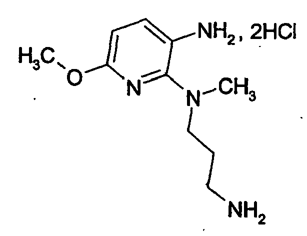 | 246 | 246.74 |
| **Ex. 10** | 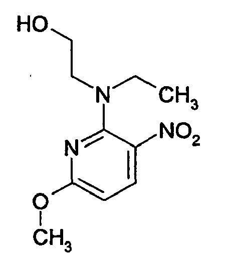 | 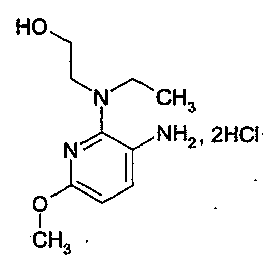 | 247 | 247,72 |
| **Ex.11** | 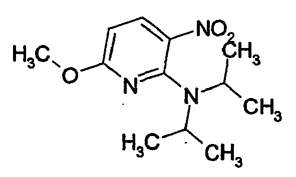 | 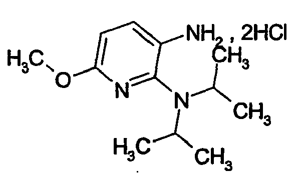 | 259 | 259,78 |
| **Ex.12** | 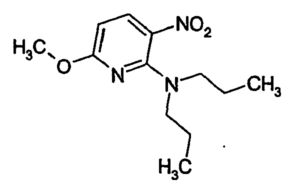 | 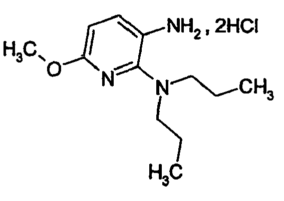 | 259 | 259,78 |
| **Ex.13** | 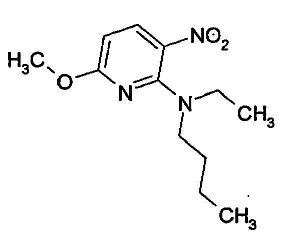 | 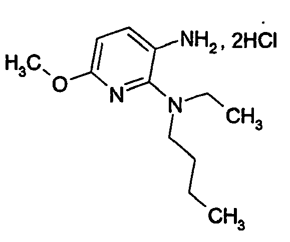 | 259 | 259,78 |
| **Ex.14** | 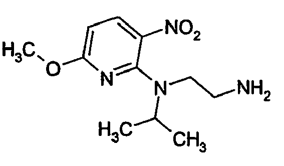 | 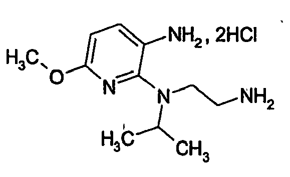 | 260 | 260,76 |
| **Ex.15** | 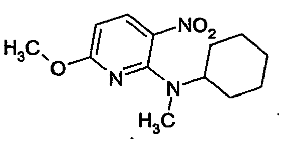 | 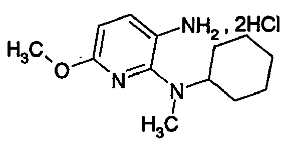 | 271 | 271,79 |
| **Ex.16** | 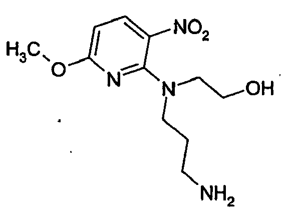 | 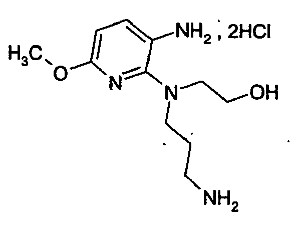 | 276 | 276,76 |
| **Ex.17** | 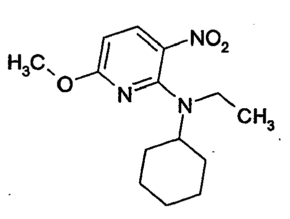 | 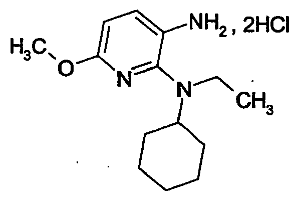 | 285 | 285,81 |
| **Ex.18** | 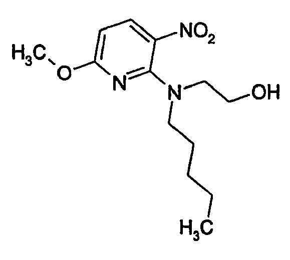 | 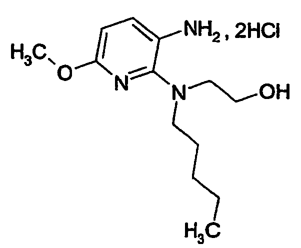 | 289 | 289.80 |
| **Ex.19** | 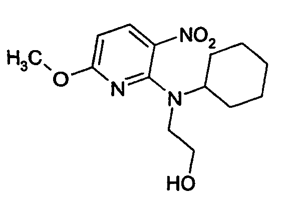 | 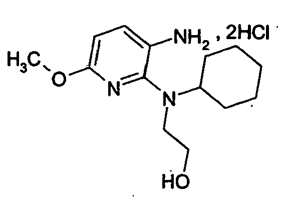 | 301 | 301,81 |
| **Ex.20** | 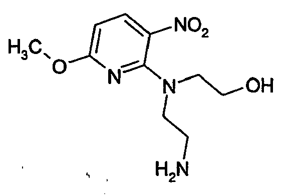 | 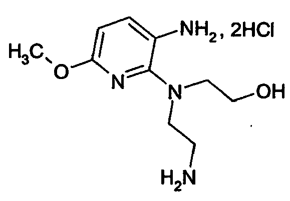 | 262 | 262,74 |
| **Ex.21** | 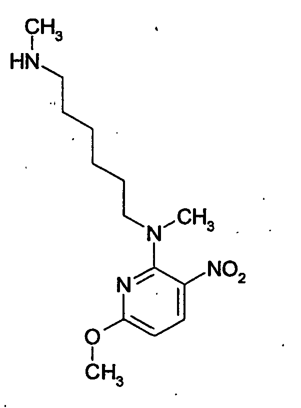 | 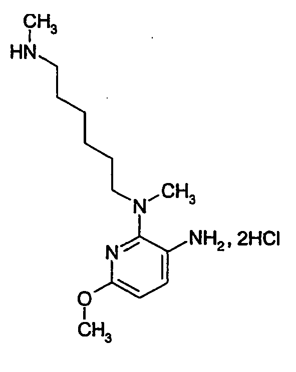 | 302 | 302,85 |

### EXEMPLES DE TEINTURE

### EXEMPLE 1 DE TEINTURE EN MILIEU ACIDE

On a préparé la composition tinctoriale suivante :

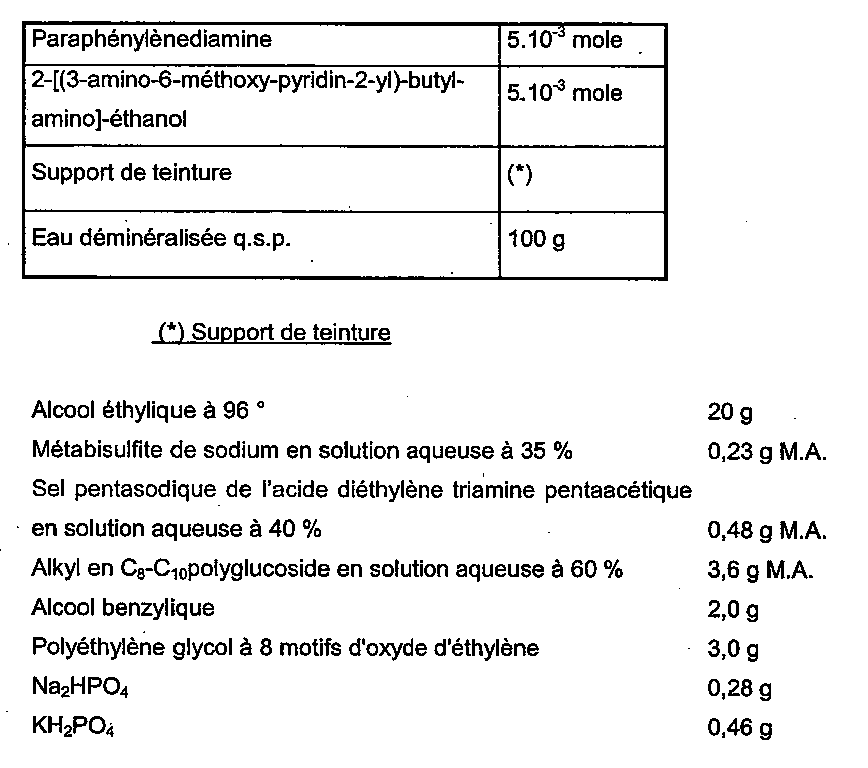

Au moment de l'emploi, la composition est mélangée avec un poids égal d'eau oxygénée à 20 volumes (6 % en poids). Le pH de la composition finale est égal à 7. Le mélange obtenu est appliqué sur des mèches de cheveux gris à 90 % de blancs. Après 30 minutes de pose, les mèches sont lavées avec un shampooing standard, rinçées puis séchées. Chaque mèche est évaluée avant et après la teinture dans le système L*a*b*, au moyen d'un spectrocolorimètre CM 2002 MINOLTA ®, (Illuminant D65).

Dans l'espace L*a*b*, la clarté est indiquée par la valeur L* sur une échelle de 0 à 100 alors que les coordonnées chromatiques sont exprimées par a* et b* qui indiquent deux axes de couleur, a* l'axe rouge-vert et b* l'axe jaune-bleu. Selon ce système, plus la valeur de L* est élevée, plus la couleur est claire et peu intense. Inversement, plus la valeur de L* est faible, plus la couleur est foncée ou très intense.

Les résultats de teinture suivants ont été obtenus :

| **Cheveux naturels** | | | **Cheveux permanentés** | | |
|---|---|---|---|---|---|
| **L*** | **a*** | **b*** | **L*** | **a*** | **b*** |
| 16,44 | 2,03 | -5,09 | 9,51 | 1,97 | -2,97 |

### EXEMPLES 2 A 7 DE TEINTURE EN MILIEU ALCALIN

On a préparé les compositions tinctoriales dans les proportions suivantes :

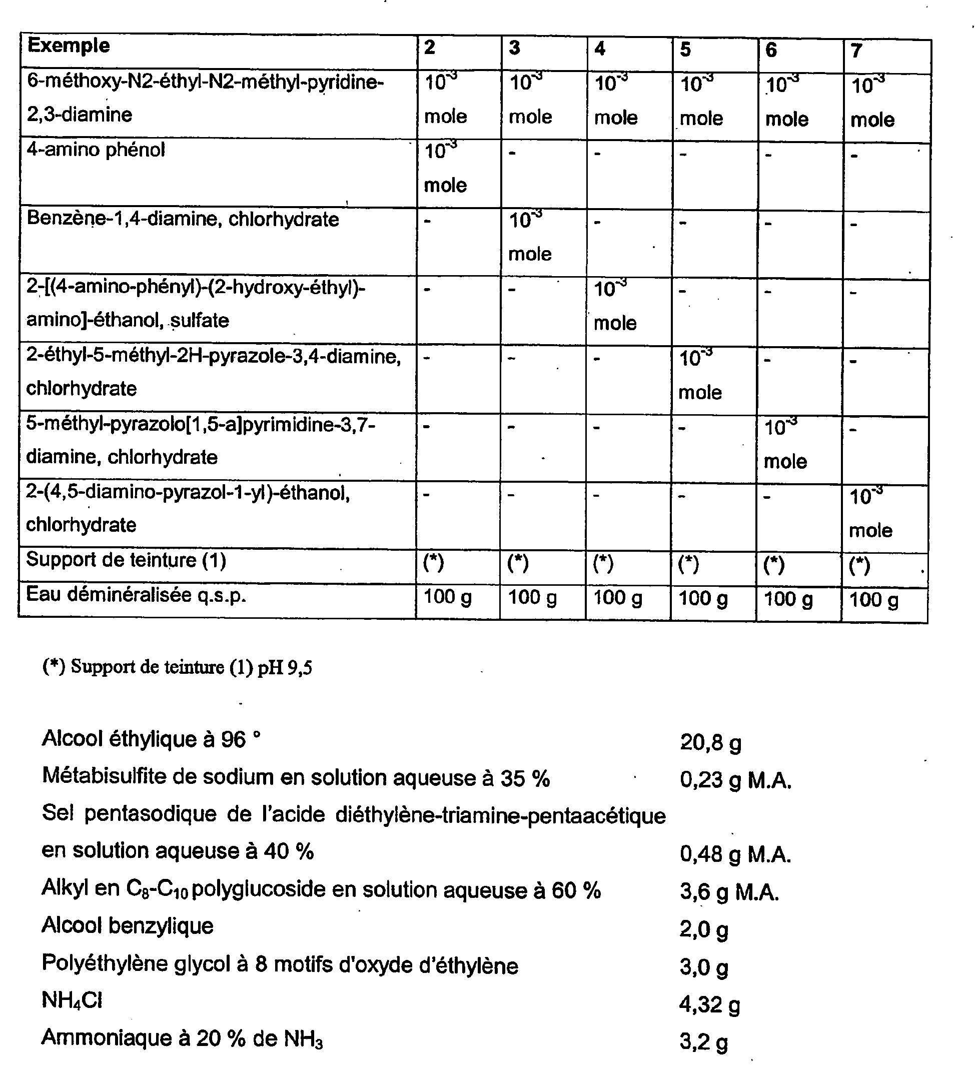

Au moment de l'emploi, chaque composition est mélangée avec un poids égal d'eau oxygénée à 20 volumes (6 % en poids). On obtient un pH final de 9,5.

Chaque mélange obtenu est appliqué sur des cheveux gris à 90 % de blancs. Après 30 minutes de pose, les cheveux sont rincés, lavés avec un shampooing standard, rincés à nouveau puis séchés.

Les résultats de teinture suivants ont été obtenus.

| **Exemple** | **2** | **3** | **4** | **5** | **6** | **7** |
|---|---|---|---|---|---|---|
| **Nuance observée** | Brun rouge | Gris intense | Gris bleu intense | Gris | Violet intense | Violet intense |

### EXEMPLES 8 A 11 DE TEINTURE EN MILIEU ALCALIN

On a préparé les compositions tinctoriales dans les proportions suivantes :

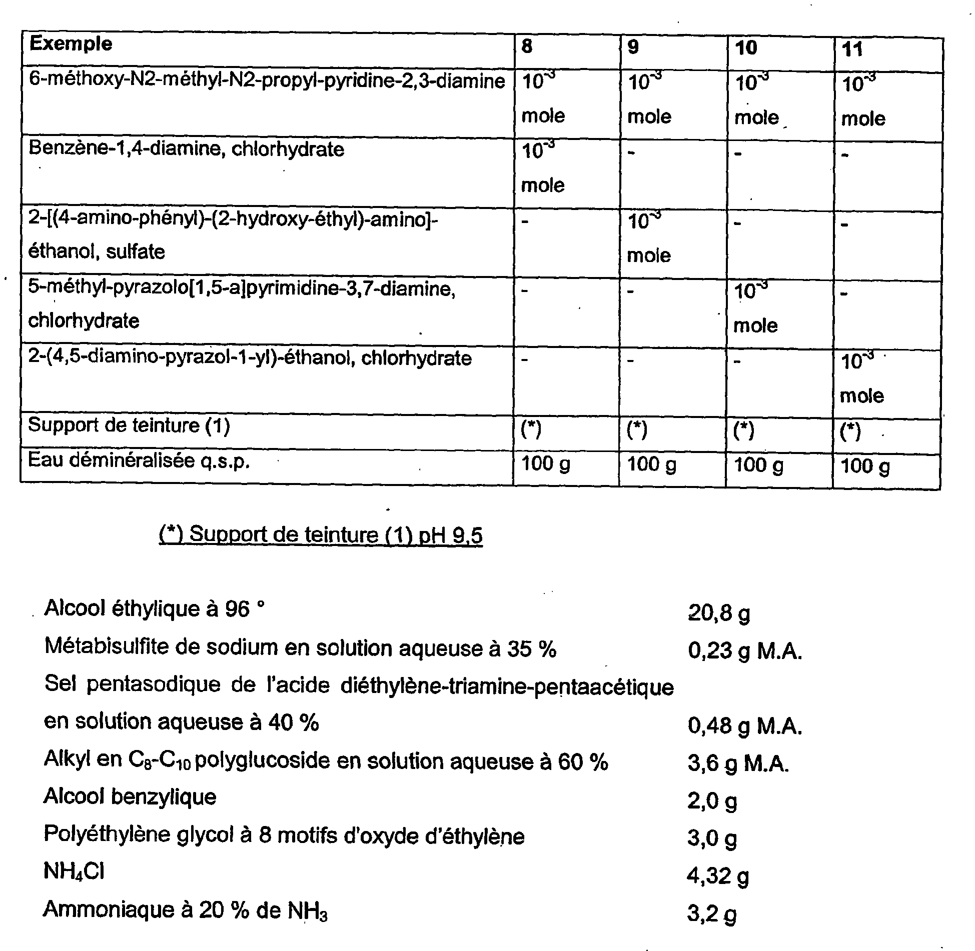

Au moment de l'emploi, chaque composition est mélangée avec un poids égal d'eau oxygénée à 20 volumes (6 % en poids). On obtient un pH final de 9,5.

Chaque mélange obtenu est appliqué sur des cheveux gris à 90 % de blancs. Après 30 minutes de pose, les cheveux sont rincés, lavés avec un shampooing standard, rincés à nouveau puis séchés.

Les résultats de teinture suivants ont été obtenus.

| **Exemple** | **8** | **9** | **10** | **11** |
|---|---|---|---|---|
| **Nuance observée** | Brun rouge | Brun | Violet-rouge | Rouge |

### EXEMPLES 12 A 15 DE TEINTURE EN MILIEU ALCALIN

On a préparé les compositions tinctoriales dans les proportions suivantes :

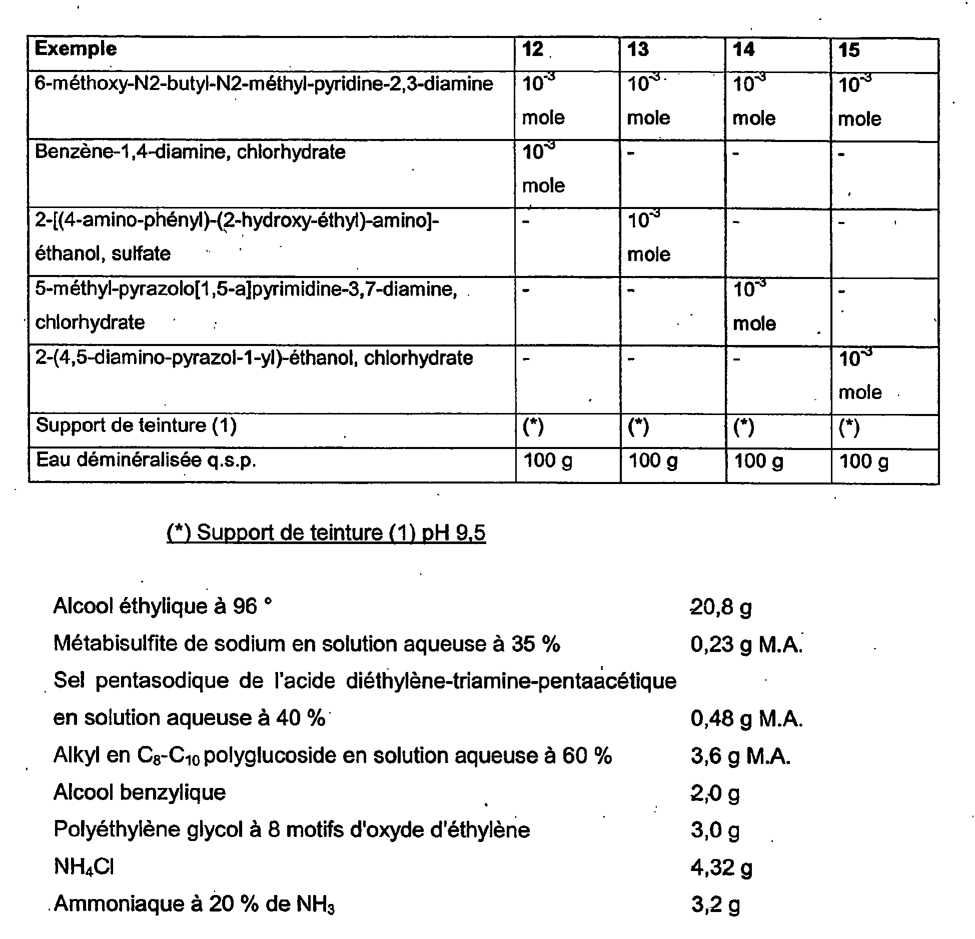

Au moment de l'emploi, chaque composition est mélangée avec un poids égal d'eau oxygénée à 20 volumes (6 % en poids). On obtient un pH final de 9,5.

Chaque mélange obtenu est appliqué sur des cheveux gris à 90 % de blancs. Après 30 minutes de pose, les cheveux sont rincés, lavés avec un shampooing standard, rincés à nouveau puis séchés.

Les résultats de teinture suivants ont été obtenus.

| **Exemple** | **12** | **13** | **14** | **15** |
|---|---|---|---|---|
| **Nuance observée** | Brun intense | Gris | Violet intense | Brun rouge |

### EXEMPLES 16 A 21 DE TEINTURE EN MILIEU ALCALIN

On a préparé les compositions tinctoriales dans les proportions suivantes :

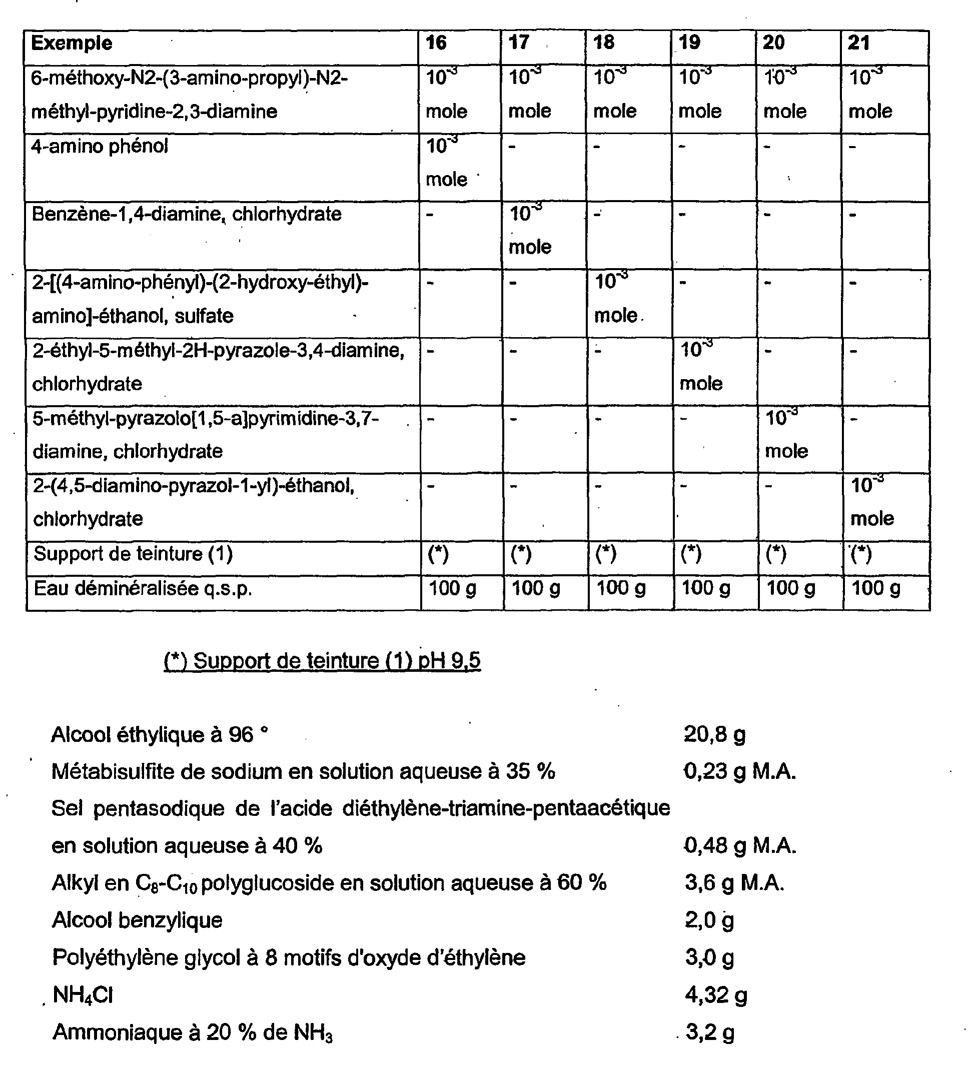

Au moment de l'emploi, chaque composition est mélangée avec un poids égal d'eau oxygénée à 20 volumes (6 % en poids): On obtient un pH final de 9,5.

Chaque mélange obtenu est appliqué sur des cheveux gris à 90 % de blancs. Après 30 minutes de pose, les cheveux sont rincés, lavés avec un shampooing standard, rincés à nouveau puis séchés.

Les résultats de teinture suivants ont été obtenus.

| **Exemple** | **16** | **17** | **18** | **19** | **20** | **21** |
|---|---|---|---|---|---|---|
| **Nuance observée** | Brun orangé | Gris violet intense | Bleu intense | Gris | Gris violet intense | Violet intense |

### EXEMPLES 22 A 23 DE TEINTURE EN MILIEU ALCALIN

On a préparé les compositions tinctoriales dans les proportions suivantes :

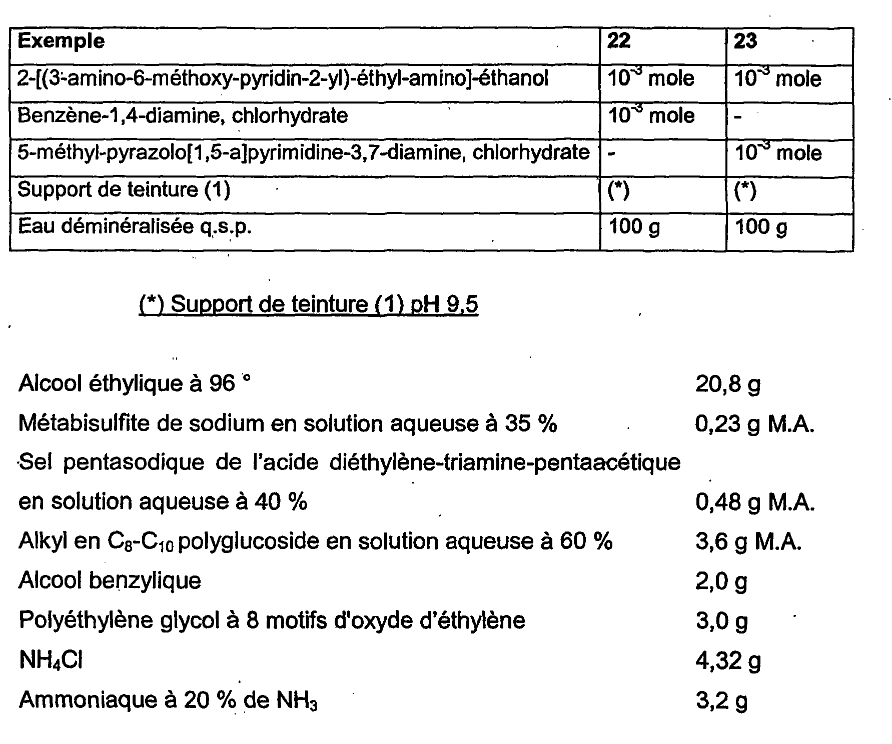

Au moment de l'emploi, chaque composition est mélangée avec un poids égal d'eau oxygénée à 20 volumes (6 % en poids). On obtient un pH final de 9,5.

Chaque mélange obtenu est appliqué sur des cheveux gris à 90 % de blancs. Après 30 minutes de pose, les cheveux sont rincés, lavés avec un shampooing standard, rincés à nouveau puis séchés.

Les résultats de teinture suivants ont été obtenus.

| **Exemple** | **22** | **23** |
|---|---|---|
| **Nuance observée** | Brun orangé | Violet-rouge |

### EXEMPLES 24 A 25 DE TEINTURE EN MILIEU ALCALIN

On a préparé les compositions tinctoriales dans les proportions suivantes :

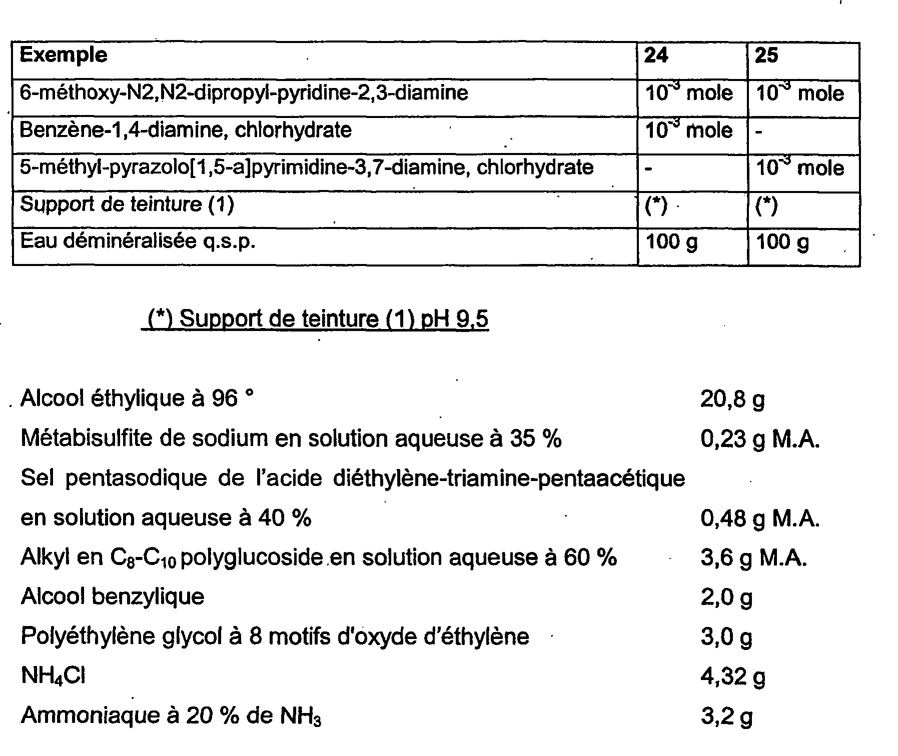

Au moment de l'emploi, chaque composition est mélangée avec un poids égal d'eau oxygénée à 20 volumes (6 % en poids). On obtient un pH final de 9,5.

Chaque mélange obtenu est appliqué sur des cheveux gris à 90 % de blancs. Après 30 minutes de pose, les cheveux sont rincés, lavés avec un shampooing standard, rincés à nouveau puis séchés.

Les résultats de teinture suivants ont été obtenus.

| **Exemple** | **24** | **25** |
|---|---|---|
| **Nuance observée** | Brun rouge | Brun rouge |

### EXEMPLES 26 A 29 DE TEINTURE EN MILIEU ALCALIN

On a préparé les compositions tinctoriales dans les proportions suivantes :

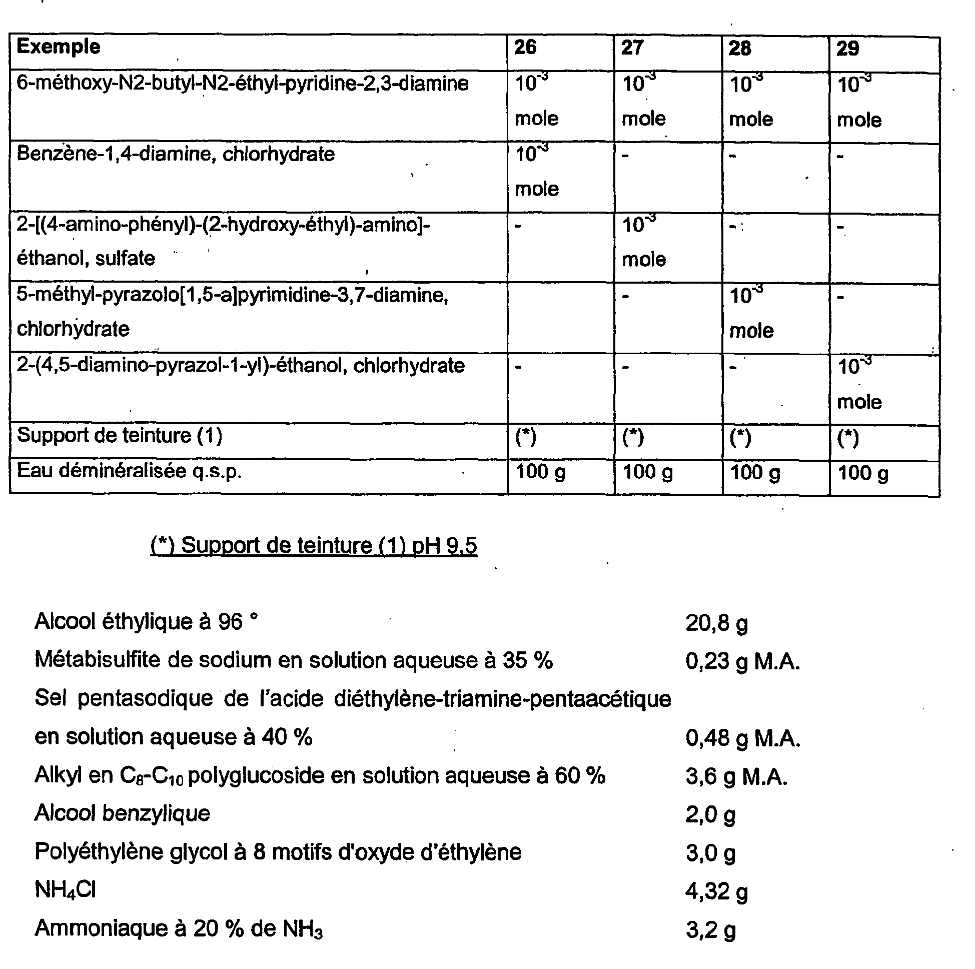

Au moment de l'emploi, chaque composition est mélangée avec un poids égal d'eau oxygénée à 20 volumes (6 % en poids). On obtient un pH final de 9,5.

Chaque mélange obtenu est appliqué sur des cheveux gris à 90 % de blancs. Après 30 minutes de pose, les cheveux sont rincés, lavés avec un shampooing standard, rincés à nouveau puis séchés.

Les résultats de teinture suivants ont été obtenus.

| **Exemple** | **26** | **27** | **28** | **29** |
|---|---|---|---|---|
| **Nuance observée** | Brun | Gris | Violet intense | Gris rouge |

### EXEMPLES 30 A 35 DE TEINTURE EN MILIEU ALCALIN

On a préparé les compositions tinctoriales dans les proportions suivantes :

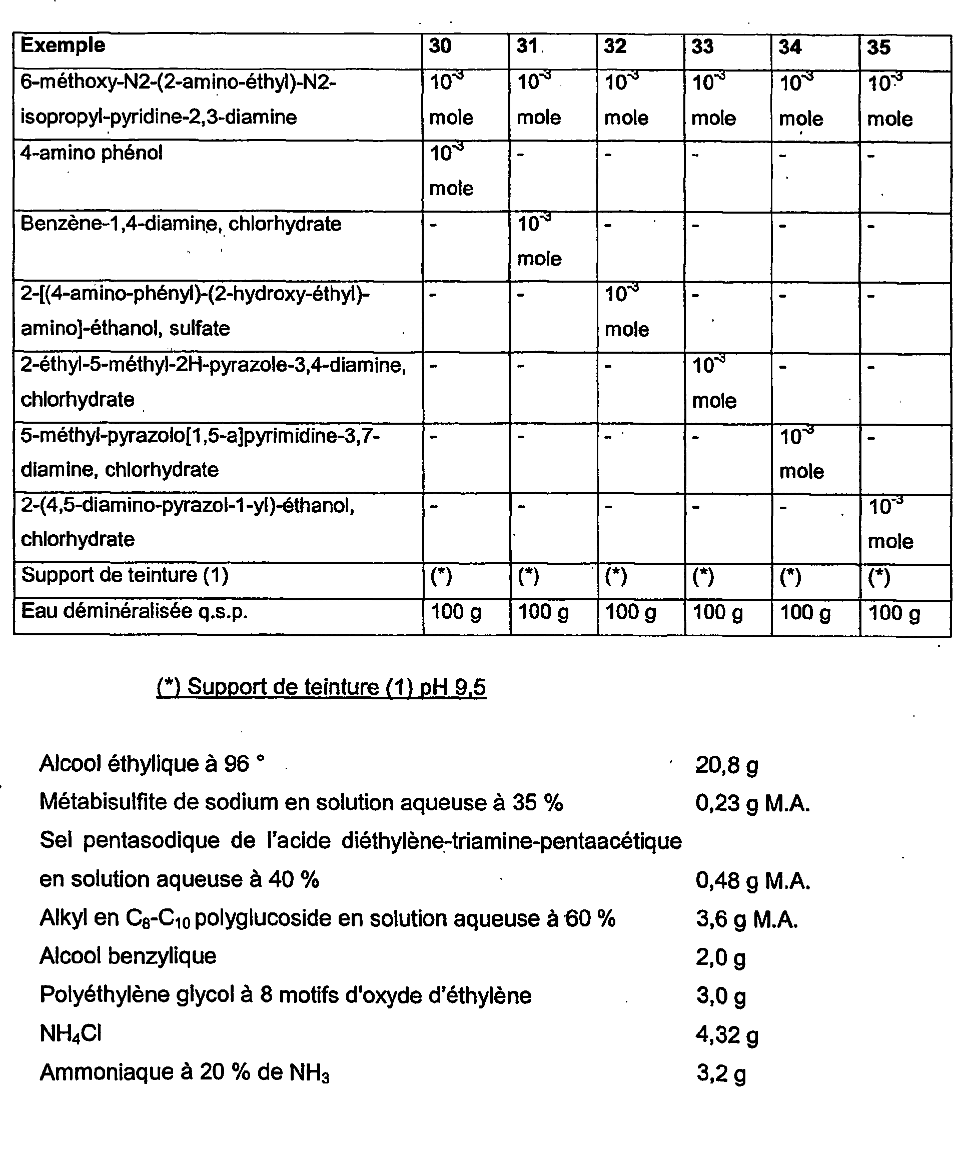

Au moment de l'emploi, chaque composition est mélangée avec un poids égal d'eau oxygénée à 20 volumes (6 % en poids). On obtient un pH final de 9,5.

Chaque mélange obtenu est appliqué sur des cheveux gris à 90 % de blancs. Après 30 minutes de pose, les cheveux sont rincés, lavés avec un shampooing standard, rincés à nouveau puis séchés.

Les résultats de teinture suivants ont été obtenus.

| **Exemple** | **30** | **31** | **32** | **33** | **34** | **35** |
|---|---|---|---|---|---|---|
| **Nuance observée** | Orangé | Gris intense | Gris vert-bleu intense | Brun jaune | Gris violet intense | Gris violet |

### EXEMPLES 36 A 39 DE TEINTURE EN MILIEU ALCALIN

On a préparé les compositions tinctoriales dans les proportions suivantes :

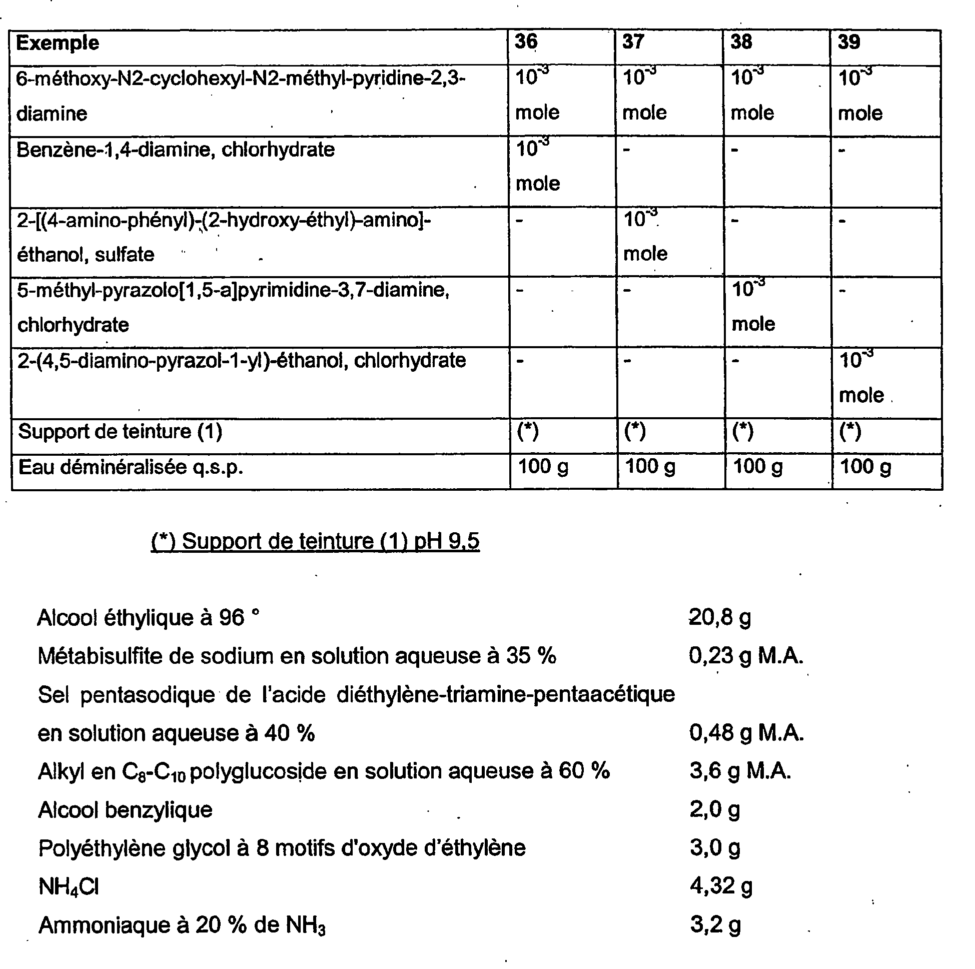

Au moment de l'emploi, chaque composition est mélangée avec un poids égal d'eau oxygénée à 20 volumes (6 % en poids). On obtient un pH final de 9,5.

Chaque mélange obtenu est appliqué sur des cheveux gris à 90 % de blancs. Après 30 minutes de pose, les cheveux sont rincés, lavés avec un shampooing standard, rincés à nouveau puis séchés.

Les résultats de teinture suivants ont été obtenus.

| **Exemple** | **36** | **37** | **38** | **39** |
|---|---|---|---|---|
| **Nuance observée** | Brun rouge intense | Gris | Gris violet intense | Brun rouge |

### EXEMPLES 40 A 42 DE TEINTURE EN MILIEU ALCALIN

On a préparé les compositions tinctoriales dans les proportions suivantes :

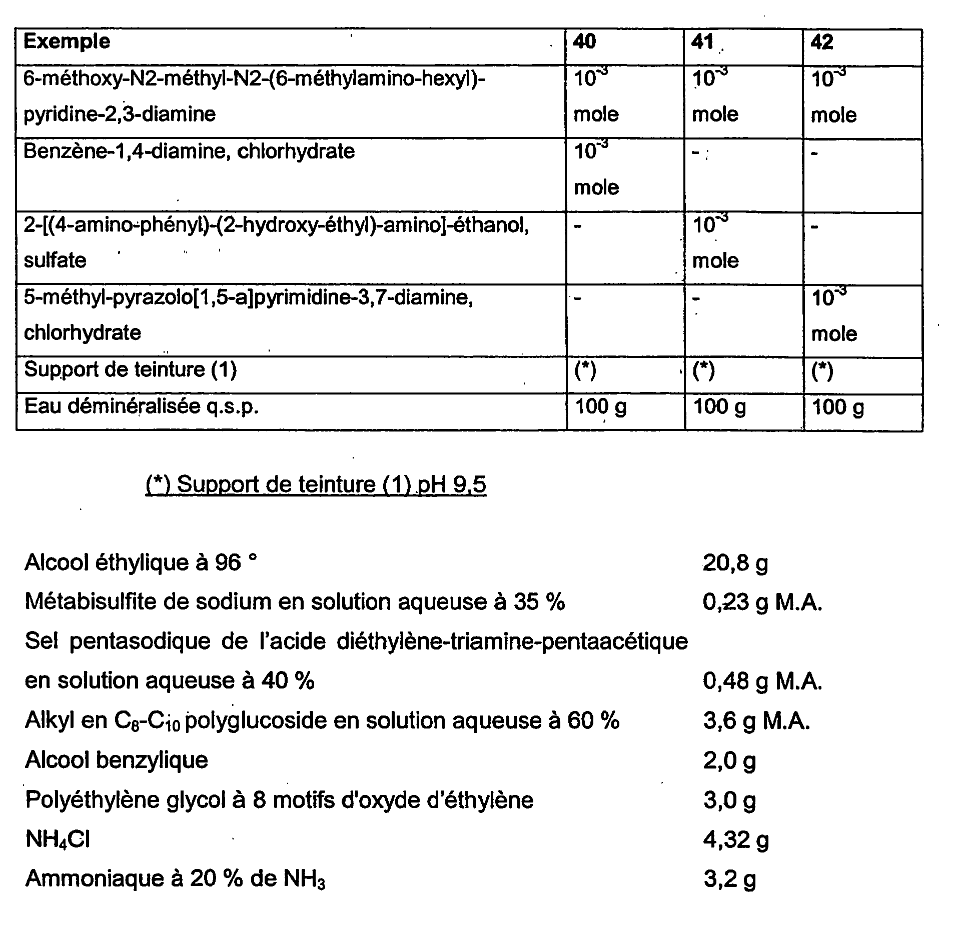

Au moment de l'emploi, chaque composition est mélangée avec un poids égal d'eau oxygénée à 20 volumes (6 % en poids). On obtient un pH final de 9,5.

Chaque mélange obtenu est appliqué sur des cheveux gris à 90 % de blancs. Après 30 minutes de pose, les cheveux sont rincés, lavés avec un shampooing standard, rincés à nouveau puis séchés.

Les résultats de teinture suivants ont été obtenus.

| **Exemple** | **40** | **41** | **42** |
|---|---|---|---|
| **Nuance observée** | Brun | Gris | Violet-rouge intense |

### EXEMPLES 43 A 46 DE TEINTURE EN MILIEU ALCALIN

On a préparé les compositions tinctoriales dans les proportions suivantes :

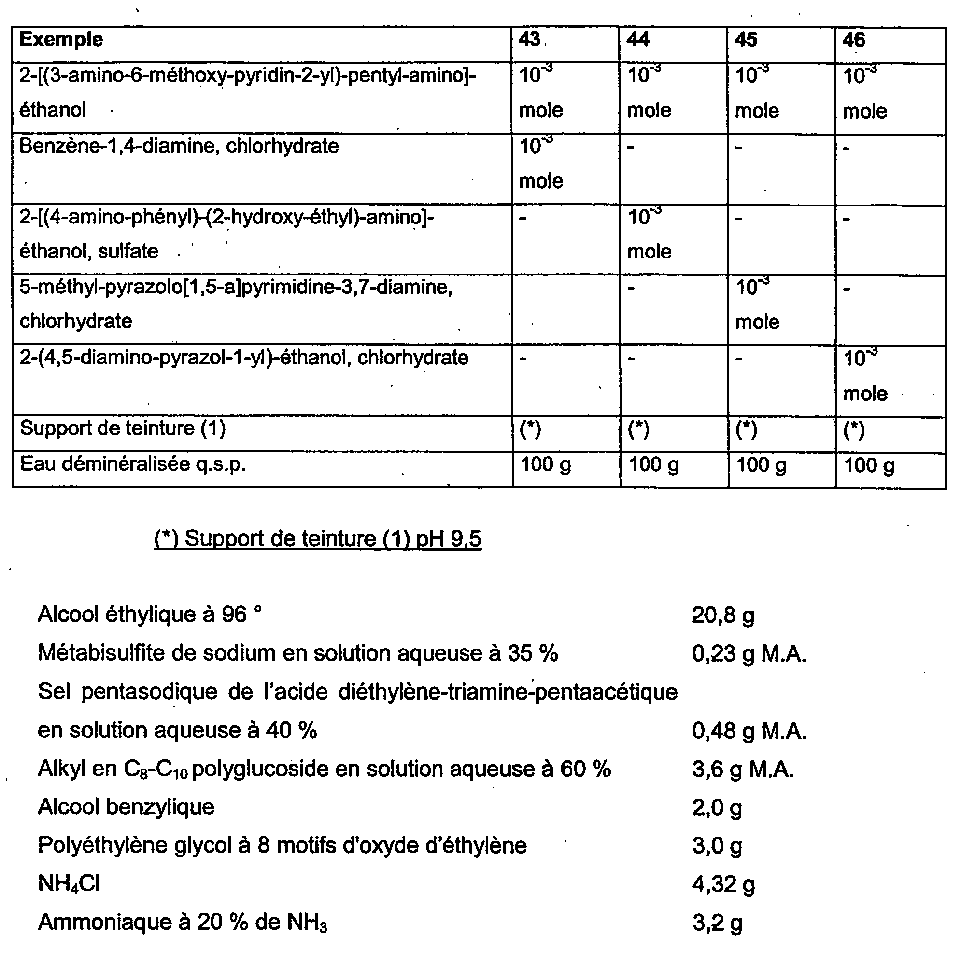

Au moment de l'emploi, chaque composition est mélangée avec un poids égal d'eau oxygénée à 20 volumes (6 % en poids). On obtient un pH final de 9,5.

Chaque mélange obtenu est appliqué sur des cheveux gris à 90 % de blancs. Après 30 minutes de pose, les cheveux sont rincés, lavés avec un shampooing standard, rincés à nouveau puis séchés.

Les résultats de teinture suivants ont été obtenus.

| **Exemple** | **43** | **44** | **45** | **46** |
|---|---|---|---|---|
| **Nuance observée** | Brun | Gris jaune-vert | Gris violet-rouge intense | Brun rouge |

### EXEMPLES 47 A 52 DE TEINTURE EN MILIEU ALCALIN

On a préparé les compositions tinctoriales dans les proportions suivantes :

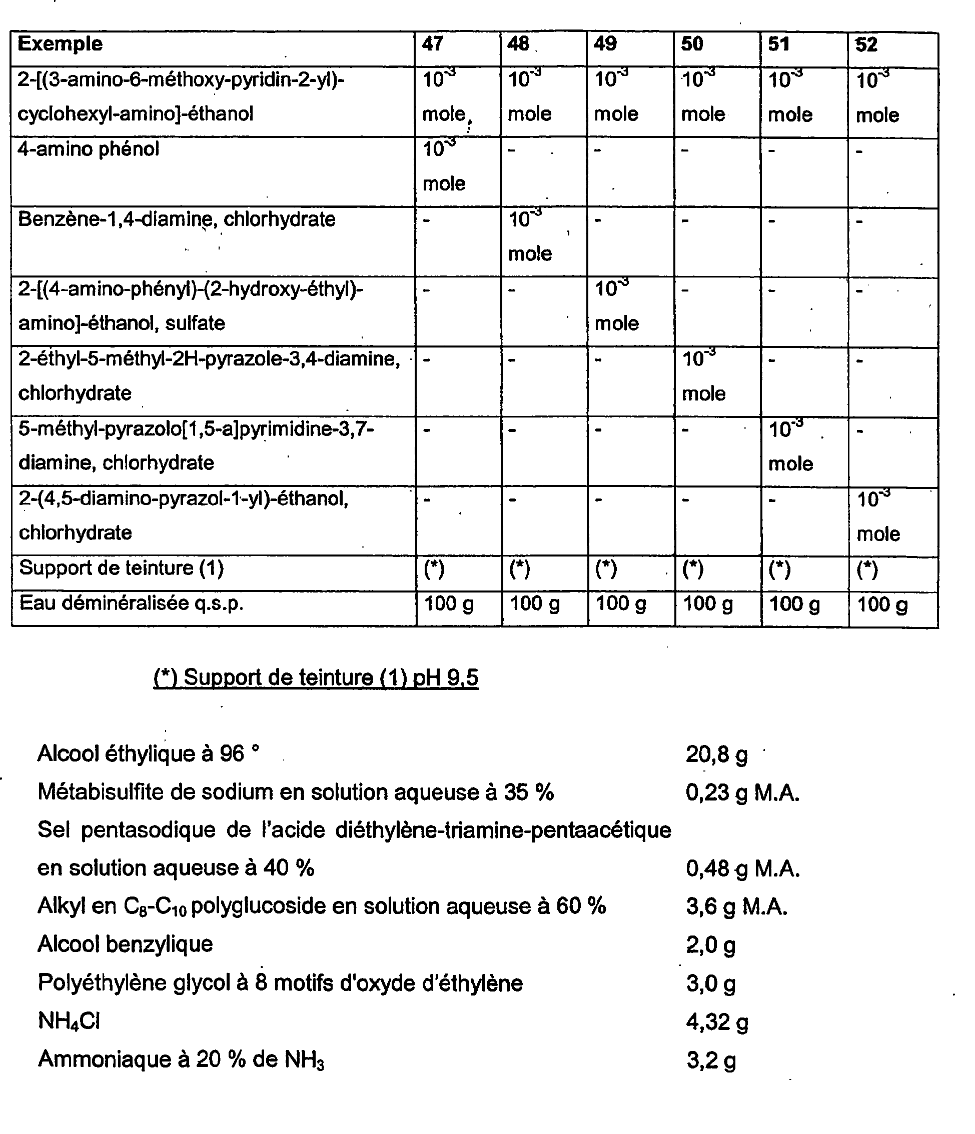

Au moment de l'emploi, chaque composition est mélangée avec un poids égal d'eau oxygénée à 20 volumes (6 % en poids). On obtient un pH final de 9,5.

Chaque mélange obtenu est appliqué sur des cheveux gris à 90 % de blancs. Après 30 minutes de pose, les cheveux sont rincés, lavés avec un shampooing standard, rincés à nouveau puis séchés.

Les résultats de teinture suivants ont été obtenus.

| **Exemple** | **47** | **48** | **49** | **50** | **51** | **52** |
|---|---|---|---|---|---|---|
| **Nuance observée** | Rouge | Gris intense | Gris vert-bleu intense | Gris | Gris violet intense | Gris violet |

### EXEMPLES 53 A 57 DE TEINTURE EN MILIEU ALCALIN

On a préparé les compositions tinctoriales dans les proportions suivantes :

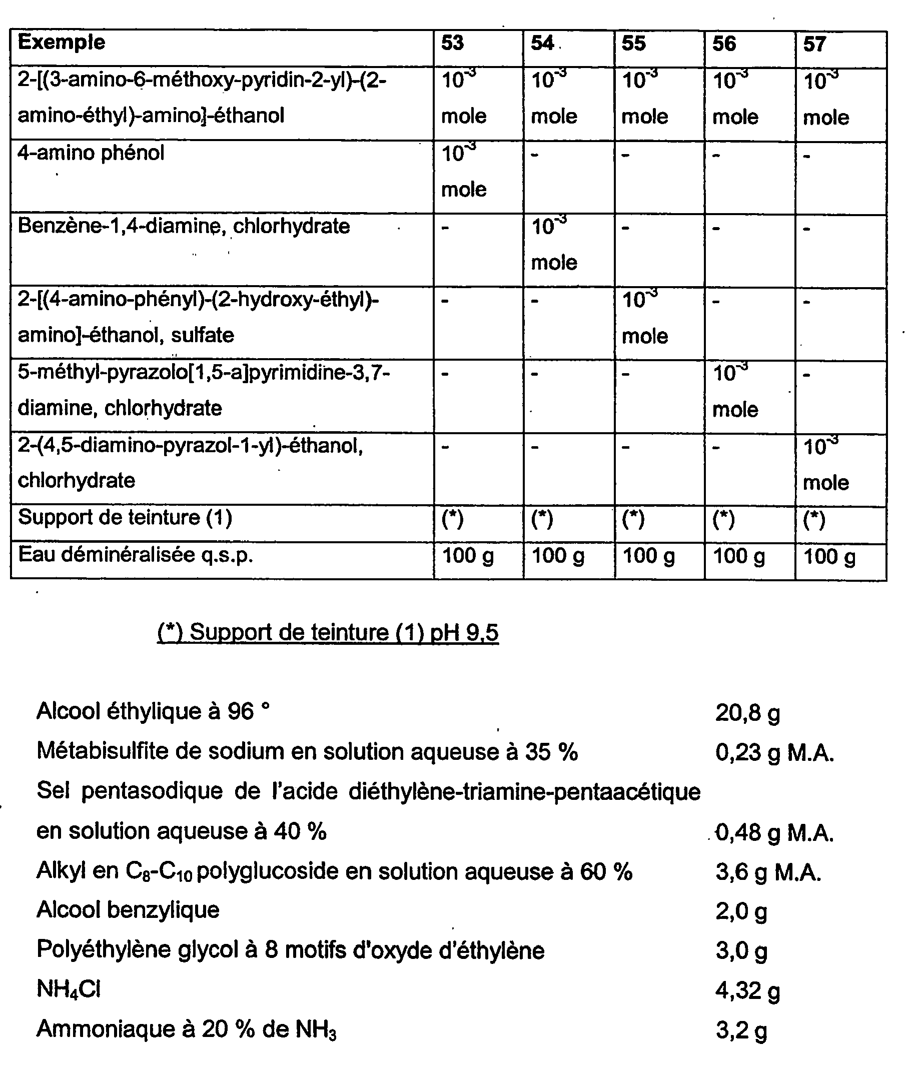

Au moment de l'emploi, chaque composition est mélangée avec un poids égal d'eau oxygénée à 20 volumes (6 % en poids). On obtient un pH final de 9,5.

Chaque mélange obtenu est appliqué sur des cheveux gris à 90 % de blancs. Après 30 minutes de pose, les cheveux sont rincés, lavés avec un shampooing standard, rincés à nouveau puis séchés.

Les résultats de teinture suivants ont été obtenus.

| **Exemple** | **53** | **54** | **55** | **56** | **57** |
|---|---|---|---|---|---|
| **Nuance observée** | Orangé | Gris intense | Gris vert-bleu | Gris violet intense | Gris violet |

### EXEMPLES 58 A 61 DE TEINTURE EN MILIEU ALCALIN

On a préparé les compositions tinctoriales dans les proportions suivantes :

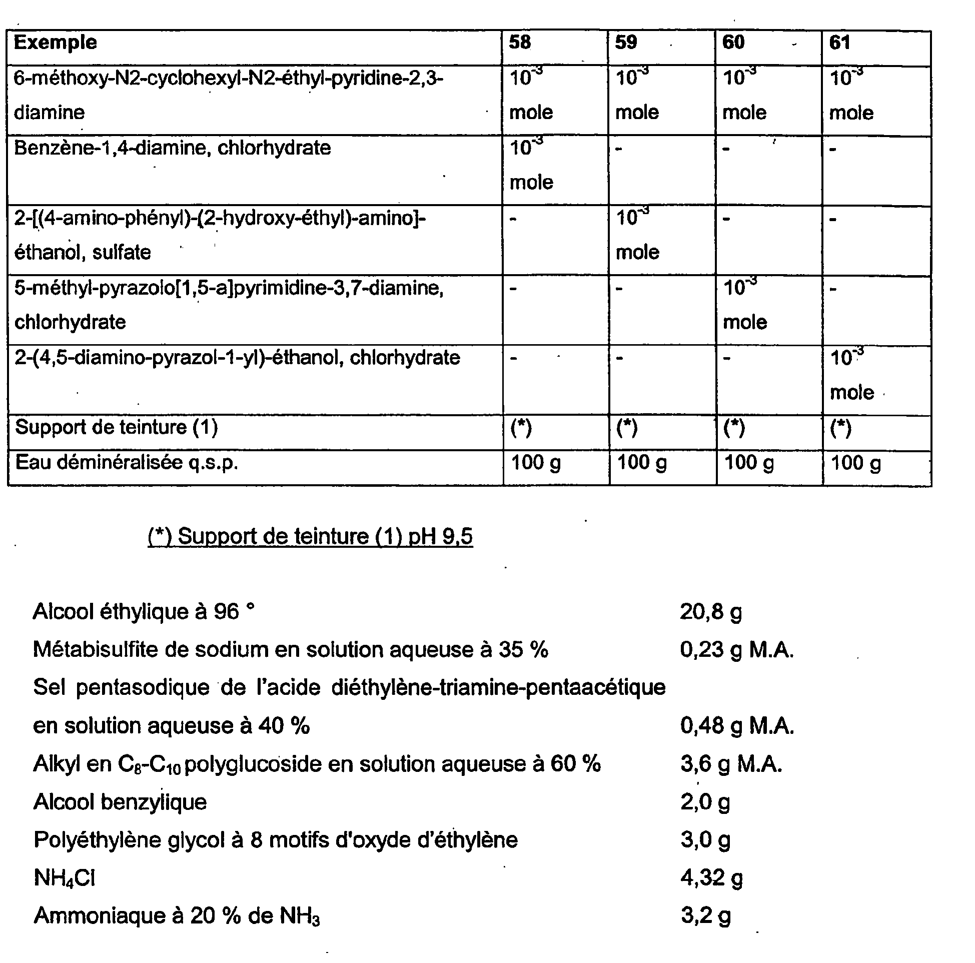

Au moment de l'emploi, chaque composition est mélangée avec un poids égal d'eau oxygénée à 20 volumes (6 % en poids). On obtient un pH final de 9,5.

Chaque mélange obtenu est appliqué sur des cheveux gris à 90 % de blancs. Après 30 minutes de pose, les cheveux sont rincés, lavés avec un shampooing standard, rincés à nouveau puis séchés.

Les résultats de teinture suivants ont été obtenus.

| **Exemple** | **58** | **59** | **60** | **61** |
|---|---|---|---|---|
| **Nuance observée** | Brun rouge | Brun | Gris rouge | Rouge |

### EXEMPLES 62 A 68 DE TEINTURE EN MILIEU ACIDE

On a préparé les compositions tinctoriales dans les proportions suivantes :

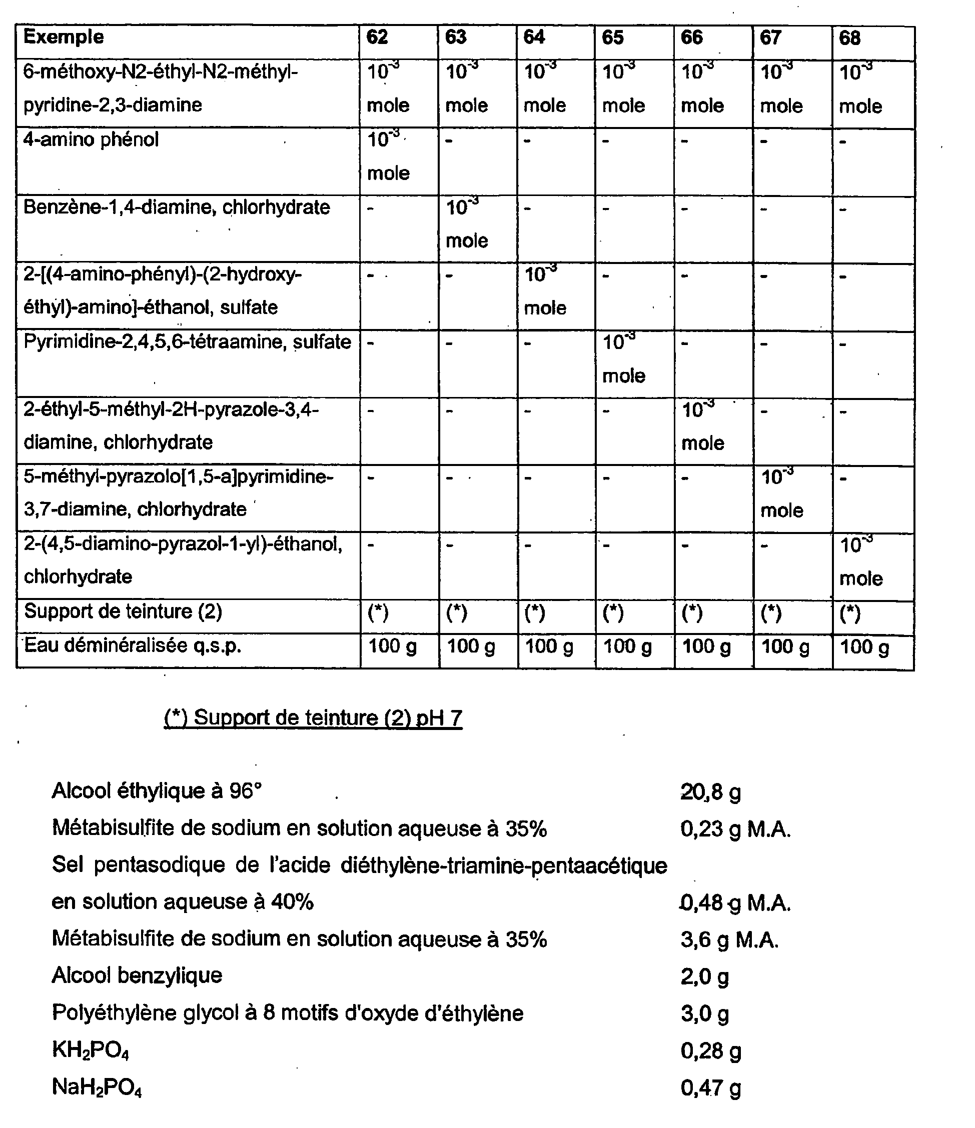

Au moment de l'emploi, chaque composition est mélangée avec un poids égal d'eau oxygénée à 20 volumes (6 % en poids). On obtient un pH final de 7.

Chaque mélange obtenu est appliqué sur des cheveux gris à 90 % de blancs. Après 30 min de pose, les cheveux sont rincés, lavés avec un shampooing standard, rincés à nouveau puis séchés.

Les résultats de teinture suivants ont été obtenus.

| **Exemple** | **62** | **63** | **64** | **65** | **66** | **67** | **68** |
|---|---|---|---|---|---|---|---|
| **Nuance observée** | Orangé | Brun intense | Gris intense | Brun jaune | Gris | Gris intense | Brun intense |

### EXEMPLES 69 A 75 DE TEINTURE EN MILIEU ACIDE

On a préparé les compositions tinctoriales dans les proportions suivantes :

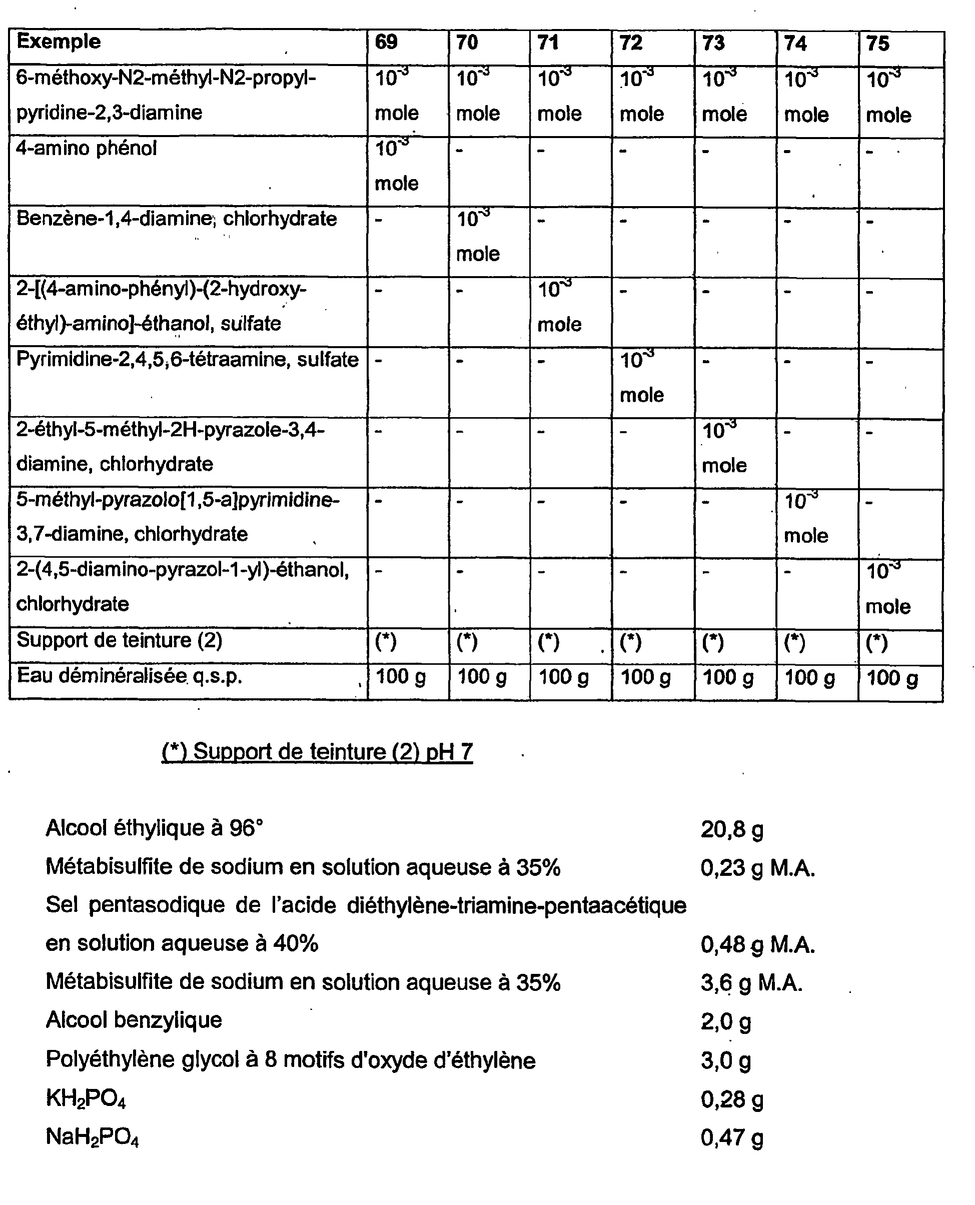

Au moment de l'emploi, chaque composition est mélangée avec un poids égal d'eau oxygénée à 20 volumes (6 % en poids). On obtient un pH final de 7.

Chaque mélange obtenu est appliqué sur des cheveux gris à 90 % de blancs. Après 30 min de pose, les cheveux sont rincés, lavés avec un shampooing standard, rincés à nouveau puis séchés.

Les résultats de teinture suivants ont été obtenus.

| **Exemple** | **69** | **70** | **71** | **72** | **73** | **74** | **75** |
|---|---|---|---|---|---|---|---|
| **Nuance observée** | Orangé | Gris . bleu intense | Vert-bleu intense | Jaune | Gris intense | Gris intense | Gris violet intense |

### EXEMPLES 76 A 82 DE TEINTURE EN MILIEU ACIDE

On a préparé les compositions tinctoriales dans les proportions suivantes :

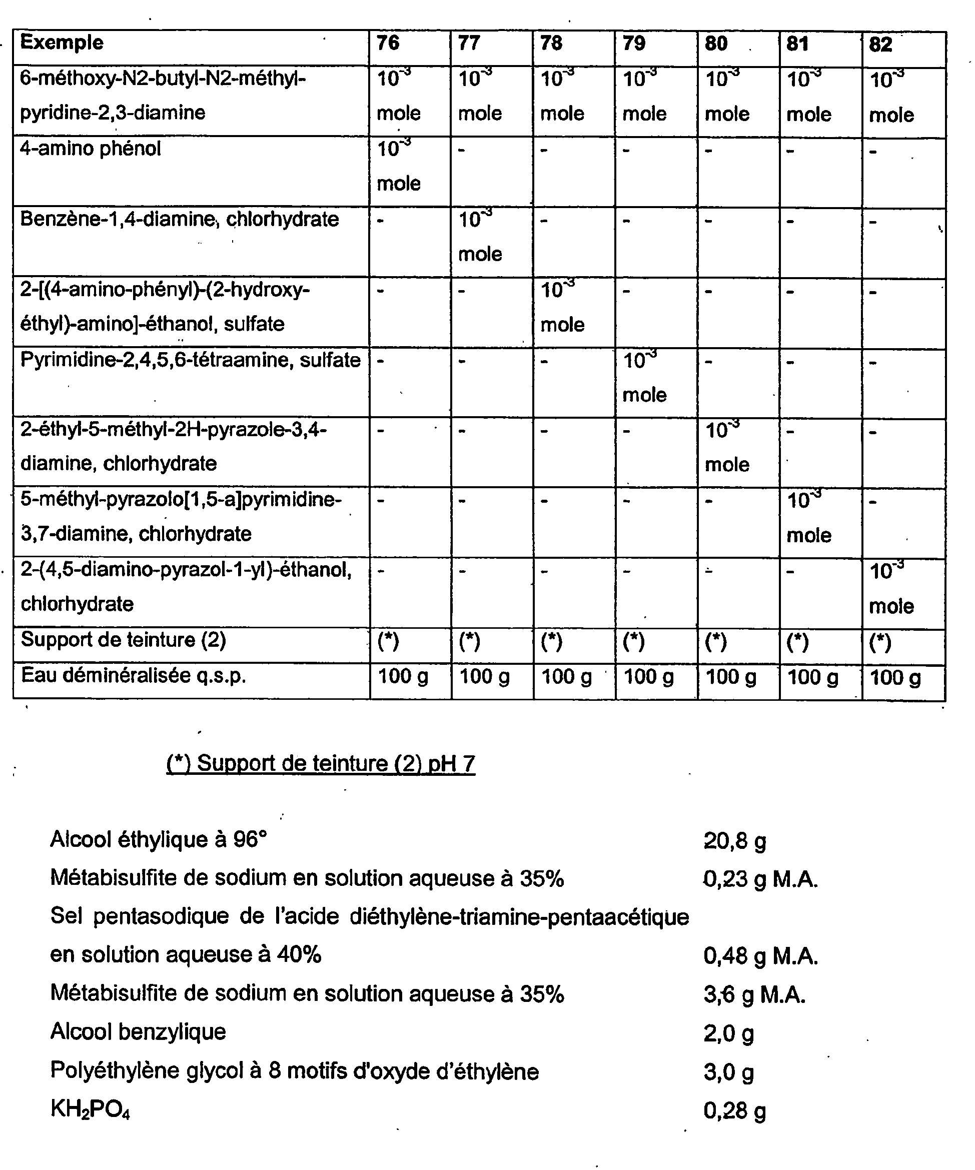

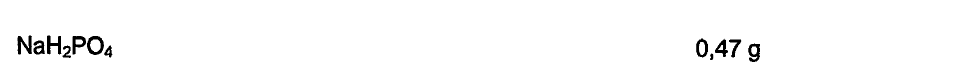

Au moment de l'emploi, chaque composition est mélangée avec un poids égal d'eau oxygénée à 20 volumes (6 % en poids). On obtient un pH final de 7.

Chaque mélange obtenu est appliqué sur des cheveux gris à 90 % de blancs. Après 30 min de pose, les cheveux sont rincés, lavés avec un shampooing standard, rincés à nouveau puis séchés.

Les résultats de teinture suivants ont été obtenus.

| **Exemple** | **76** | **77** | **78** | **79** | **80** | **81** | **82** |
|---|---|---|---|---|---|---|---|
| **Nuance observée** | Orangé | Gris bleu intense | Vert-bleu intense | Brun jaune | Gris | Gris violet intense | Gris violet intense |

### EXEMPLES 83 A 89 DE TEINTURE EN MILIEU ACIDE

On a préparé les compositions tinctoriales dans les proportions suivantes :

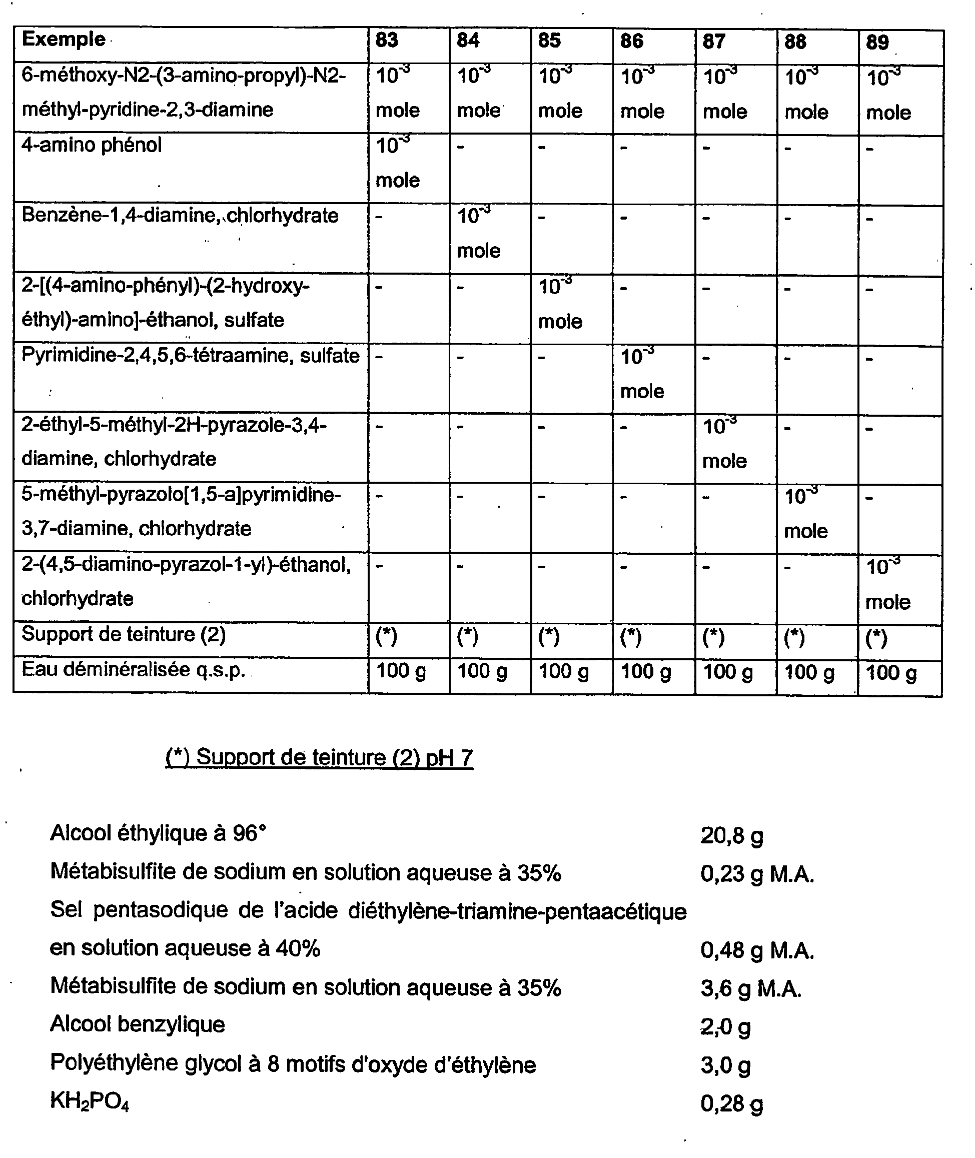

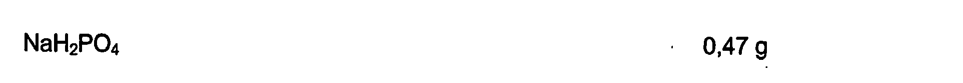

Au moment de l'emploi, chaque composition est mélangée avec un poids égal d'eau oxygénée à 20 volumes (6 % en poids). On obtient un pH final de 7.

Chaque mélange obtenu est appliqué sur des cheveux gris à 90 % de blancs. Après 30 min de pose, les cheveux sont rincés, lavés avec un shampooing standard, rincés à nouveau puis séchés.

Les résultats de teinture suivants ont été obtenus.

| **Exemple** | **83** | **84** | **85** | **86** | **87** | **88** | **89** |
|---|---|---|---|---|---|---|---|
| **Nuance observée** | Brun jaune intense | Gris intense | Gris bleu intense | Gris intense | Gris intense | Gris intense | Gris intense |

### EXEMPLES 90 A 94 DE TEINTURE EN MILIEU ACIDE

On a préparé les compositions tinctoriales dans les proportions suivantes :

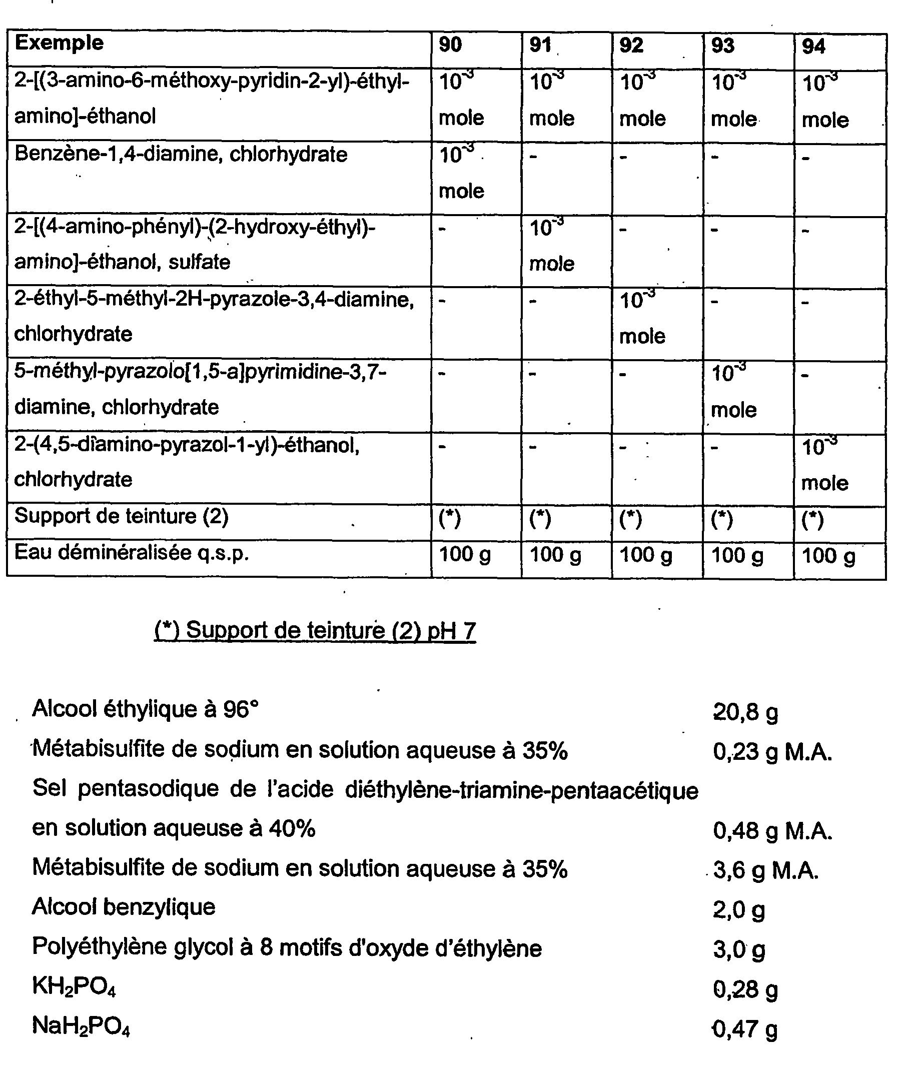

Au moment de l'emploi, chaque composition est mélangée avec un poids égal d'eau oxygénée à 20 volumes (6 % en poids). On obtient un pH final de 7.

Chaque mélange obtenu est appliqué sur des cheveux gris à 90 % de blancs. Après 30 min de pose, les cheveux sont rincés, lavés avec un shampooing standard, rincés à nouveau puis séchés.

Les résultats de teinture suivants ont été obtenus.

| **Exemple** | **90** | **91** | **92** | **93** | **94** |
|---|---|---|---|---|---|
| **Nuance observée** | Gris bleu intense | Vert-bleu | Brun | Gris violet intense | Violet intense |

### EXEMPLES 95 A 99 DE TEINTURE EN MILIEU ACIDE

On a préparé les compositions tinctoriales dans les proportions suivantes :

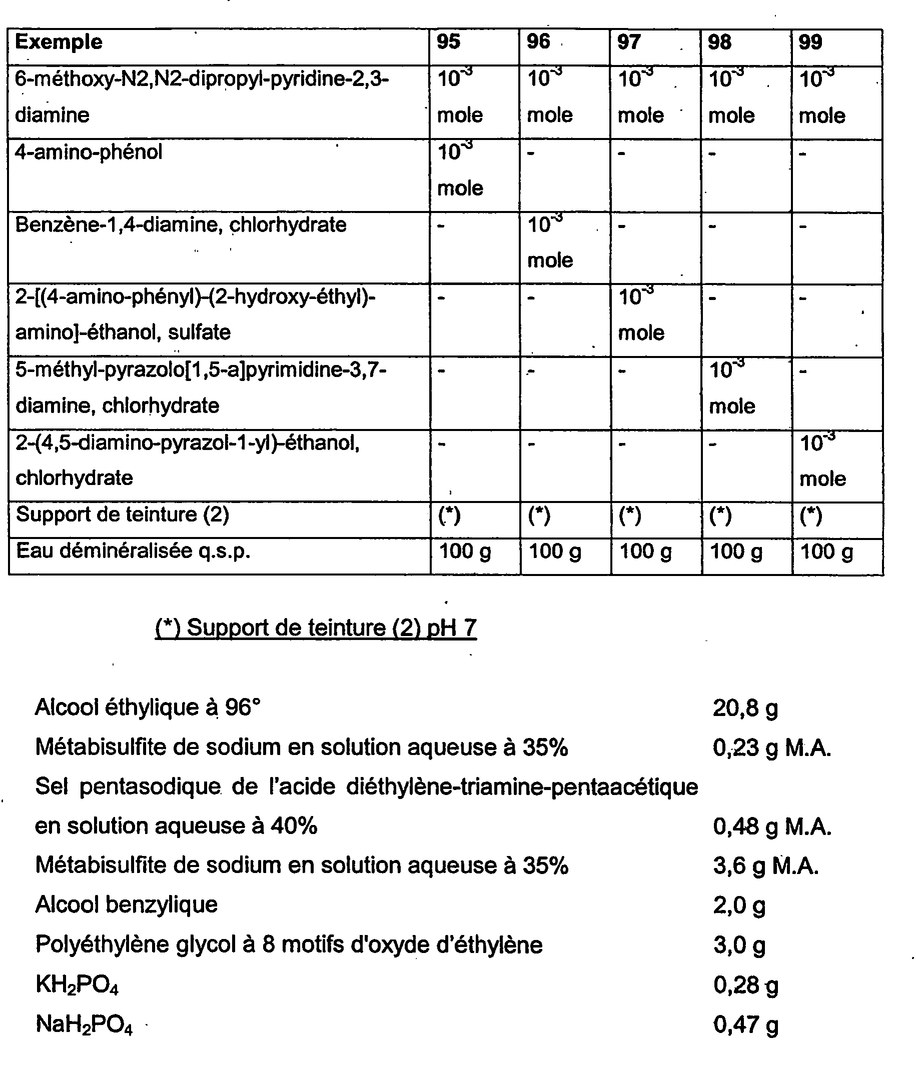

Au moment de l'emploi, chaque composition est mélangée avec un poids égal d'eau oxygénée à 20 volumes (6 % en poids). On obtient un pH final de 7.

Chaque mélange obtenu est appliqué sur des cheveux gris à 90 % de blancs. Après 30 min de pose, les cheveux sont rincés, lavés avec un shampooing standard, rincés à nouveau puis séchés.

Les résultats de teinture suivants ont été obtenus.

| **Exemple** | **95** | **96** | **97** | **98** | **99.** |
|---|---|---|---|---|---|
| **Nuance observée** | Orangé | Gris intense | Gris vert-bleu intense | Gris violet intense | Rouge |

### EXEMPLES 100 A 106 DE TEINTURE EN MILIEU ACIDE

On a préparé les compositions tinctoriales dans les proportions suivantes :

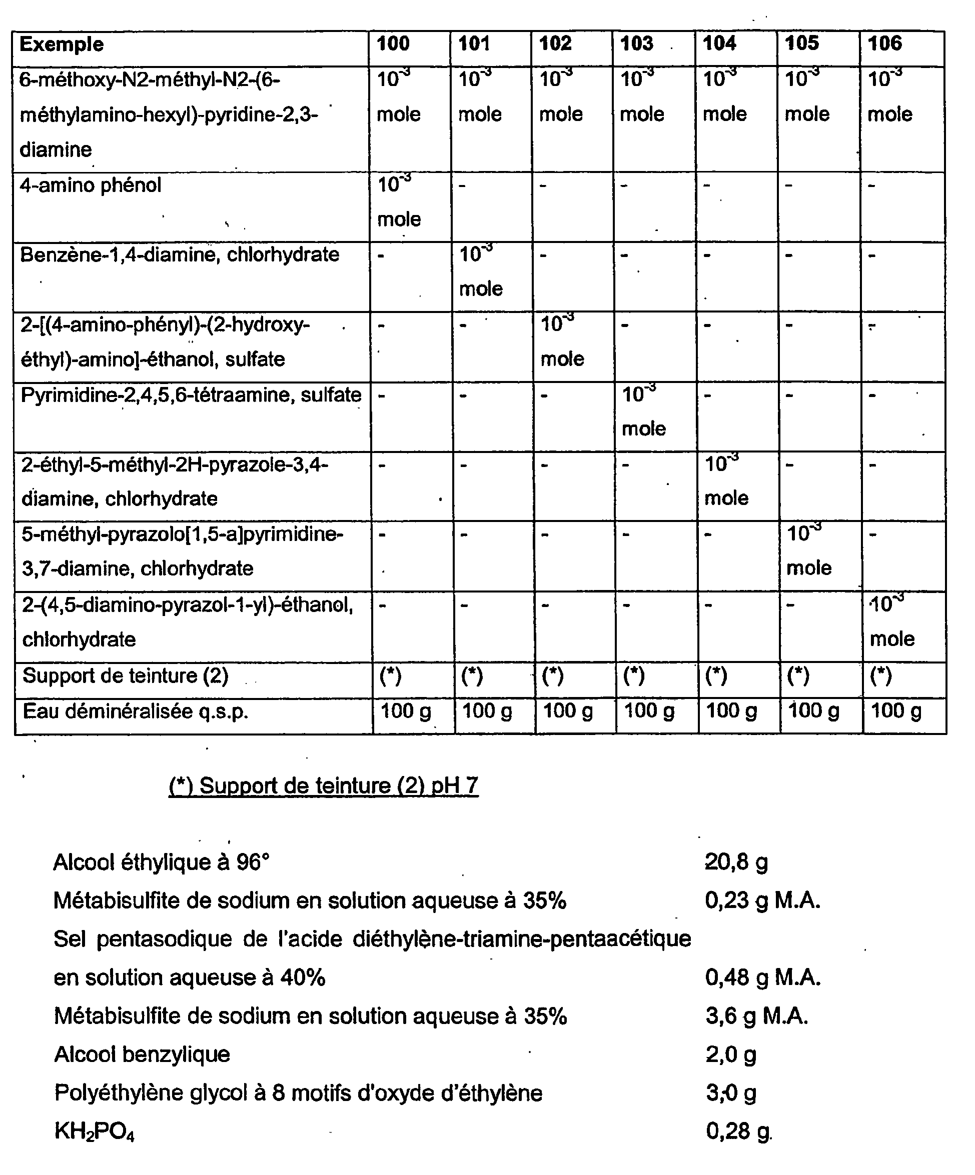

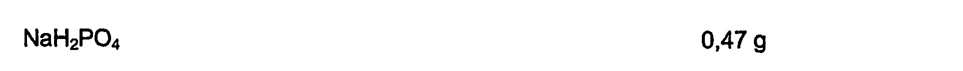

Au moment de l'emploi, chaque composition est mélangée avec un poids égal d'eau oxygénée à 20 volumes (6 % en poids). On obtient un pH final de 7.

Chaque mélange obtenu est appliqué sur des cheveux gris à 90 % de blancs. Après 30 min de pose, les cheveux sont rincés, lavés avec un shampooing standard, rincés à nouveau puis sèches.

Les résultats de teinture suivants ont été obtenus.

| **Exemple** | **100** | **101** | **102** | **103** | **104** | **105** | **106** |
|---|---|---|---|---|---|---|---|
| **Nuance observée** | Orangé | Gris bleu , intense | Vert-bleu intense | Brun jaune | Gris | Gris violet intense | Gris violet intense |

### EXEMPLES 107 A 113 DE TEINTURE EN MILIEU ACIDE

On a préparé les compositions tinctoriales dans les proportions suivantes :

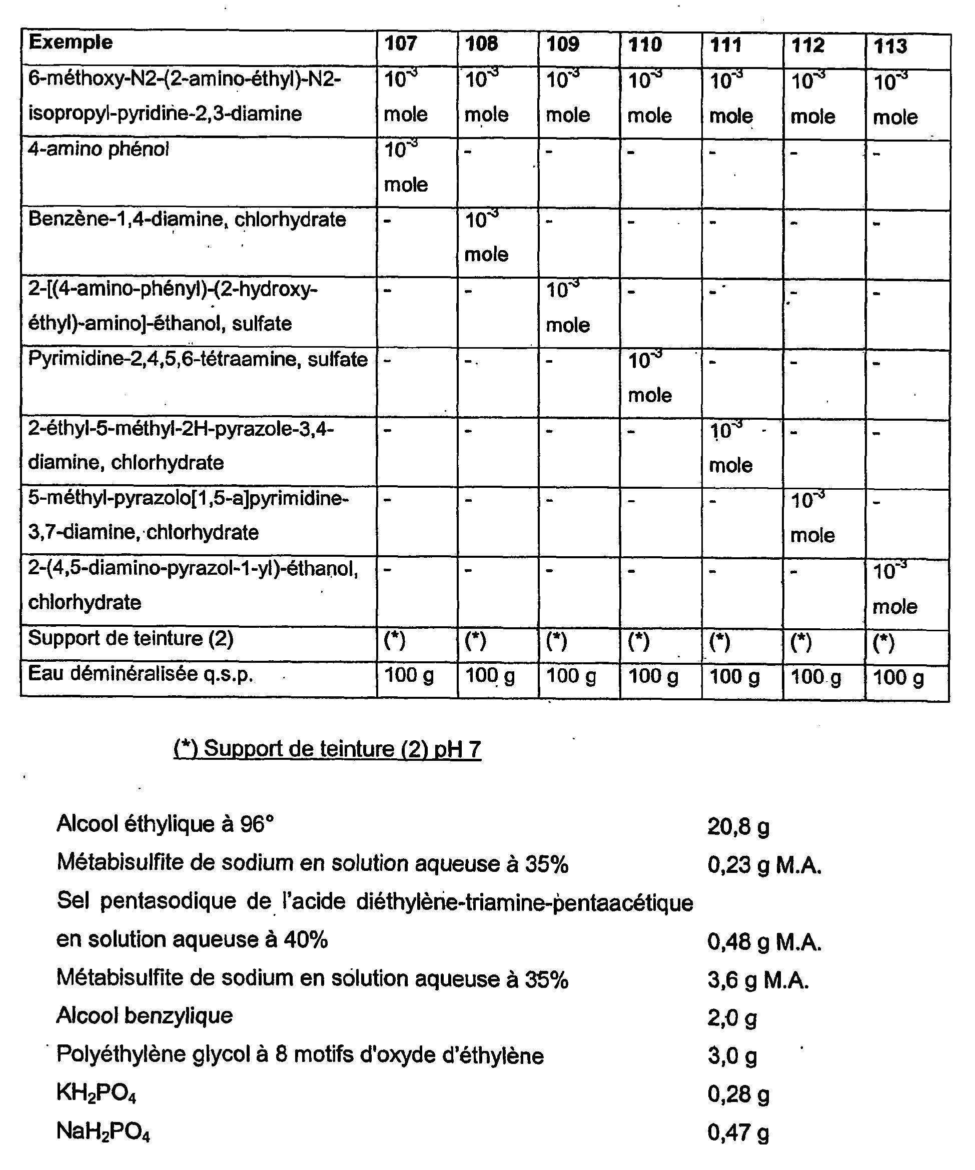

Au moment de l'emploi, chaque composition est mélangée avec un poids égal d'eau oxygénée à 20 volumes (6 % en poids). On obtient un pH final de 7.

Chaque mélange obtenu est appliqué sur des cheveux gris à 90 % de blancs. Après 30 min de pose, les cheveux sont rincés, lavés avec un shampooing standard, rincés à nouveau puis séchés.

Les résultats de teinture suivants ont été obtenus.

| **Exemple** | **107** | **108** | **109** | **110** | **111** | **112** | **113** |
|---|---|---|---|---|---|---|---|
| **Nuance observée** | Brun jaune intense | Gris intense | Gris intense | Gris | Gris | Gris intense | Gris intense |

### EXEMPLES 114 A 120 DE TEINTURE EN MILIEU ACIDE

On a préparé les compositions tinctoriales dans les proportions suivantes :

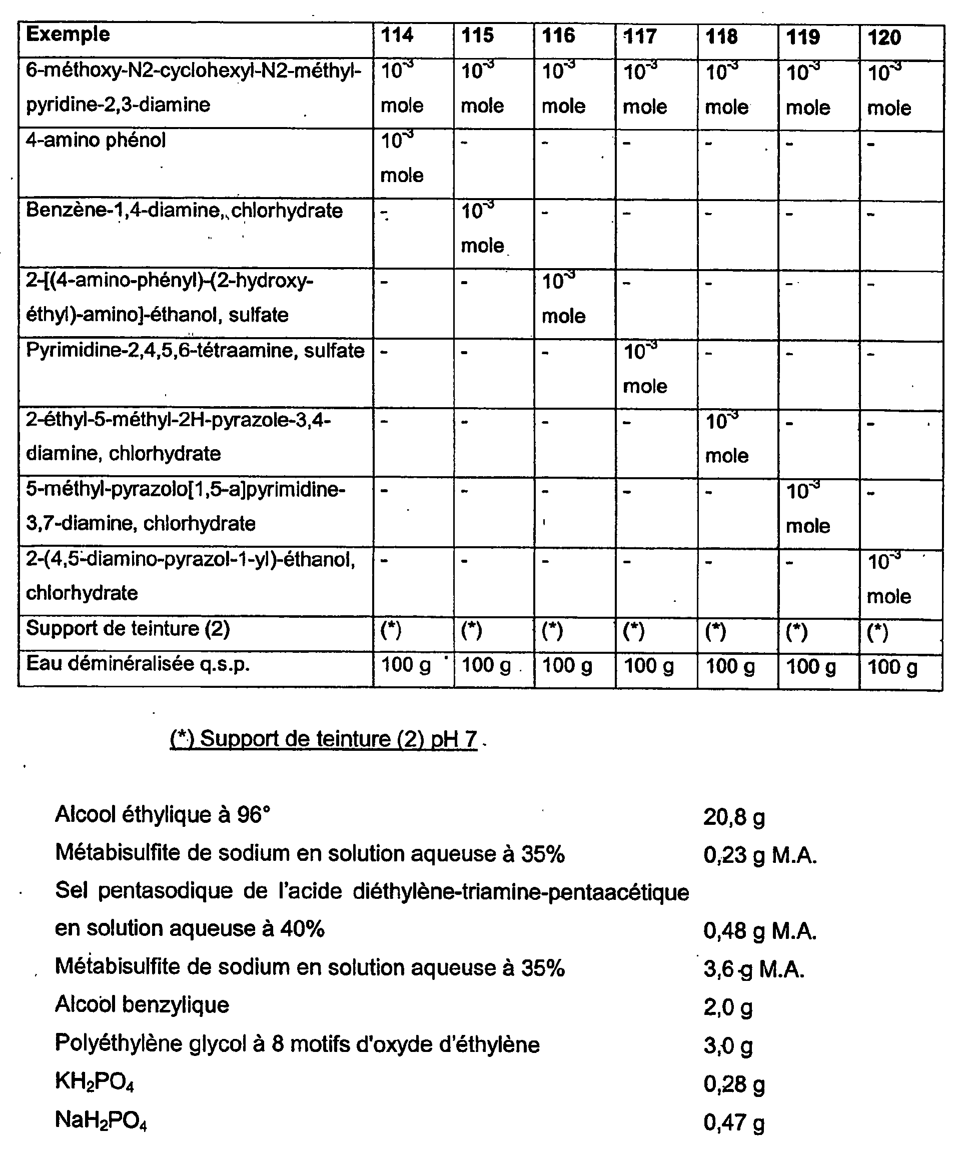

Au moment de l'emploi, chaque composition est mélangée avec un poids égal d'eau oxygénée à 20 volumes (6 % en poids). On obtient un pH final de 7.

Chaque mélange obtenu est appliqué sur des cheveux gris à 90 % de blancs. Après 30 min de pose, les cheveux sont rincés, lavés avec un shampooing standard, rincés à nouveau puis séchés.

Les résultats de teinture suivants ont été obtenus.

| **Exemple** | **114** | **115** | **116** | **117** | **118** | **119** | **120** |
|---|---|---|---|---|---|---|---|
| **Nuance observée** | Orangé | Gris intense | Vert-bleu intense | Brun jaune | Brun | Gris violet intense | Gris violet intense |

### EXEMPLES 121 A 127 DE TEINTURE EN MILIEU ACIDE

On a préparé les compositions tinctoriales dans les proportions suivantes :

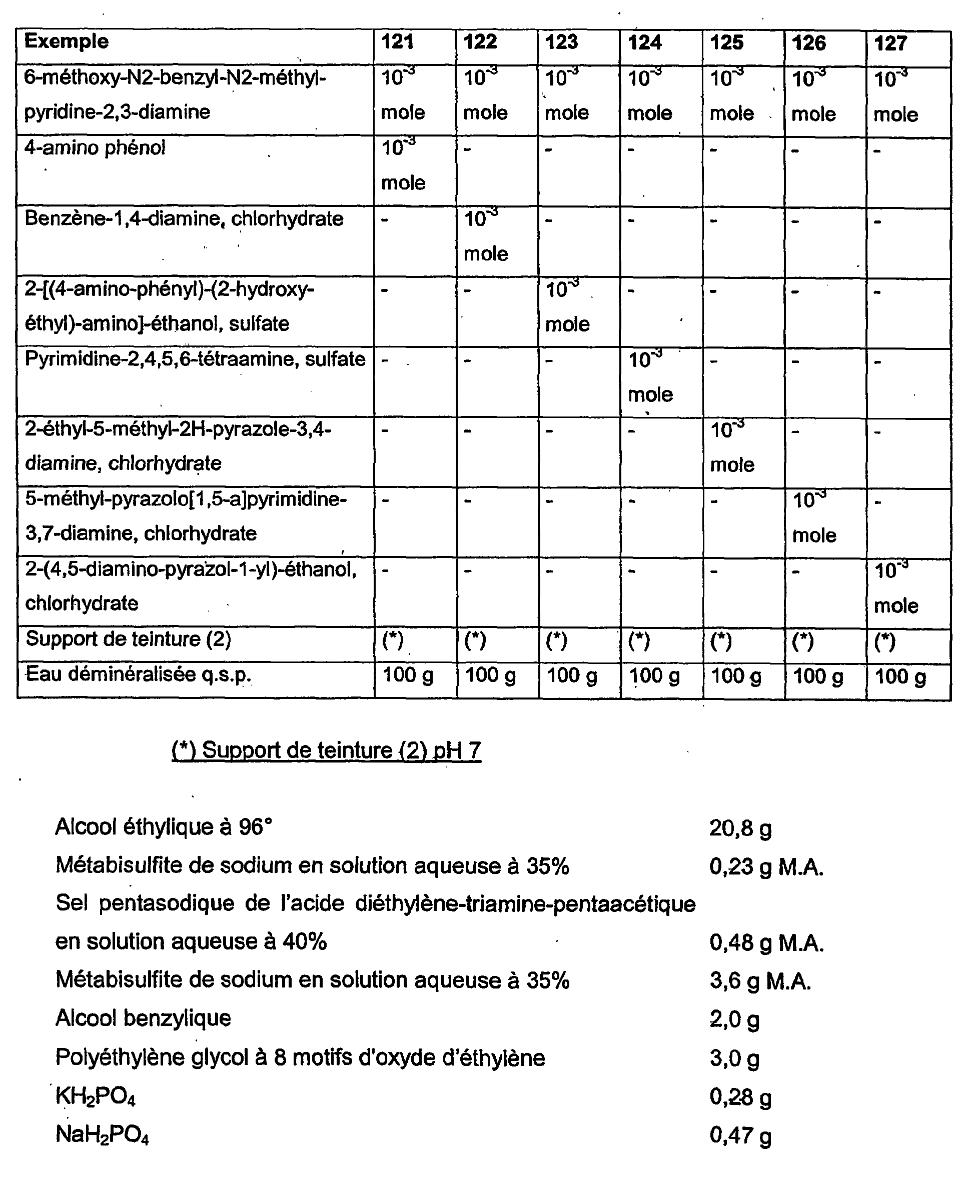

Au moment de l'emploi, chaque composition est mélangée avec un poids égal d'eau oxygénée à 20 volumes (6 % en poids). On obtient un pH final de 7.

Chaque mélange obtenu est appliqué sur des cheveux gris à 90 % de blancs. Après 30 min de pose, les cheveux sont rincés, lavés avec un shampooing standard, rincés à nouveau puis séchés.

Les résultats de teinture suivants ont été obtenus.

| **Exemple** | **121** | **122** | **123** | **124** | **125** | **126** | **127** |
|---|---|---|---|---|---|---|---|
| **Nuance observée** | Orangé | Orangé intense | Orangé intense | Jaune intense | Jaune intense | Brun jaune intense | Brun orangé intense |

### EXEMPLES 128 A 134 DE TEINTURE EN MILIEU ACIDE

On a préparé les compositions tinctoriales dans les proportions suivantes :

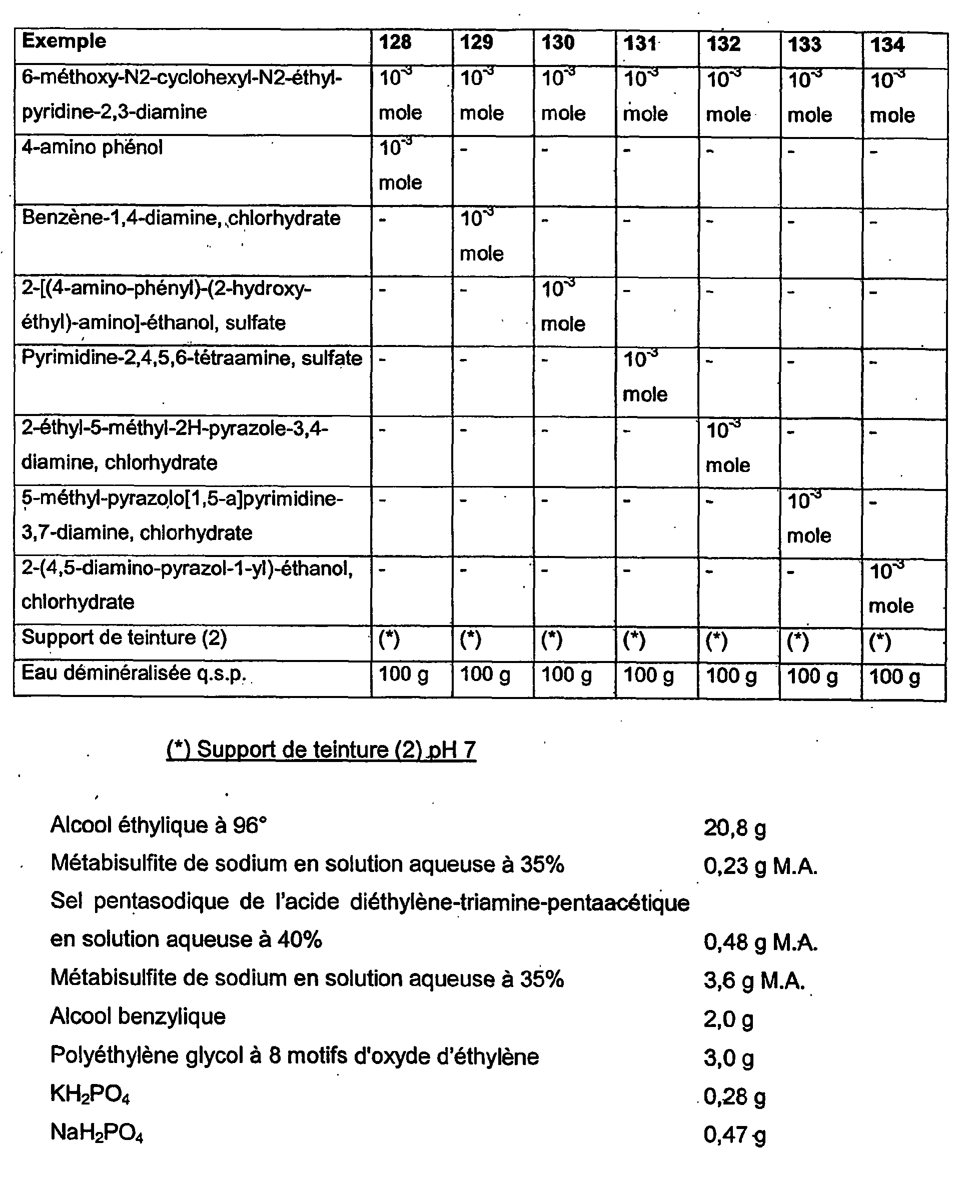

Au moment de l'emploi, chaque composition est mélangée avec un poids égal d'eau oxygénée à 20 volumes (6 % en poids). On obtient un pH final de 7.

Chaque mélange obtenu est appliqué sur des cheveux gris à 90 % de blancs. Après 30 min de pose, les cheveux sont rincés, lavés avec un shampooing standard, rincés à nouveau puis séchés.

Les résultats de teinture suivants ont été obtenus.

| **Exemple** | 128 | 129 | 130 | 131 | 132 | 133 | 134 |
|---|---|---|---|---|---|---|---|
| **Nuance observée** | Orangé | Brun rouge intense | Gris intense | Brun jaune | Brun | Gris intense | Brun intense |

### EXEMPLES 135 A 141 DE TEINTURE EN MILIEU ACIDE

On a préparé les compositions tinctoriales dans les proportions suivantes :

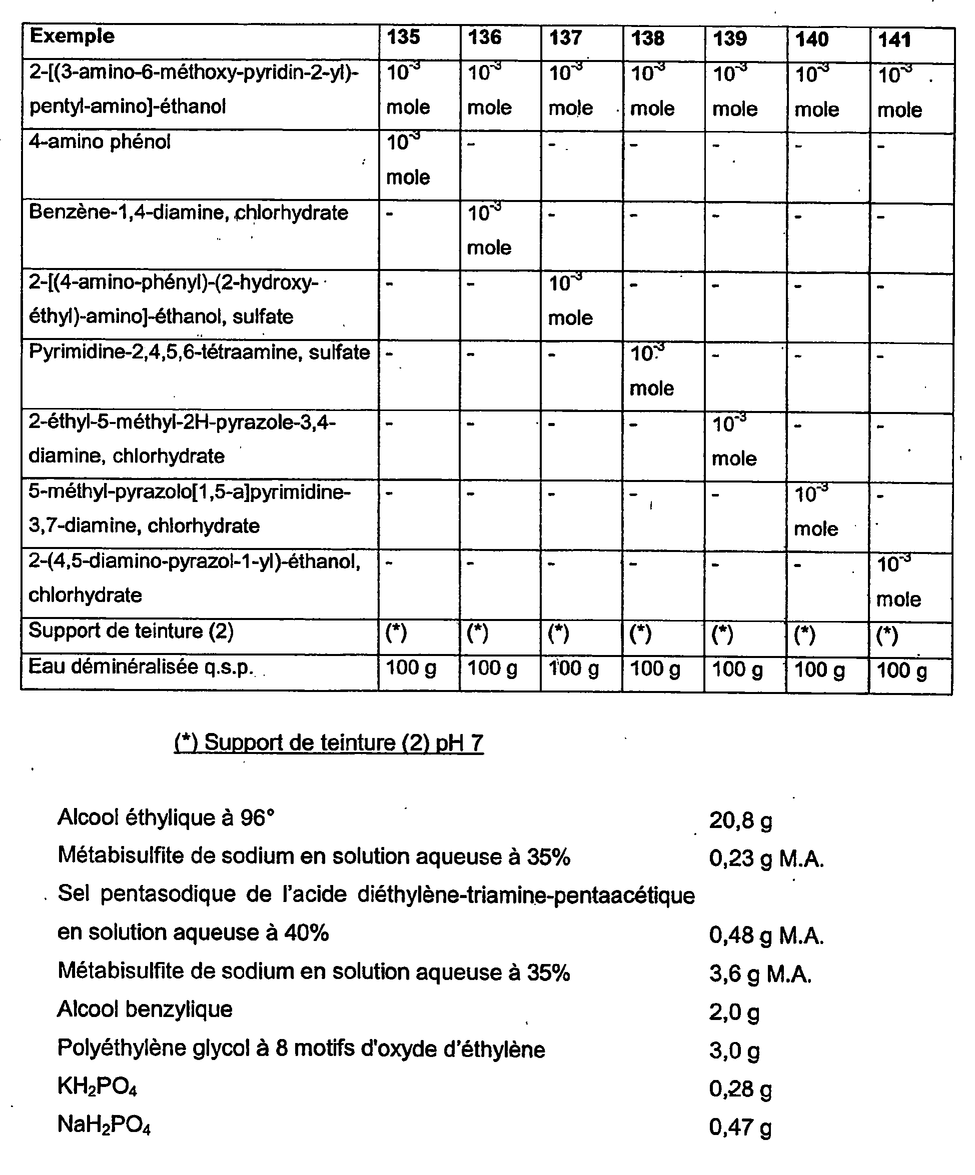

Au moment de l'emploi, chaque composition est mélangée avec un poids égal d'eau oxygénée à 20 volumes (6 % en poids). On obtient un pH final de 7.

Chaque mélange obtenu est appliqué sur des cheveux gris à 90 % de blancs. Après 30 min de pose, les cheveux sont rincés, lavés avec un shampooing standard, rincés à nouveau puis séchés.

Les résultats de teinture suivants ont été obtenus.

| **Exemple** | **135** | **136** | **137** | **138** | **139** | **140** | **141** |
|---|---|---|---|---|---|---|---|
| **Nuancé observée** | Jaune | Brun | Gris vert-bleu intense | Brun jaune | Gris | Gris violet intense | Gris violet intense |

### EXEMPLES 142 A 148 DE TEINTURE EN MILIEU ACIDE

On a préparé les compositions tinctoriales dans les proportions suivantes :

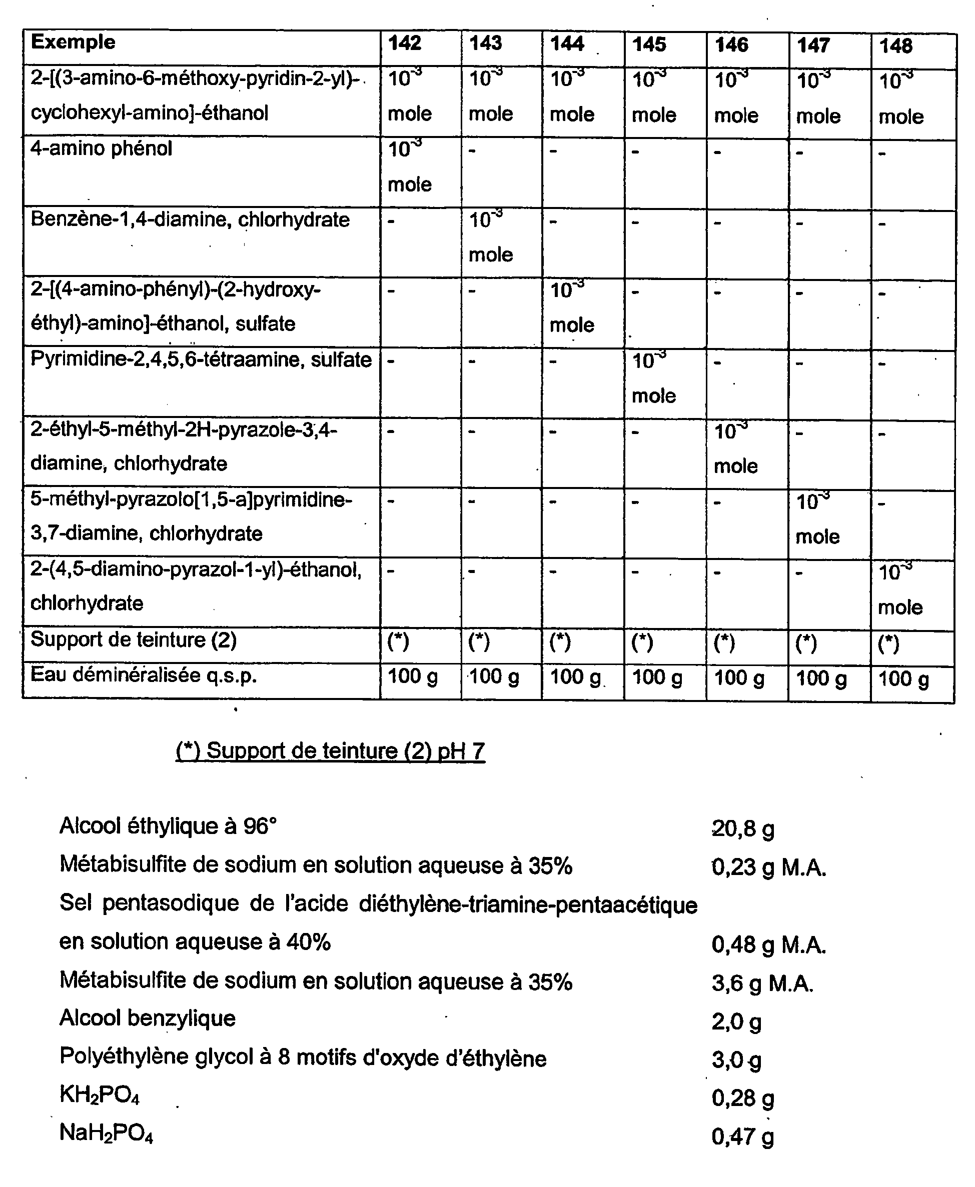

Au moment de l'emploi, chaque composition est mélangée avec un poids égal d'eau oxygénée à 20 volumes (6 % en poids). On obtient un pH final de 7.

Chaque mélange obtenu est appliqué sur des cheveux gris à 90 % de blancs. Après 30 min de pose, les cheveux sont rincés, lavés avec un shampooing standard, rincés à nouveau puis séchés.

Les résultats de teinture suivants ont été obtenus.

| **Exemple** | **142** | **143** | **144** | **145** | **146** | **147** | **148** |
|---|---|---|---|---|---|---|---|
| **Nuance observée** | Orangé | Brun rouge | Gris intense | Brun jaune | Gris jaune-vert | Gris intense | Brun intense |

### EXEMPLES 149 A 155 DE TEINTURE EN MILIEU ACIDE

On a préparé les compositions tinctoriales dans les proportions suivantes :

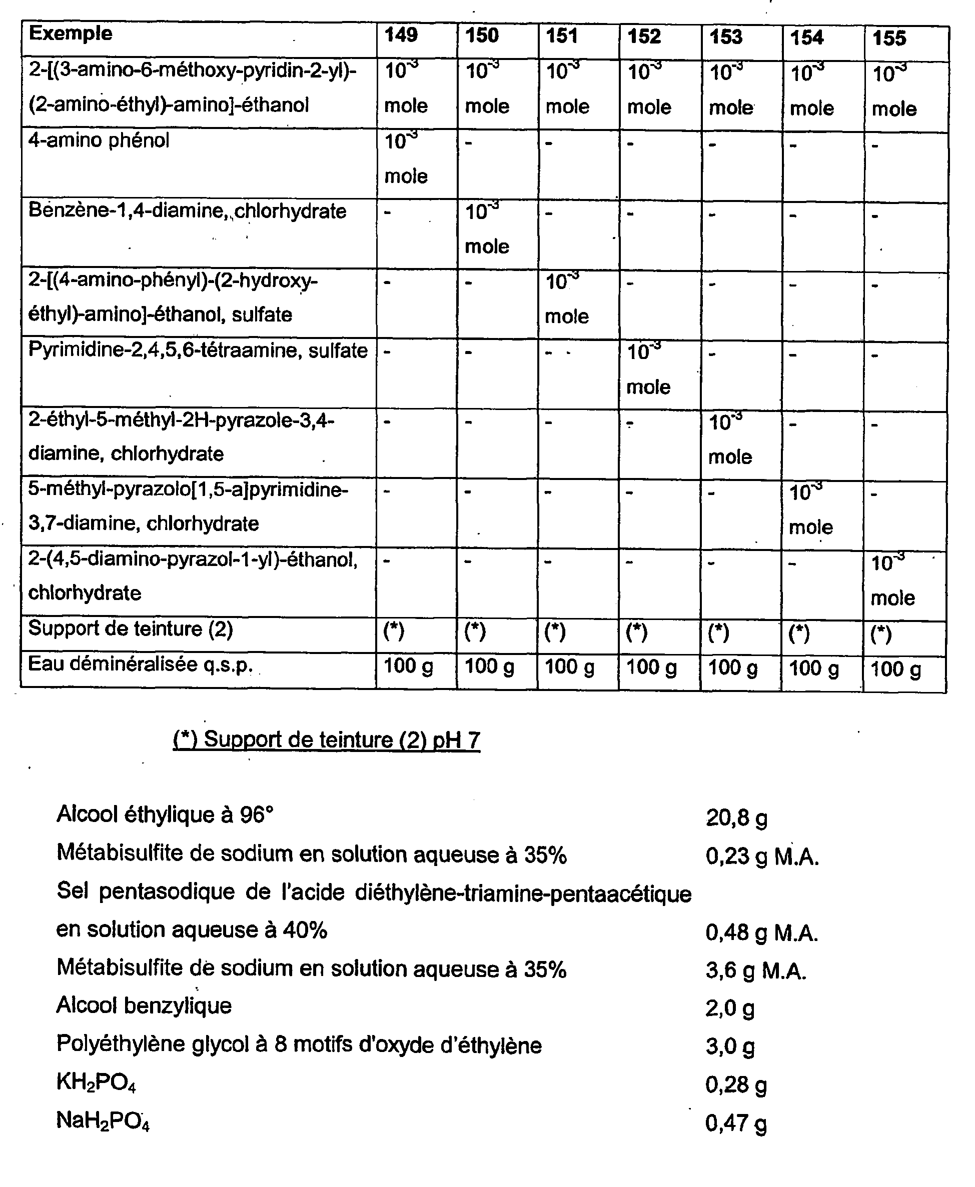

Au moment de l'emploi, chaque composition est mélangée avec un poids égal d'eau oxygénée à 20 volumes (6 % en poids). On obtient un pH final de 7.

Chaque mélange obtenu est appliqué sur des cheveux gris à 90 % de blancs. Après 30 min de pose, les cheveux sont rincés, lavés avec un shampooing standard, rincés à nouveau puis séchés.

Les résultats de teinture suivants ont été obtenus.

| **Exemple** | **149** | **150** | **151** | **152** | **153** | **154** | **155** |
|---|---|---|---|---|---|---|---|
| **Nuance observée** | Brun orangé | Gris intense | Gris bleu intense | Gris | Gris | Gris intense | Gris intense |

### EXEMPLES 156 A 161 DE TEINTURE EN MILIEU ACIDE

On a préparé les compositions tinctoriales dans les proportions suivantes :

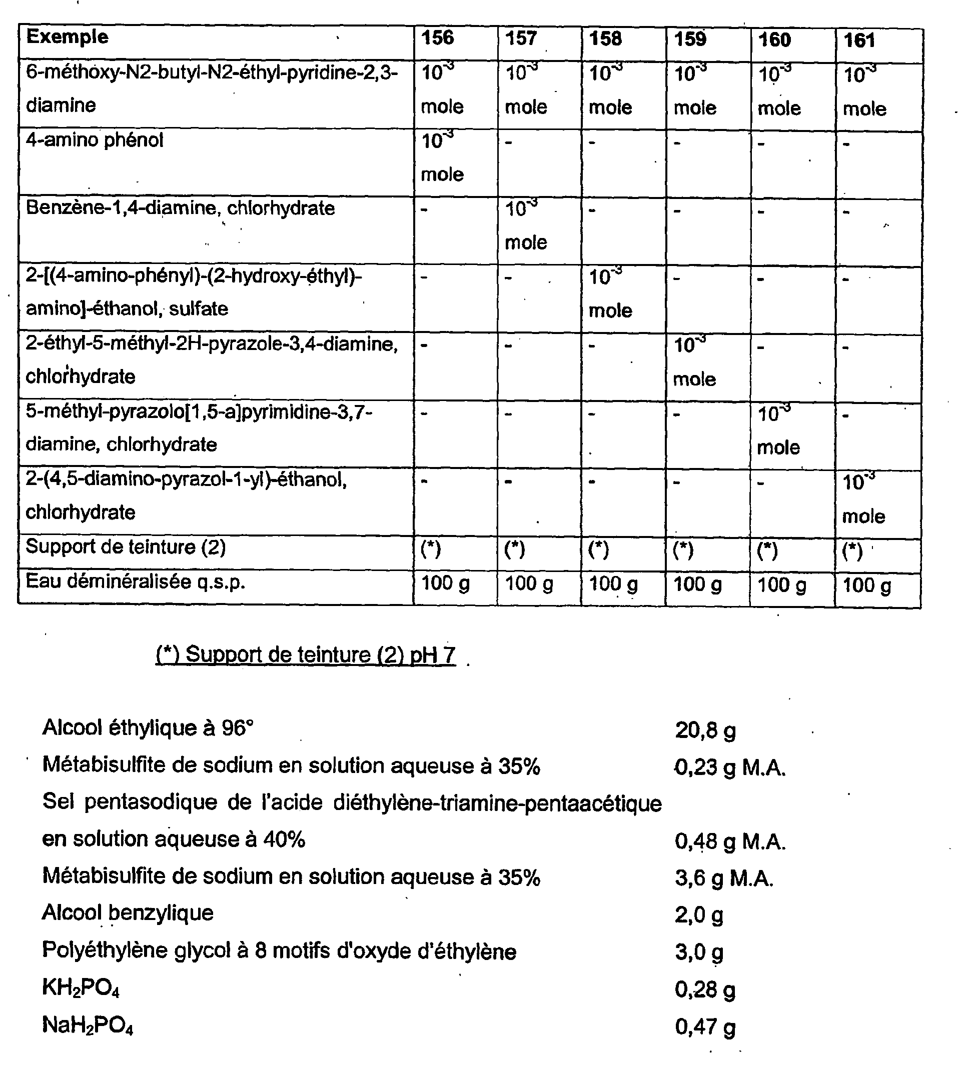

Au moment de l'emploi, chaque composition est mélangée avec un poids égal d'eau oxygénée à 20 volumes (6 % en poids). On obtient un pH final de 7.

Chaque mélange obtenu est appliqué sur des cheveux gris à 90 % de blancs. Après 30 min de pose, les cheveux sont rincés, lavés avec un shampooing standard, rincés à nouveau puis séchés.

Les résultats de teinture suivants ont été obtenus.

| **Exemple** | **156** | **157** | **158** | **159** | **160** | **161** |
|---|---|---|---|---|---|---|
| **Nuance observée** | Brun orangé | Gris bleu intense | Vert-bleu intense | Gris violet | Gris violet intense | Violet intense |

## Revendications

1. Composition tinctoriale comprenant, dans un milieu de teinture approprié :
- au moins une base d'oxydation, et
- au moins un coupleur 6-alcoxy-2,3-diaminopyridine de formule (I) ou l'un de ses sels d'addition correspondants : 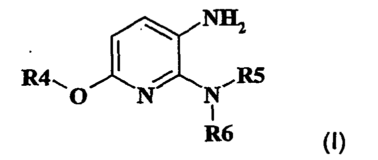 dans laquelle :
- R₄ représente un radical alkyle en C₁-C₄ linéaire ou ramifié éventuellement substitué par un ou plusieurs radicaux hydroxyle, alcoxy en C₁-C₂, NR₇R₈ où R₇ et R₈ sont choisis parmi un atome d'hydrogène, un alkyle en C₁-C₄, un (poly)hydroxyalkyle en C₂-C₆, un (poly)aminoalkyle en C₂-C₆ ou un amino-hydroxyalkyle en C₂-C₆ ;
- R₅ représente un radical alkyle en C₁-C₆ linéaire ou ramifié éventuellement substitué par un ou plusieurs radicaux choisis parmi les radicaux alcoxy en C₁-C₂, hydroxy, carboxy, sulfonique (-SO₃H), NR₉R₁₀ ; un radical acylaminoéthyle ; un radical 2-(2-hydroxyéthyloxy)-éthyle ;
- R₆ représente un radical alkyle en C₁-C₆ linéaire ou ramifié éventuellement substitué par un ou plusieurs radicaux choisis parmi les radicaux alcoxy en C₁-C₂, hydroxy, carboxy, sulfonique, NR'₉R'₁₀, ou acylamino (RCONH- avec R étant un alkyle en C₁-C₄) ;
- R₉, R₁₀, R₉ et R₁₀' désignent indépendamment l'un de l'autre, un atome d'hydrogène ; un radical alkyle en C₁-C₄ éventuellement substitué par un ou plusieurs radicaux hydroxy, amino, mono- ou di- alkylamino, alcoxy ou acylamino.

2. Composition selon la revendication 1 dans laquelle R₆ représente un radical alkyle substitué.

3. Composition selon la revendication 1 dans laquelle R₅ et R₆ représentent indépendamment un radical alkyle en C₁-C₄ éventuellement substitué par un ou plusieurs radicaux choisis parmi les radicaux hydroxy, alcoxy en C₁-C₂, amino, (di)alkylamino en C₁-C₂, carboxy, (di)hydroxyalkylamino en C₁-C₂ ; un radical 2-acylaminoéthyle ; un radical 2-(2-hydroxyéthyloxy)-éthyle.

4. Composition selon la revendication 3 dans laquelle R₅ représente un radical méthyle, éthyle, propyle, 2-hydroxyéthyle, 2-aminoéthyle, 2-carboxyéthyle, 2-acylaminoéthyle, 2-hydroxypropyle, 3-hydroxypropyle, 3-aminopropyle, 2-N,N-diméthylaminoéthyle, 2-N-méthylaminoéthyle, 2-(2-hydroxyéthylamino)-éthyle, 2-(2-hydroxyéthyloxy)-éthyle.

5. Composition selon l'une quelconque des revendications 1 à 3, dans laquelle R₆ représente un radical alkyle en C₁-C₄ substitué par un ou plusieurs radicaux choisis parmi les radicaux hydroxy, alcoxy en C₁-C₂, amino, (di)alkylamino en C₁-C₂, carboxy, acylamino, (di)hydroxyalkylamino ; un radical 2-(2-hydroxyéthyloxy)-éthyle.

6. Composition selon la revendication 5, dans laquelle R₆ représente un radical 2-hydroxyéthyle, 2-aminoéthyle, 2-carboxyéthyle, 2-acylaminoéthyle, 2-hydroxypropyle, 3-hydroxypropyle , 3-aminopropyle, 2-N,N-diméthylaminoéthyle, 2-N-méthylaminoéthyle, 2-(2-hydroxyéthylamino)-éthyle, 2-(2-hydroxyéthyloxy)-éthyle.

7. Composition selon l'une quelconque des revendications 1 à 6 dans laquelle R₄ représente un radical alkyle en C₁-C₄ éventuellement substitué par un ou plusieurs radicaux hydroxyle, alcoxy en C₁-C_{2.}

8. Composition selon la revendication 7 dans laquelle R₄ représente un radical alkyle en C₁-C₄.

9. Composition selon la revendication 8 dans laquelle R₅ est un radical alkyle et R₆ est un radical alkyle substitué par un ou plusieurs hydroxy.

10. Composition selon la revendication 8 dans laquelle R₅ et R₆ représentent un radical alkyle substitué par un ou plusieurs hydroxy.

11. Composition selon l'une quelconque des revendications 1 à 10 dans laquelle le composé de formule (I) est choisi parmi :
- le 2-[(3-amino-6-méthoxy-pyridin-2-yl)-méthyl-amino]-éthanol ;
- le 2-[(3-amino-6-méthoxy-pyridin-2-yl)-butyl-amino]-éthanol ;
- le 2-[(3-amino-6-méthoxy-pyridin-2 yl)-(2-hydroxy-éthyl)-amino]-éthanol ;
- le 3-[(3-amino-6-méthoxy-pyridin-2-yl)-méthyl-amino]-propane-1,2-diol ;
- le 6-méthoxy-N2,N2-bis-(2-méthoxy-éthyl)-pyridine-2, 3-diamine ;
- le 6-méthoxy-N2-éthyl-N2-méthyl-pyridine-2,3-diamine ;
- le 6-méthoxy-N2-méthyl-N2-propyl-pyridine-2,3-diamine ;
- le 6-méthoxy-N2-butyl-N2-méthyl-pyridine-2,3-diamine ;
- le 6-méthoxy-N2-(3-amino-propyl)-N2-méthyl-pyridine-2,3-diamine ;
- le 2-[(3-amino-6-méthoxy-pyridin-2-yl)-éthyl-amino]-éthanol ;
- le 6-méthoxy-N2,N2-diisopropyl-pyridine-2,3-diamine ;
- le 6-méthoxy-N2,N2-dipropyl-pyridine-2,3-diamine ;
- le 6-méthoxy-N2-butyl-N2-éthyl-pyridine-2,3-diamine ;
- le 6-méthoxy-N2-(2-amino-éthyl)-N2-isopropyl-pyridine-2,3-diamine ;
- le 6-methoxy-N2-cyclohexyl-N2-méthyl-pyridine-2,3-diamine ;
- le 2-[(3-amino-6-méthoxy-pyridin-2-yl)-(3-amino-propyl)-amino]-éthanol ;
- le 6-méthoxy-N2-cyclohexyl-N2-éthyl-pyridine-2,3-diamine ;
- le 2-[(3-amino-6-méthoxy-pyridin-2-yl)-pentyl-amino]-éthanol ;
- le 2-[(3-amino-6-méthoxy-pyridin-2-yl)-cyclohexyl-amino]-éthanol ;
- le 2-[(3-amino-méthoxy-pyridin-2-yl)- (2-amino-éthyl)-amino]-éthanol ;
- le 6-méthoxy-N2-méthyl-N2-(6-méthylamino-hexyl)-pyridine-2,3-diamine ;
ainsi que leurs sels d'addition.

12. Composition selon l'une quelconque des revendications 1 à 11 dans laquelle la ou les bases d'oxydation sont choisies parmi les paraphénylènediamines, les bis-phénylalkylènediamines, les para-aminophénols, les bis-para-aminophénols, les ortho-aminophénols, les bases hétérocycliques et leurs sels d'addition.

13. Composition selon l'une quelconque des revendications 1 à 12 dans laquelle la ou les bases d'oxydation sont chacune présentes en quantité comprise 0,001 et 10 % en poids du poids total de la composition tinctoriale.

14. Composition selon l'une quelconque des revendications 1 à 13, comprenant un ou plusieurs coupleurs additionnels choisis parmi les métaphénylènediamines, les méta-aminophénols, les métadiphénols, les coupleurs naphtaléniques et les coupleurs hétérocycliques autres que les coupleurs de formule (I) tels que définis dans l'une quelconque des revendications 1 à 11 et leurs sels d'addition.

15. Composition selon l'une quelconque des revendications 1 à 14 dans laquelle le ou les coupleurs sont présents chacun en quantité comprise entre 0,001 et 10 % en poids du poids total de la composition tinctoriale.

16. Composition selon l'une quelconque des revendications précédentes dans lequel le milieu de teinture est un milieu cosmétique approprié pour la teinture des fibres kératiniques.

17. Composition selon l'une quelconque des revendications précédentes contenant de plus un agent oxydant.

18. Procédé de teinture d'oxydation des fibres kératiniques **caractérisé en ce qu'**on applique sur les fibres une composition telle que définie à l'une quelconque des revendications 1 à 16, en présence d'un agent oxydant, pendant un temps suffisant permettant d'obtenir la couleur désirée.

19. Procédé selon la revendication 18, dans lequel l'agent oxydant est choisi parmi le peroxyde d'hydrogène, le peroxyde d'urée, les bromates de métaux alcalins, les persels, les peracides et les enzymes oxydases.

20. Procédé selon l'une des revendications 18 ou 19 dans lequel l'agent oxydant est mélangé au moment de l'emploi à la composition telle que définie selon l'une quelconque des revendications 1 à 16.

21. Procédé selon l'une quelconque des revendications 18 à 19, dans lequel l'agent oxydant est appliqué sur les fibres simultanément ou séquentiellement à la composition telle que définie selon l'une quelconque des revendications 1 à 16 sous forme d'une composition oxydante.

22. Dispositif à plusieurs compartiments dans lequel un premier compartiment tinctoriale contient une composition telle que définie à l'une quelconque des revendications 1 à 16 et un deuxième compartiment contient une composition oxydante.

23. Kit pour la teinture de fibres kératiniques comprenant d'une part une composition telle que définie selon l'une quelconque des revendications 1 à 16 et d'autre part une composition oxydante.

24. Utilisation de la composition telle que définie selon l'une quelconque des revendications 1 à 16 pour la teinture des fibres kératiniques.

25. Composé 6-alcoxy-2,3-diaminepyridine de formule (I) telle que définie dans l'une quelconque des revendications 1 à 11 à l'exception de la 2-(N-méthyl N-(β-hydroxy-éthyl)amino) 3-amino 6-méthoxy pyridine

## Claims

1. Dye composition comprising, in a medium that is suitable for dyeing:
- at least one oxidation base, and
- at least one 6-alkoxy-2,3-diaminopyridine coupler of formula (I) or a corresponding addition salt thereof: 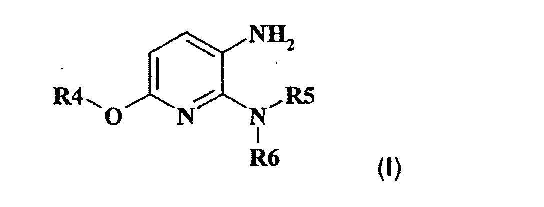 in which:
- R₄ represents a linear or branched C₁-C₄ alkyl radical optionally substituted with one or more hydroxyl, C₁-C₂ alkoxy or NR₇R₈ radicals in which R₇ and R₈ are chosen from a hydrogen atom, a C₁-C₄ alkyl, a C₂-C₆ (poly)hydroxyalkyl, a C₂-C₆ (poly)aminoalkyl or a C₂-C₆ aminohydroxyalkyl;
- R₅ represents a linear or branched C₁-C₆ alkyl radical optionally substituted with one or more radicals chosen from C₁-C₂ alkoxy, hydroxyl, carboxyl, sulphonic (-SO₃H) or NR₉R₁₀ radicals; an acylaminoethyl radical; a 2-(2-hydroxyethyloxy)ethyl radical;
- R₆ represents a linear or branched C₁-C₆ alkyl radical optionally substituted with one or more radicals chosen from C₁-C₂ alkoxy, hydroxyl, carboxyl, sulphonic, NR'₉R'₁₀ or acylamino radicals (RCONH- with R being a C₁-C₄ alkyl);
- R₉, R₁₀, R₉' and R₁₀' denote, independently of each other, a hydrogen atom; a C₁-C₄ alkyl radical optionally substituted with one or more hydroxyl, amino, monoalkylamino, dialkylamino, alkoxy or acylamino radicals.

2. Composition according to Claim 1, in which R₆ represents a substituted alkyl radical.

3. Composition according to Claim 1, in which R₅ and R₆ independently represent a C₁-C₄ alkyl radical optionally substituted with one or more radicals chosen from hydroxyl, C₁-C₂ alkoxy, amino, C₁-C₂ (di)alkylamino, carboxyl and C₁-C₂ (di)hydroxyalkylamino radicals; a 2-acylaminoethyl radical; a 2-(2-hydroxyethyloxy)ethyl radical.

4. Composition according to Claim 3, in which R₅ represents a methyl, ethyl, propyl, 2-hydroxyethyl, 2-aminoethyl, 2-carboxyethyl, 2-acylaminoethyl, 2-hydroxypropyl, 3-hydroxypropyl, 3-aminopropyl, 2-N,N-dimethylaminoethyl, 2-N-methylaminoethyl, 2-(2-hydroxyethylamino)ethyl or 2-(2-hydroxyethyloxy)ethyl radical.

5. Composition according to any one of Claims 1 to 3, in which R₆ represents a C₁-C₄ alkyl radical substituted with one or more radicals chosen from hydroxyl, C₁-C₂ alkoxy, amino, C₁-C₂ (di)alkylamino, carboxyl, acylamino and (di)hydroxyalkylamino radicals; a 2-(2-hydroxyethyloxy)ethyl radical.

6. Composition according to Claim 5, in which R₆ represents a 2-hydroxyethyl, 2-aminoethyl, 2-carboxyethyl, 2-acylaminoethyl, 2-hydroxypropyl, 3-hydroxypropyl, 3-aminopropyl, 2-N,N-dimethylaminoethyl, 2-N-methylaminoethyl, 2-(2-hydroxyethylamino)ethyl or 2-(2-hydroxyethyloxy)ethyl radical.

7. Composition according to any one of Claims 1 to 6, in which R₄ represents a C₁-C₄ alkyl radical optionally substituted with one or more hydroxyl or C₁-C₂ alkoxy radicals.

8. Composition according to Claim 7, in which R₄ represents a C₁-C₄ alkyl radical.

9. Composition according to Claim 8, in which R₅ is an alkyl radical and R₆ is an alkyl radical substituted with one or more hydroxyl.

10. Composition according to Claim 8, in which R₅ and R₆ represent an alkyl radical substituted with one or more hydroxyl.

11. Composition according to any one of Claims 1 to 10, in which the compound of formula (I) is chosen from:
- 2-[(3-amino-6-methoxy-2-pyridyl)methylamino]ethanol;
- 2-[(3-amino-6-methoxy-2-pyridyl)butylamino]ethanol;
- 2-[(3-amino-6-methoxy-2-pyridyl)(2-hydroxyethyl)amino]ethanol;
- 3-[(3-amino-6-methoxy-2-pyridyl)methylamino]propane-1,2-diol;
- 6-methoxy-N2,N2-bis(2-methoxyethyl)pyridine-2,3-diamine;
- 6-methoxy-N2-ethyl-N2-methylpyridine-2,3-diamine;
- 6-methoxy-N2-methyl-N2-propylpyridine-2,3-diamine;
- 6-methoxy-N2-butyl-N2-methylpyridine-2,3-diamine;
- 6-methoxy-N2-(3-aminopropyl)-N2-methylpyridine-2,3-diamine;
- 2-[(3-amino-6-methoxy-2-pyridyl)ethylamino]ethanol;
- 6-methoxy-N2,N2-diisopropylpyridine-2,3-diamine;
- 6-methoxy-N2,N2-dipropylpyridine-2,3-diamine;
- 6-methoxy-N2-butyl-N2-ethylpyridine-2,3-diamine;
- 6-methoxy-N2-(2-aminoethyl)-N2-isopropylpyridine-2,3-diamine;
- 6-methoxy-N2-cyclohexyl-N2-methylpyridine-2,3-diamine;
- 2-[(3-amino-6-methoxy-2-pyridyl)(3-aminopropyl)amino]ethanol;
- 6-methoxy-N2-cyclohexyl-N2-ethylpyridine-2,3-diamine;
- 2-[(3-amino-6-methoxy-2-pyridyl)pentylamino]ethanol;
- 2-[(3-amino-6-methoxy-2-pyridyl)cyclohexylamino]ethanol;
- 2-[(3-amino-6-methoxy-2-pyridyl)(2-aminoethyl)amino]ethanol;
- 6-methoxy-N2-methyl-N2-(6-methylaminohexyl)pyridine-2,3-diamine;
and the addition salts thereof.

12. Composition according to any one of Claims 1 to 11, in which the oxidation base(s) is (are) chosen from para-phenylenediamines, bis(phenyl)alkylenediamines, para-aminophenols, bis-para-aminophenols, ortho-aminophenols and heterocyclic bases, and the addition salts thereof.

13. Composition according to any one of Claims 1 to 12, in which the oxidation base(s) is (are) each present in an amount of between 0.001% and 10% by weight relative to the total weight of the dye composition.

14. Composition according to any one of Claims 1 to 13, comprising one or more additional couplers chosen from meta-phenylenediamines, meta-aminophenols, meta-diphenols, naphthalene-based couplers and heterocyclic couplers other than the couplers of formula (I) as defined in any one of Claims 1 to 11, and the addition salts thereof.

15. Composition according to any one of Claims 1 to 14, in which the coupler(s) is (are) each present in an amount of between 0.001% and 10% by weight relative to the total weight of the dye composition.

16. Composition according to any one of the preceding claims, in which the dyeing medium is a cosmetic medium that is suitable for dyeing keratin fibres.

17. Composition according to any one of the preceding claims, also comprising an oxidizing agent.

18. Process for the oxidation dyeing of keratin fibres, **characterized in that** a composition as defined in any one of Claims 1 to 16 is applied to the fibres, in the presence of an oxidizing agent, for a time that is sufficient to allow the desired colour to be obtained.

19. Process according to Claim 18, in which the oxidizing agent is chosen from hydrogen peroxide, urea peroxide, alkali metal bromates, persalts, peracids and oxidase enzymes.

20. Process according to either of Claims 18 and 19, in which the oxidizing agent is mixed at the time of use with the composition as defined according to any one of Claims 1 to 16.

21. Process according to either of Claims 18 and 19, in which the oxidizing agent is applied to the fibres simultaneously with or sequentially to the composition as defined according to any one of Claims 1 to 16, in the form of an oxidizing composition.

22. Multi-compartment device, in which a first dye compartment contains a composition as defined in any one of Claims 1 to 16 and a second compartment contains an oxidizing composition.

23. Kit for dyeing keratin fibres, comprising firstly a composition as defined according to any one of Claims 1 to 16, and secondly an oxidizing composition.

24. Use of the composition as defined according to any one of Claims 1 to 16, for dyeing keratin fibres.

25. 6-Alkoxy-2,3-diaminopyridine compound of formula (I) as defined in any one of Claims 1 to 11, with the exception of 2-(N-methyl-N-(β-hydroxyethyl)amino)-3-amino-6-methoxypyridine.

## Patentansprüche

1. Farbmittelzusammensetzung, die in einem zum Färben geeigneten Medium enthält:
- mindestens eine Oxidationsbase, und
- mindestens einen 6-Alkoxy-2,3-diaminopyridinkuppler der Formel (I) oder eines seiner entsprechenden Additionssalze 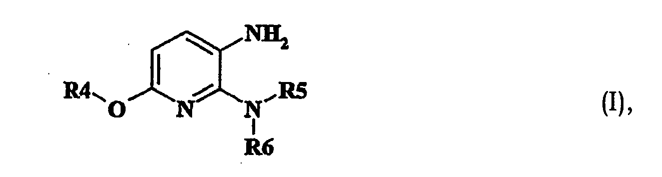 worin bedeuten:
- R₄ eine geradkettige oder verzweigte C₁₋₄-Alkylgruppe, die gegebenenfalls substituiert ist mit einer oder mehreren Gruppen Hydroxy, C₁₋₂-Alkoxy, NR₇R₈, wobei R₇ und R₈ unter Wasserstoff, C₁₋₄-Alkyl, C₂₋₆-(Poly)hydroxyalkyl, C₂₋₆ (Poly)aminoalkyl oder C₂₋₆-Aminohydroxyalkyl ausgewählt sind;
- R₅ eine geradkettige oder verzweigte C₁₋₆-Alkylgruppe, die gegebenenfalls mit einer oder mehreren Gruppen substituiert ist, die ausgewählt sind unter C₁₋₂-Alkoxy, Hydroxy, Carboxy, Sulfonsäure (-SO₃H), NR₉R₁₀; eine Acylaminoethylgruppe; eine 2-(2-Hydroxyethyloxy)-ethylgruppe;
- R₆ eine geradkettige oder verzweigte C₁₋₆-Alkylgruppe, die gegebenenfalls substituiert ist mit einer oder mehreren Gruppen, die ausgewählt sind unter C₁₋₂-Alkoxy, Hydroxy, Carboxy, Sulfonsäure, NR'₉R'₁₀, oder Acylamino (RCONH-, wobei R eine C₁₋₄-Alkylgruppe ist);
- R₉, R₁₀, R₉, und R_{10'} unabhängig voneinander ein Wasserstoffatom; eine C₁₋₄-Alkylgruppe, die gegebenenfalls mit einer oder mehreren Gruppen Hydroxy, Amino, Mono- oder Dialkylamino, Alkoxy oder Acylamino substituiert ist.

2. Zusammensetzung nach Anspruch 1, wobei R₆ eine substituierte Alkylgruppe bedeutet.

3. Zusammensetzung nach Anspruch 1, wobei R₅ und R₆ unabhängig voneinander eine C₁₋₄-Alkylgruppe bedeuten, die gegebenenfalls mit einer oder mehreren Gruppen substituiert ist, die unter den Gruppen Hydroxy, C₁₋₂-Alkoxy, Amino, C₁₋₄-(Di)alkylamino, Carboxy, C₁₋₂-(Di)hydroxyalkylamino ausgewählt sind; eine 2-Acylaminoethylgruppe; eine 2-(2-Hydroxyethyloxy)-ethylgruppe bedeuten.

4. Zusammensetzung nach Anspruch 3, worin R₅ Methyl, Ethyl, Propyl, 2-Hydroxyethyl, 2-Aminoethyl, 2-Carboxyethyl, 2-Acylaminoethyl, 2-Hydroxypropyl, 3-Hydroxypropyl, 3-Aminopropyl, 2-N,N-Dimethylaminoethyl, 2-N-Methylaminoethyl, 2-(2-Hydroxyethylamino)-ethyl, 2-(2-Hydroxyethyloxy)-ethyl bedeutet.

5. Zusammensetzung nach einem der Ansprüche 1 bis 3, wobei R₆ eine C₁₋₄-Alkylgruppe, die mit einer oder mehreren Gruppen substituiert ist, die unter Hydroxy, C₁₋₂-Alkoxy, Amino, C₁₋₂-(Di)alkylamino, Carboxy, Acylamino, (Di)hydroxyalkylamino ausgewählt sind; eine 2-(2-Hydroxyethyloxy)-ethylgruppe bedeutet.

6. Zusammensetzung nach Anspruch 5, wobei R₆ 2-Hydroxyethyl, 2-Aminoethyl, 2-Carboxyethyl, 2-Acylaminoethyl, 2-Hydroxypropyl, 3-Hydroxypropyl, 3-Aminopropyl, 2-N,N-Dimethylaminoethyl, 2-N-Methylaminoethyl, 2-(2-Hydroxyethylamino)-ethyl, 2-(2-Hydroxyethyloxy)-ethyl bedeutet.

7. Zusammensetzung nach einem der Ansprüche 1 bis 6, wobei R₄ eine C₁₋₄-Alkylgruppe bedeutet, die gegebenenfalls mit einer oder zwei Gruppen Hydroxy, C₁₋₂-Alkoxy substituiert ist.

8. Zusammensetzung nach Anspruch 7, wobei R₄ eine C₁₋₄-Alkylgruppe ist.

9. Zusammensetzung nach Anspruch 8, wobei R₅ eine Alkylgruppe bedeutet und R₆ eine mit einer oder mehreren Hydroxygruppen substituierte Alkylgruppe ist.

10. Zusammensetzung nach Anspruch 8, wobei R₅ und R₆ eine Alkylgruppe bedeuten, die mit einer oder mehreren Hydroxygruppen substituiert ist.

11. Zusammensetzung nach einem der Ansprüche 1 bis 10, wobei die Verbindung der Formel (I) ausgewählt ist unter:
- 2-[(3-Amino-6-methoxy-pyridin-2-yl)-methyl-amino]-ethanol;
- 2-[(3-Amino-6-methoxy-pyridin-2-yl)-butyl-amino]-ethanol;
- 2-[(3-Amino-6-methoxy-pyridin-2-yl)-(2-hydroxy-ethyl)-amino]-ethanol;
- 3-[(3-Amino-6-methoxy-pyridin-2-yl)-methyl-amino]-propan-1,2-diol;
- 6-Methoxy-N2,N2-bis(2-methoxy-ethyl)-pyridin-2,3-diamin;
- 6-Methoxy-N2-ethyl-N2-methyl-pyridin-2,3-diamin;
- 6-Methoxy-N2-methyl-N2-propyl-pyridin-2,3-diamin;
- 6-Methoxy-N2-butyl-N2-methyl-pyridin-2,3-diamin;
- 6-Methoxy-N2-(3-amino-propyl)-N2-methyl-pyridin-2,3-diamin;
- 2-[(3-Amino-6-methoxy-pyridin-2-yl)-ethyl-amino]-ethanol ;
- 6-Methoxy-N2,N2-diisopropyl-pyridin-2,3-diamin ;
- 6-Methoxy-N2,N2-dipropyl-pyridin-2,3-diamin ;
- 6-Methoxy-N2-butyl-N2-ethyl-pyridin-2,3-diamin;
- 6-Methoxy-N2-(2-amino-ethyl)-N2-isopropyl-pyridin-2,3-diamin;
- 6-Methoxy-N2-cyclohexyl-N2-methyl-pyridin-2,3-diamin;
- 2-[(3-Amino-6-methoxy-pyridin-2-yl)-(3-amino-propyl)-amino]-ethanol;
- 6-Methoxy-N2-cyclohexyl-N2-ethyl-pyridin-2,3-diamin;
- 2-[(3-Amino-6-methoxy-pyridin-2-yl)-pentyl-amino]-ethanol;
- 2-[(3-Amino-6-methoxy-pyridin-2-yl)-cyclohexyl-amino]-ethanol;
- 2-[(3-Amino-6-methoxy-pyridin-2-yl)-(2-amino-ethyl)-amino]-ethanol;
- 6-Methoxy-N2-methyl-N2-(6-methylamino-hexyl)-pyridin-2,3-diamin;
sowie deren Additionssalzen.

12. Zusammensetzung nach einem der Ansprüche 1 bis 11, wobei die Oxidationsbase(n) unter den *p*-Phenylendiaminen, Bisphenylalkylendiaminen, *p*-Aminophenolen, Bis-*p*-aminophenolen, *o-*Aminophenolen, heterocyclischen Basen und deren Additionssalzen ausgewählt sind.

13. Zusammensetzung nach einem der Ansprüche 1 bis 12, wobei die Oxidationsbase oder die Oxidationsbasen jede in einer Menge von 0,001 bis 10 Gew.-% des Gesamtgewichts der Farbmittelzusammensetzung enthalten sind.

14. Zusammensetzung nach einem der Ansprüche 1 bis 13, die einen oder mehrere zusätzliche Kuppler enthält, die unter den m-Phenylendiaminen, m-Aminophenolen, m-Dihydroxybenzolen, Naphthalinkupplern und heterocyclischen Kupplern, die von den Kupplern der Formel (I) nach einem der Ansprüche 1 bis 11 verschieden sind, und deren Additionssalzen ausgewählt sind.

15. Zusammensetzung nach einem der Ansprüche 1 bis 14, wobei der oder die Kuppler jeder in einer Menge von 0,001 bis 10 Gew.-% des Gesamtgewichts der Farbmittelzusammensetzung enthalten sind.

16. Zusammensetzung nach einem der vorhergehenden Ansprüche, wobei das Farbmittelmedium ein zum Färben von Keratinfasern geeignetes kosmetisches Medium ist.

17. Zusammensetzung nach einem der vorhergehenden Ansprüche, die ferner ein Oxidationsmittel enthält.

18. Verfahren zum oxidativen Färben von Keratinfasern, **dadurch gekennzeichnet, dass** auf die Fasern eine Zusammensetzung nach einem der Ansprüche 1 bis 16 in Gegenwart eines Oxidationsmittels während einer Zeitspanne aufgebracht wird, die ausreichend ist, damit die gewünschte Färbung gebildet wird.

19. Verfahren nach Anspruch 18, wobei das Oxidationsmittel unter Wasserstoffperoxid, Harnstoffperoxid, Alkalimetallbromaten, Salzen von Persäuren, Persäuren und Oxidasen ausgewählt ist.

20. Verfahren nach einem der Ansprüche 18 oder 19, wobei das Oxidationsmittel bei der Anwendung mit der Zusammensetzung nach einem der Ansprüche 1 bis 16 vermischt wird.

21. Verfahren nach einem der Ansprüche 18 bis 19, wobei das Oxidationsmittel gleichzeitig mit der Zusammensetzung nach einem der Ansprüche 1 bis 16 in Form einer oxidierenden Zusammensetzung auf die Fasern aufgetragen wird oder getrennt davon anschließend.

22. Vorrichtung mit mehreren Abteilungen, wobei eine erste Farbmittelabteilung eine Zusammensetzung nach einem der Ansprüche 1 bis 16 und eine zweite Abteilung eine oxidierende Zusammensetzung enthält.

23. Kit zum Färben von Keratinfasern, die einerseits eine Zusammensetzung nach einem der Ansprüche 1 bis 16 und andererseits eine oxidierende Zusammensetzung enthält.

24. Verwendung der Zusammensetzung nach einem der Ansprüche 1 bis 16 zum Färben von Keratinfasern.

25. 6-Alkoxy-2,3-diaminopyridin der Formel (I) nach einem der Ansprüche 1 bis 11, wobei das 2-(N-Methyl-N-(β-hydroxy-ethyl)-amino-3-amino-6-methoxypyridin ausgenommen ist.
